# EUROPEAN PATENT SPECIFICATION

(11) **EP 3 247 201 B1**
(45) Date of publication and mention of the grant of the patent: **06.11.2019**
(21) Application number: 16704793.5
(22) Date of filing: 21.01.2016
(51) Int. Cl.: A01K 1/015, A23K 10/37, A01G 31/00, C05F 11/02, C05F 17/00, C05G 3/04

(54) **METHODS FOR UPGRADING SPENT BIOMASS MATERIAL**
VERFAHREN ZUR VEREDELUNG VON VERBRAUCHTEM BIOMASSEMATERIAL
PROCÉDÉS DE VALORISATION D'UN MATÉRIAU DE BIOMASSE ÉPUISÉ

(30) Priority: 22.01.2015 DK 201570040; 22.01.2015 DK 201570039; 09.12.2015 DK 201570813
(43) Date of publication of application: 29.11.2017
(73) Proprietor: Advanced Substrate Technologies A/S, 8960 Randers SØ (DK)
(72) Inventor: HOFF, Svend Kristian, 8300 Odder (DK)
(74) Representative: Høiberg P/S
(86) International application number: PCT/DK2016/050018
(87) International publication number: WO 2016/116113

(56) References cited:
- EP-A1- 1 688 475
- WO-A1-2015/007290
- GB-A- 2 161 466
- US-A- 5 557 873
- US-A1- 2004 025 715
- DATABASE WPI Week 199143 Thomson Scientific, London, GB; AN 1991-316458 XP002756436, & SU 1 629 026 A (GRASS STRAW PROCESS) 23 February 1991 (1991-02-23)

## Description

### Technical Field

The present disclosure relates to a fibrous solid substrate in pellet form obtained from spent biomass material from a biogas fermenter following an anaerobic fermentation and biogas production and use of said pellet in agriculture. The present disclosure also relates to biomass material processing and renewable energy production. More specifically, the present disclosure relates to methods for upgrading spent biomass material from a biogas production unit to fertilizer products, fibers and substrate for biogas.

### Background

Many traditional energy sources, such as e.g. fossil fuels, are being depleted faster than new ones are being made. Fossil fuels are non-renewable resources because they take millions of years to form. Also, use of fossil fuels raises serious environmental concerns. Renewable energy sources represent a promising alternative to many traditional energy sources. Biomass represents one source of renewable energy and increased exploitation of the energy potential of a biomass may result in an increased production of renewable energy sources, such as biogas.

If biomass energy is to have a long-term, commercial future, the organic material must be processed to generate affordable, clean and efficient energy forms, such as liquid and gaseous fuels, or electricity. Biomass processing is important to ensure an efficient exploitation of the biomass energy. However, the energy potential can often be difficult to exploit. An increased exploitation of the energy potential of a biomass may result in an increased production of renewable energy sources, such as biogas; and the degasified material, i.e. the spent biomass material can be processed and transformed into fertilizer products and substrates with added value.

Additionally, spent biomass materials having a reduced organic nitrogen content can be upgraded, sanitized and re-used in the production of many edible products.

Extensive scientific and engineering work has been conducted on the biogasification of waste materials. The fundamental technique relies on an anaerobic digestion or fermentation process. However, anaerobic microorganisms responsible for methanogenesis are inhibited by ammonia and methanogenesic anaerobic bacteria are inhibited by and cease to metabolise nutrients effectively at high ammonia concentration levels.

The problems associated with ammonia inhibition have made currently available technical solutions difficult to operate - especially when using biomasses containing relatively high amounts of nitrogen.

To mitigate these problems, it has been attempted to control the carbon to nitrogen (C/N) ratio of the biomass material subjected to fermentation. However, state-of-the-art solutions continue to suffer from a number of disadvantages. For example, adjusting the ammonia concentration in a reactor by adjusting the C/N ratio of the biomass is a slow process, and adjusting the C/N ratio may prove insufficient when handling biomasses prone to generating relatively high ammonia concentrations during anaerobic digestion.

Lime pressure cooking and ammonia stripping has been disclosed as a way to reduce the amount of organic and inorganic nitrogen present in a biomass to be subjected to anaerobic fermentation. Reference is made e.g. to US 7,883,884.

Furthermore, many complex biomasses contain macromolecular constituents which are difficult to metabolize for microbial organisms traditionally involved in the production of biogas. In particular, macromolecular constituents, such as cellulose, hemicellulose, lignocellulose and lignin, are present in many biomass materials and can only be metabolized to a limited extent during a biogas fermentation process.

There is a need for improved and more cost effective pre-treatment methods for rendering the afore-mentioned, recalcitrant biomass material constituents more accessible for many microbial organisms present during different stages of a biogas production.

There is a need for more efficient utilization of energy and nutrients potentials in spent biomass material from biogas production. Biogas production plants produce large amounts of spent biomass material comprising liquid and solid parts which must be processed and fractionated to be usable for further biogas production and as fertilizers.

WO 2015/007290 (PCT/DK2014/050220) discloses a method for manufacturing a fibrous solid substrate obtained from a biomass material from a biogas fermenter following an anaerobic fermentation and biogas production. Other methods for treating other biomasses are also known. EP 1 688 475 discloses a method for treating non-fermented manure slurry, US 5 557 873 discloses sludge water treatment systems for treating waste water sludge and sewage sludge, and Database WPI week 199143 (Thomson Scientific, London, GB) discloses a method of treating straw.

### Summary

The invention is as defined in the claims.

A first aspect of the present disclosure relates to the use of a fibrous solid substrate in pellet form in a method of cultivating fungal cells and/or spores; as a fertilizer; as a soil improver; and/or as litter for animals; wherein said fibrous solid substrate in pellet form is obtained by a method comprising the steps of
a. providing a biomass material comprising solid and liquid parts from a biogas fermenter following an anaerobic fermentation and biogas production,
b. subjecting the fermented biomass material of step a. to one or more separation steps resulting in the provision of
   i) a fibrous solid fraction comprising organic and inorganic nitrogen parts and having a reduced content (w/w) of water, and comprising one or more macromolecular nutrient constituents selected from the group consisting of cellulose, hemicellulose, lignin and lignocellulose, and
   ii) at least one liquid fraction comprising solid and liquid organic and inorganic phosphor-containing parts,
c. subjecting the fibrous solid fraction of step b. to a sanitation treatment, said treatment
   comprising the steps of i) heating the fibrous solid fraction to a temperature of more than 70°C, optionally under alkaline pH conditions, and optionally ii) subjecting the fibrous solid fraction comprising organic and inorganic nitrogen parts to a pressure of more than 1 bar,
   wherein said treatment i) reduces or eliminates viable microorganisms present in the fibrous solid fraction, and/or ii) reduces the contents of volatile nitrogen-containing compounds and/or precursor volatile compounds present in the fibrous solid fraction, and
d. obtaining a fibrous solid fraction comprising organic and inorganic nitrogen parts having a reduced content of volatile nitrogen-containing compounds,
e. optionally subjecting said fibrous solid fraction to one or more of heating, drying, evaporation, pressure, and/or alkaline pH conditions,
f. optionally adding to said fibrous solid fraction one or more solid and/or liquid supplemental nutrient substrate compositions, and
g. compressing said fibrous solid fraction of step d., e. and/or f., thereby generating a fibrous solid substrate in pellet form.

A second aspect of the present disclosure relates to the use of a fibrous solid substrate in pellet form in a method for cultivating fungal cells and/or spores comprising the steps of
a. providing fungal cells and/or spores,
b. providing the fibrous solid substrate in pellet form according to any of the preceding claims, wherein the fibrous solid substrate in pellet form optionally further comprises one or more supplemental nutrient substrate compositions suitable for fungal growth,
c. adding water to the fibrous solid substrate in pellet form to obtain a wetted fibrous substrate,
d. contacting the fungal cells and/or spores with the wetted fibrous substrate,
e. cultivating the fungal cells and/or spores in said substrate, and
f. optionally obtaining a spent fungal substrate.

### Definitions

Batch fermentation: Unit operation of a fermenter where one fermenter cycle of e.g. feedstock preparation, biogas fermentation, volatile compound production, and product formation is completed before the next fermentation cycle is started.
Bioenergy: The production, conversion, and use of material directly or indirectly produced by photosynthesis (including organic waste) to manufacture fuels and substitutes for petrochemical and other energy-intensive products.
Biogas: a mixture of gases produced by the breakdown of organic matter in the absence of oxygen. Biogas primarily contain methane (CH₄), and also carbon dioxide (CO₂), and may have small amounts of hydrogen sulphide (H ₂S), moisture and siloxanes.
Biomass material: Organic material comprising fermentable carbon and nitrogen sources capable of being utilized by microbial organisms in a fermentation.
Continuous fermentation: A steady-state fermentation system in which substrate is continuously added to a fermenter while products and residues are removed at a steady rate.
Cooking: A process to turn dry starch and water into liquefied mash (water, grain hulls + germ, & dextrins) using pH controls, set temperature parameters, solids controls, & enzymes.
Dewatering: The separation of free water from the solids portion of e.g. fibrous solid fractions, spent mash, sludge, or whole stillage by screening, centrifuging, filter pressing, or other means.
Digestate: Solid fraction obtained from anaerobic fermentation and biogas production. A digestate comprises a fibrous fraction, non-fibrous solids, such as minerals, and a liquid fraction. Anaerobic digestion produces two main products: digestate and biogas.
Feedstock biomass material: A substance used as a raw material in a fermentation.
Fermentation: Biological conversion of biomass materials. Fermentation is one of several steps in the processing of biomass. A fermentation is collectively all of the metabolic processes involved in the conversion of fermentable sugars into one or more fermentation end products, such as e.g. gases, acids and alcohols.
Fiber / fibrous material: Residual fraction of plant origin and part of a digestate resulting from an anaerobic fermentation and biogas production. The chemical composition of a fibrous material or fraction can comprise or consist of e.g. cellulose and hemi-cellulose, lignin, lignocellulose, and additional plant components, such as e.g. dextrins, inulin, chitins, pectins, and beta-glucans
N-mineralization: Process of converting ammonium (ions) into ammonia (gas).
N-stripping: Process of removing volatile nitrogen containing compounds by evaporation. Abbreviated herein as NS.
N-water: Residual liquid fraction comprising small amounts of largely inorganic nitrogen containing compounds.
NH₃: Gaseous ammonia - inhibitory for a biogas fermentation in high concentrations. An example of a volatile nitrogen-containing compound.
NH₄+: Ammonium ion or ammonium salt. Capable of being converted into gaseous ammonia. An example of a precursor compound of a volatile nitrogen-containing compound.
Pellet: A pellet is a relatively small particle (mm to cm sized) created by compressing an original material.
Permeate: Liquid fraction resulting from a separation process in which a biomass material is separated into a fibrous solid fraction and one or more liquid fractions comprising solid and liquid matter. The permeate is an essentially liquid fraction void of solid substances i.e. it is a non-fibrous liquid fraction.
Pre-digested biomass material: Feed stock biomass material for an anaerobic fermentation obtained from a prior performed anaerobic fermentation.
Slurry: Manure with a total solids concentration of between approximately 5 and 15 percent. Slurries are pumpable. Above a total solids concentration of 15 percent, slurries are semisolids with a negligible angle of repose and can be scraped but not stacked for storage.
Stripping-off volatile compounds: Process of performing an evaporation under conditions at which volatile compounds will be present on a gaseous form.

### Description of Drawings

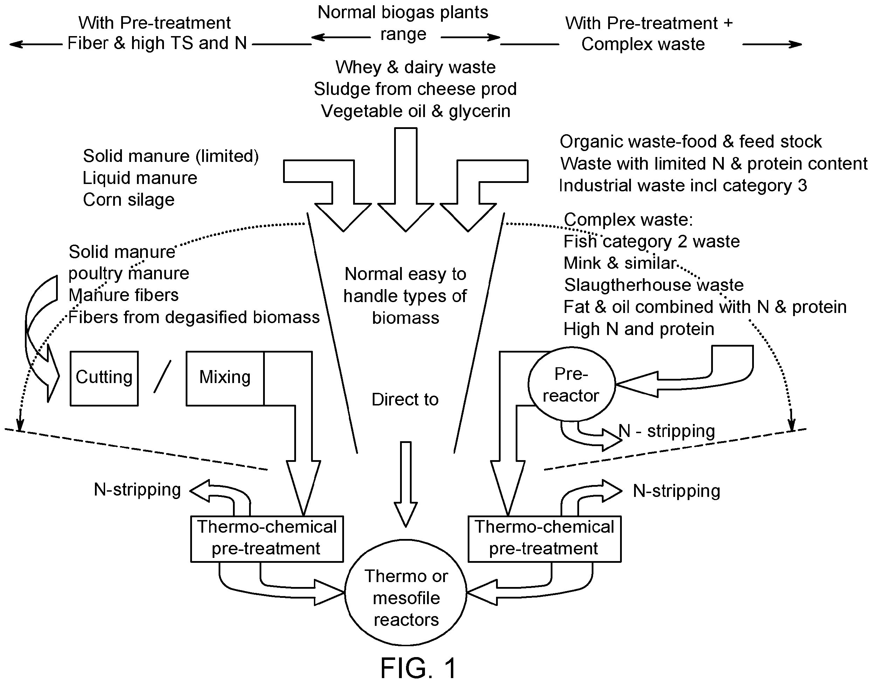

Fig. 1 illustrates the principle of producing biogas comprising operating one or more biogas reactors and one or more fermentation(s) of different biomasses; directly in a fermentation facility (thermo or mesophile reactors) for normal and easy to handle waste, and in one embodiment according to the present disclosure following a pre-treatment based on the type and complexity of biomass; such as a thermo-chemical pre-treatment, such as e.g. lime pressure cooking resulting in stripping of ammonia N.
   While conventional biogas reactors can be operated using e.g. manures, organic waste materials, corn silage, whey and dairy waste, sludge from cheese production, as well as vegetable oils, biogas reactors operated in accordance with the present disclosure can be operated using a wide range of organic materials having a high content of nitrogen such as complex wastes, especially when employing a first fermentation step in a pre-fermentation facility unit (indicated as a pre-reactor), where the primary aim is removal of first volatile solid such as nitrogen rather than biogas production.

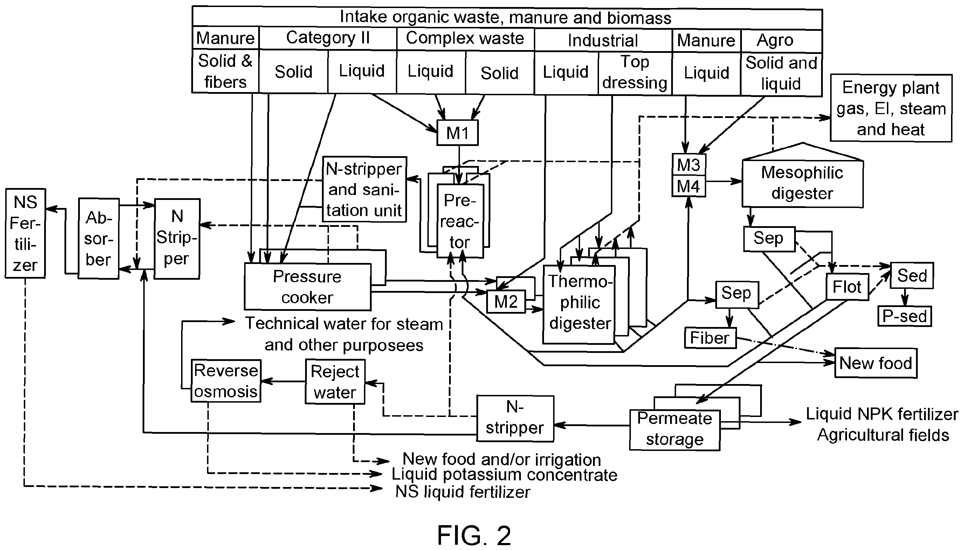

Fig. 2 illustrates a process flow for various biomass types in accordance with an embodiment of the present disclosure. Simpler manure and agro waste is directly treated in the fermentation facility (mesophilic digester) after mixing in M3 and M4. Industrial waste material is sent to the mesophilic digester after having undergone a thermophilic digestion step.
   The waste may first undergo a mixing step in mixer M2. For manure and/ or some category II waste, a thermo-chemical treatment in the pressure unit (pressure cooker) is employed prior to sending the waste to the fermentation facility. The pressure cooker is operationally connected with N-stripper for stripping the nitrogen produced during the hydrolysis process in the pressure unit.
   For complex waste and some category II waste, including straw and the like, a further pre-treatment step in the pre-reactor is performed prior to the thermo-chemical treatment, to maximize the conversion of organic N and protein. The pre-reactor is operationally connected with an N-stripper and sanitation unit, for passing before sending the sanitized first fermented biomass for the thermo-chemical treatment in the pre-reactor, followed by the fermentation for production of biogas. As part of post-processing, separation techniques may be employed for obtaining valuable recoverable like N, P, K, water.

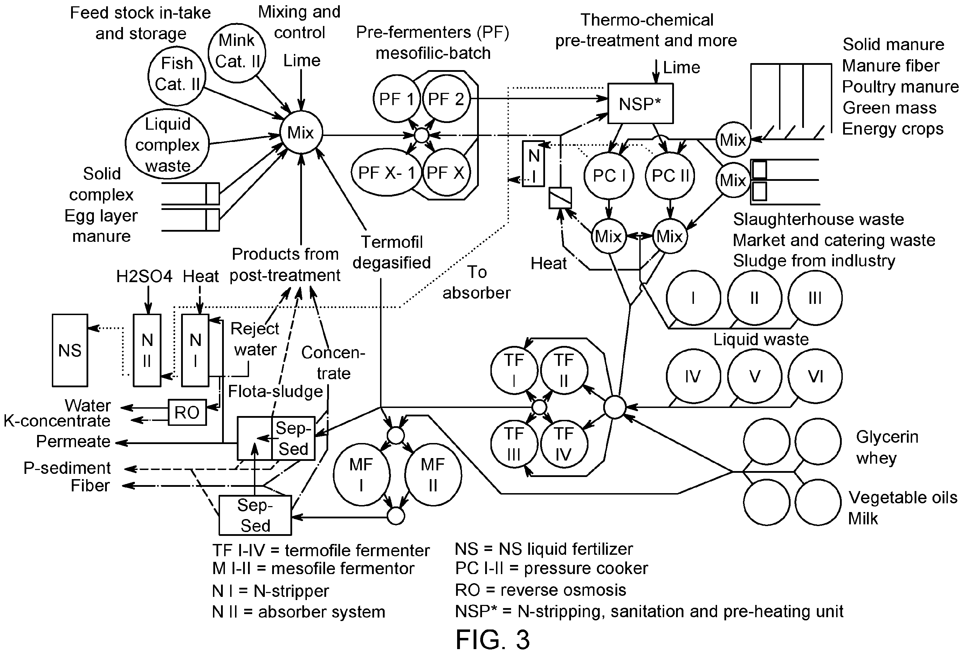

Fig. 3 illustrates a system diagram for employing the process in accordance with an embodiment of the disclosure. In particular, the system includes feed stock in-take and storage that provides category II and/ or complex biomass for biogas generation utilizing the first fermentation facility unit (pre-fermentors) and N-stripper and sanitation unit. The stripper and sanitation unit being connected to pressure unit (pressure cooker) where hydrolyses takes place. The pressure units are in-turn connected to the fermentation facility that may include a plurality of either thermophilic digesters or mesophilic potentially being interconnected.
   In other embodiments according to the present disclosure, there are biomass feed facility directly to the pressure unit (see Liquid waste I, II, and II), the biomass fed directly is then sent to the fermentation facility after hydrolysis in the pressure unit. In another embodiment according to the present disclosure, the biomass may also be directly fed to the fermentation facility - either directly to the thermophilic fermentors or directly to the mesophilic fermentors or in sequence, as shown by liquid waste IV, V and VI or by feed input of glycerin, vegetable oil, milk and whey. In another embodiment according to the present disclosure, the pre-fermentors of the pre-fermentation facility are directly connected to the fermentation facility fermentors as well. In yet another embodiment according to the present disclosure there is provided a closed loop between the pressure unit and the pre-fermenters for allowing further fermentation of the biomass material.

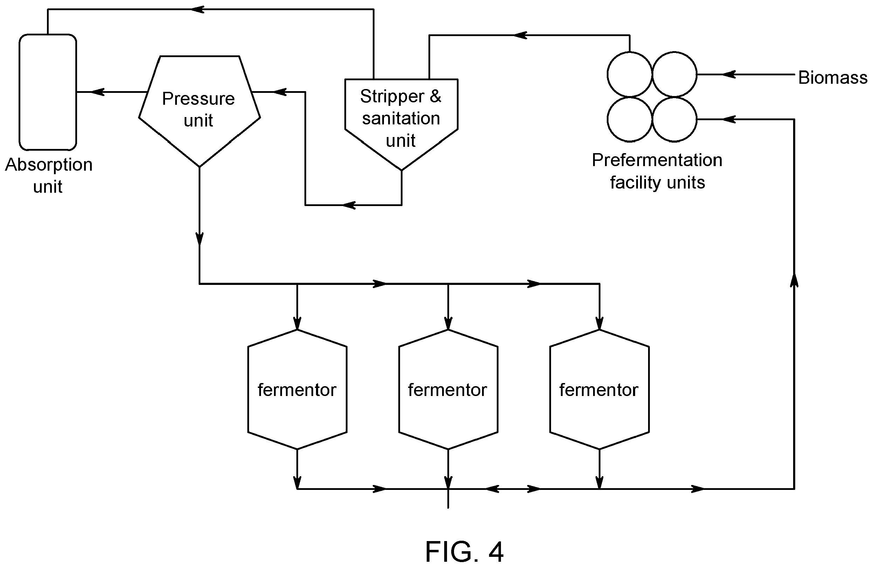

Fig. 4 illustrates a block diagram of system for handling complex waste according to an embodiment of the disclosure. The biomass is first fermented in the pre-reactor facility, thereafter the first fermented biomass is diverted to the stripper and sanitation unit. The ammonia generated during the first fermentation is stripped off and absorbed. The sanitized first fermented biomass is subjected to pressure cooking (with or without addition of lime) in the pressure unit and the ammonia generated during this stage is stripped off and absorbed. The hydrolysed biomass with reduced N content is then diverted to the fermenters for anaerobic fermentation and production of biogas. Some amount of the biomass that gets fermented in the fermenters may be diverted back to the pre-fermenters, where the amount acts as a starter culture for facilitating initiation of fermentation in the pre-fermenters. In some situations, the first fermented biomass may skip the thermo-chemical treatment in the pressure unit and may directly be sent to the fermenters for production of biogas.

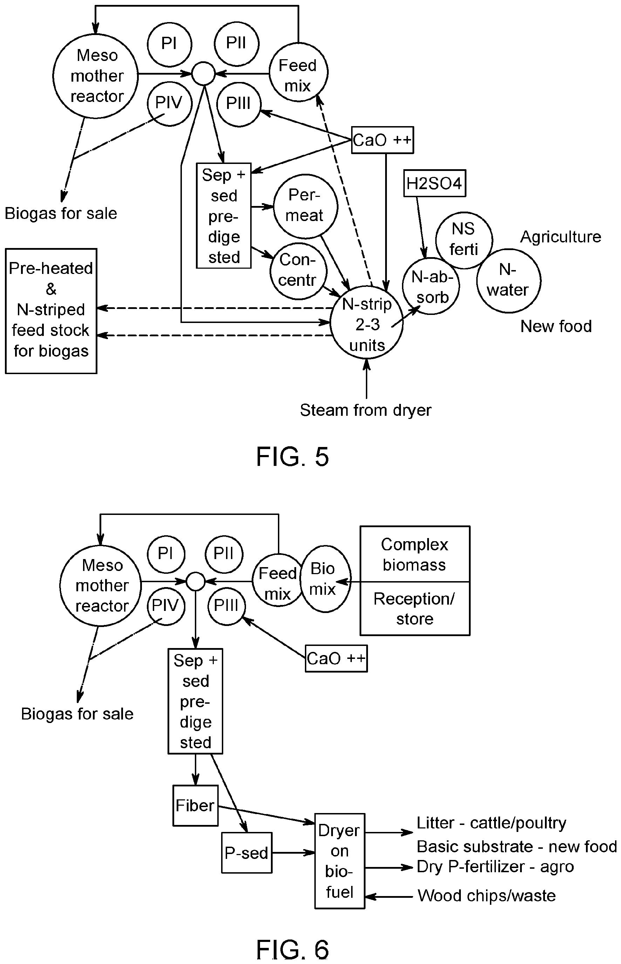

Figs. 5 & 6 illustrate principles of the handling and processing of a biomass material according to selected methods of the present disclosure. The biomasses with high dry matter and fiber content are grinded and mixed with more wet types of biomasses in the Bio-Mixer and from there led to the Feed-Mixer where its finally mixed with preheated and N-stripped biomass in order to obtain desired dry matter content and temperature before added to meso Mother reactor or pre-reactors (PI - PIV)
   Meso Mother reactor is a normal biogas reactor operated at 35-38 dg C, dry matter content ∼12 - 15% and average retention time of 12-15 days. Pre-reactors are smaller batch reactors operated at 35-38 dg C, dry matter content 15-18% and average retention time of 8-10 days + 1 day for "killing and empty" and 1 day for "reloading", to obtain the highest possible NH4+-N content before the process is "killed" by adding of CaO.
   From pre-reactors the partly degasified biomass is pumped to the Sep & Sed department (separation and sedimentation) and here it is split on desired fractions (fibers, concentrate, P-sediment and permeate) or led directly to the N-strippers. Fiber is dried in the drum dryer for evaporating inorganic N components and water.
   Concentrate - high in NH4+-N content - is led to N-strippers and after N-stripping recycled back as valuable and heated feed stock to the Feed-Mixer. P-sediment with high content of P and low in volatile dry matters is dried in the drum dryer and converted to high valuable dry P-fertilizers. Permeate - high in NH4+-N content but low in Org N and P content is led to N- strippers and after N-stripping recycled back as first diluting and heating feed stock to the Feed-Mixer.

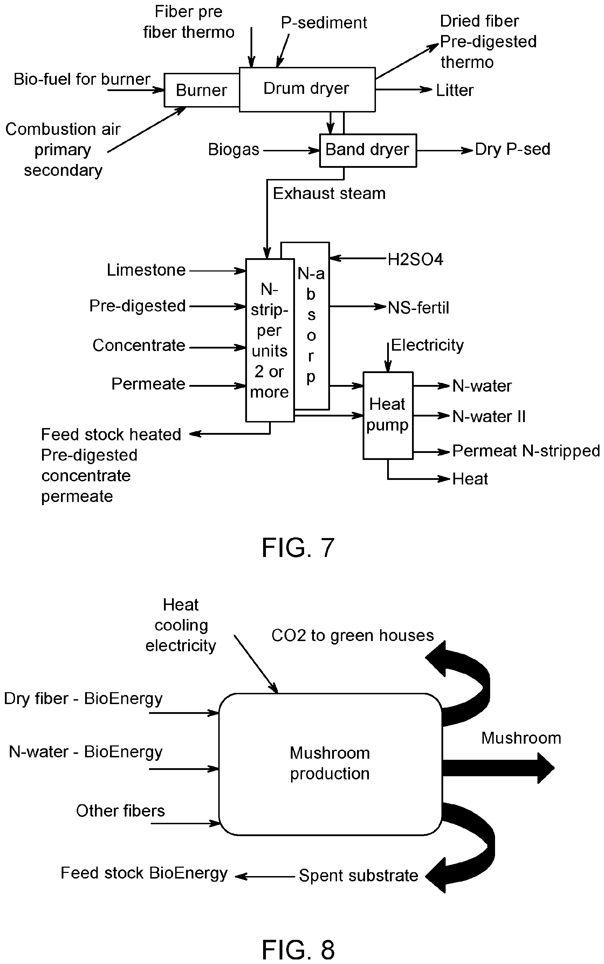

Fig. 7 illustrates the principle of drying a fibrous solid fraction by using a drum dryer fuelled with low quality wood chips and/or wood waste.
   Combustion air is taken from the production area and a slightly under pressure is created in order to reduce or preferable eliminate emission of smells and odeurants to the surrounding environment.
   The exhaust steam from drum dryer with its content of energy, water, inorganic N components, smelling and volatile substances are injected in pre-digested biomass, concentrate or permeate from Sep & Sed in the N-stripping units and integrated as part of N-stripped and heated biomass.
   CoA is added in order to secure optimal N-stripping effect. The stripped gaseous N is absorbed in absorbers and fixed in a liquid NS-fertilizer by adding H₂SO₄. The water part from absorbers is collected as N-water and used in new food production and agriculture for irrigation purposes or recycled back to the Feed-Mixer as second diluting and heating feed stock.
Fig. 8 illustrates a mushroom substrate as a mix of dry fibre from partly degasified biomass, as well as other specific types of fibres and N-water. From the substrate the mushroom takes water and part of nutrients and break down cellulose, hemicellulose, lignin and other components in order to get C. From 1,2 kg of substrate is produced 0,2 kg of exotic mushroom (e.g. Enokitake or Eryngii) and 1 kg of spent mushroom substrate or pre-treated biomass suitable as basic feed stock for biogas.
Fig. 9 illustrates one principle for separating and sedimenting a biomass material from an anaerobic fermentation. Partly degasified biomass is pumped to the inlet of the 1^{st} vibrating sieve and separated in a fibrous solid fraction, P-sediment, concentrate and permeate.

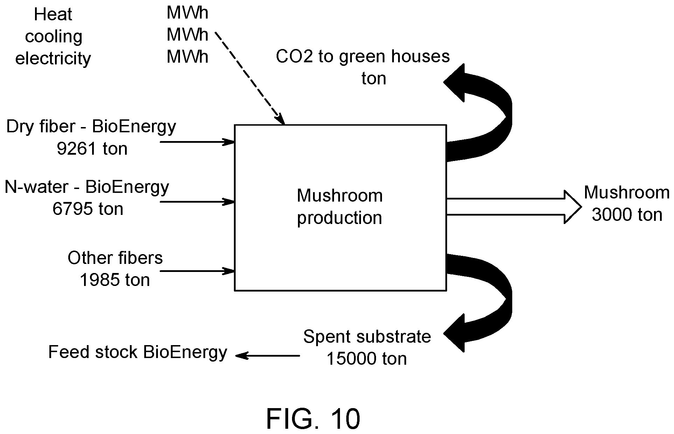

Fig. 10 illustrates the data in Table 6 (cf. examples)
Fig. 11 illustrates the circular system or value chain based on high efficiency in utilization of energy and nutrient potentials in biomass and organic waste products from agriculture and food industry.
   Starting in the left upper corner is shown a box with "Wet bio waste". The term "wet bio waste" covers biomass with content of macromolecular constituents, including cellulose, hemicellulose and lignin. A part of the "wet bio waste" - saw dust and wood chips - is after drying suitable to be stored and used as bio-fuel for drying or used as "C source" in production of "ready to use substrate" for mushroom production. Other sources like residues from production of sugar, vegetable oils and the like can after drying be stored and used as "protein and C source" in production of "ready to use substrate" for mushroom production.
   In the upper right corner focus is on production of "ready to use" substrate (fibrous solid substrate in pellet form) for production of more types of mushroom including - but not limited to - White Button, Eryngii, Enoki and Shitake. The "ready to use" term has to be understood as a substrate that can be stored until time of use and when used it is easy to handle just add water and mycelium.
   Important for a "ready to use" substrate is that it contains the needed nutrients - protein, C, N, P, K and more - and that it absorb water easy and fast and can keep water and still remain fluffy enough to avoid creation of anaerobic zones during production.
   The desired nutrients composition is obtained by adding one or more additional protein and/or C sources to the dry fibrous solid and the structure is maintained by use of dies in the pellet press with openings or holes with 12 to 16 mm and a press way that secures a compression of the material and a density of the pellets of 450 to 550 kg per m3 - a density that is 6 to 8 time higher than the density of the dry fibrous solid.
   After pressing the pellets have dry content of 85-90% or more and a temperature of 90°C or more and therefor cooling is needed before packaging in bags and the "ready to use" substrate pellets can be stored until time of use.
   The use of substrate pellets takes place at the mushroom production units - in the figure it is part of "agriculture". At time of use the normal desired moisture content of 67% in the substrate is reached by adding water, such as in the range of 1.4 - 2.8 ton of water per ton of substrate pellets.
   Finally mycelium is added and the production cycle is up and running.
   After production the spent mushroom substrate with its content of organic dry matter and nutrients can be recycled back and used as feed stock for biogas production as shown in the bottom of the figure.
   Returning back to the upper right corner of the figure is shown another optional use of the dry fibrous solid for production of litter pellets for horses, poultry or cattle. Here the raw material is 100% dry fiber solids from partly degasified biomass and in the press are used dies with 10 or 12 mm holes and a press way that secures a density of up to 600 kg per m3.
   After use in agriculture the litter pellets with additional manure and urine from animals can be recycled back and used as feed stock for biogas in parallel with spent mushroom substrate.
   Other types of residues from agriculture - straw, grass and the like - can also be integrated as inputs in the value chain. This requires some sort of additional treatment or pre-treatment of the material. Special attention in relation to this is the combined pre-treatment method based on cutting, grinding and cooking that will open up cellulose, hemicellulose and lignin and increase efficiency in utilization of energy potentials and allow higher dry matter content in the mix of biomasses to be added to the M-Meso and/or Pre reactors.
   The cutting takes place in a standard cutter adjusted to a maximum length after cutting of 5 cm and in this way a part of structure in the added biomass is maintained. An N-steamer may be introduced between the cutter and the grinder, cf, figure 12.
   The grinding takes place in a standard pellet press and die with 16 mm or bigger holes and a press way that secures a density of the granulate of 250 to 300 kg per m3 and a moisture content of 75 to 85%.
   The granulate can be stored in big bags, in a silo or just on a flour and a time of use added to the concentrate and pumped to NS1. In NS1 it will be heated up to 80°C or more in a fibrous liquid with pH of 9.5 or more and content of both CO₂ and ammonium.

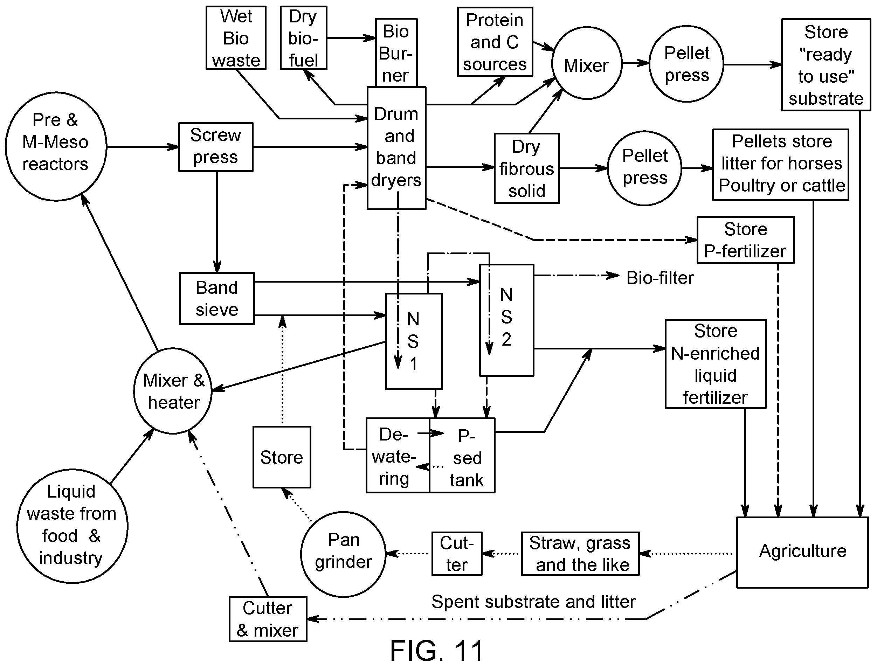

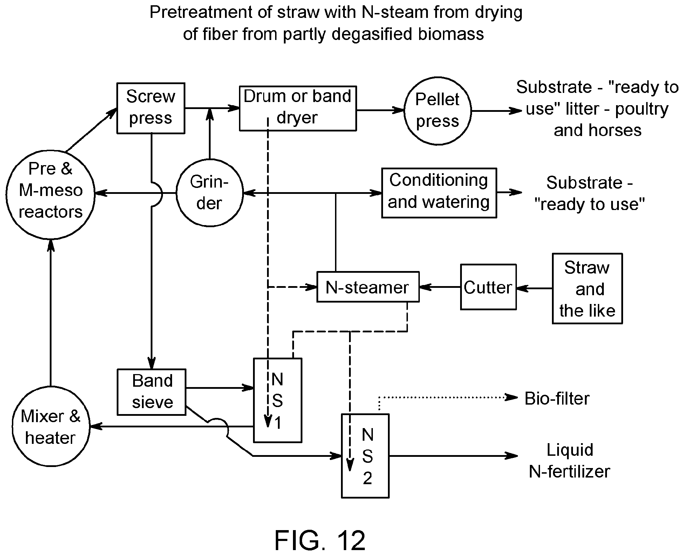

Fig. 12 illustrates ammonia N treatment or pre-treatment of materials such as complex waste, complex agricultural waste, and some category II waste, including crop residues, straw, grass and the like in an N-steamer. The N-steamer can be integrated in the set-up outlined in e.g. figure 11. Fig. 12 is specific on the intake, treatment and utilization of straw and similar types of biomasses rich in carbon. Broken lines represent gases (flow and utilization of N-steam), and solid lines represent solid materials (flow and utilization of e.g. straw).
   N-steam treatment increases the digestability and the protein content of the complex biomasses, such as straw, grass and the like, which increases its value and potential for use in biogas production, substrate and feed/fodder.
   The warm and moist N-steam from the dryer is diverted to the N-steamer (and/or NS1), together with the complex waste such as straw, grass and the like, which has first been cut and/or grinded to a desired length. Factors such as retention time, temperature, pH and moisture can be adjusted.
   The N-steamer utilises warm and moist steam comprising N from the dryer (drum or band dryer) to treat or pre-treat said materials, in order that they may be used as i) valuable biomasses for biogas production (biogas feed stock), as ii) fibre material for adding to the fibrous solid substrate in pellet form, as iii) a substrate directly utilisable for cultivating fungal cells and/or spores, or iv) a substrate directly utilisable as animal feed, optionally in pellet form.
   From the N-steamer, the treated material can be diverted to one or more of
   a) a grinder and subsequently a biogas reactor (for biogas feed stock i.e. i) above),
   b) a grinder and/or dryer and subsequently a pellet press (i.e. ii) and iv) above), and
   c) a conditioning and/or watering device, for substrates directly utilisable i.e. iii) and iv) above).

   The N-steam is diverted to NS1 for stripping and sanitation (to reduce the N-load on the system), and/or NS2 if the N-steam is no longer very warm.

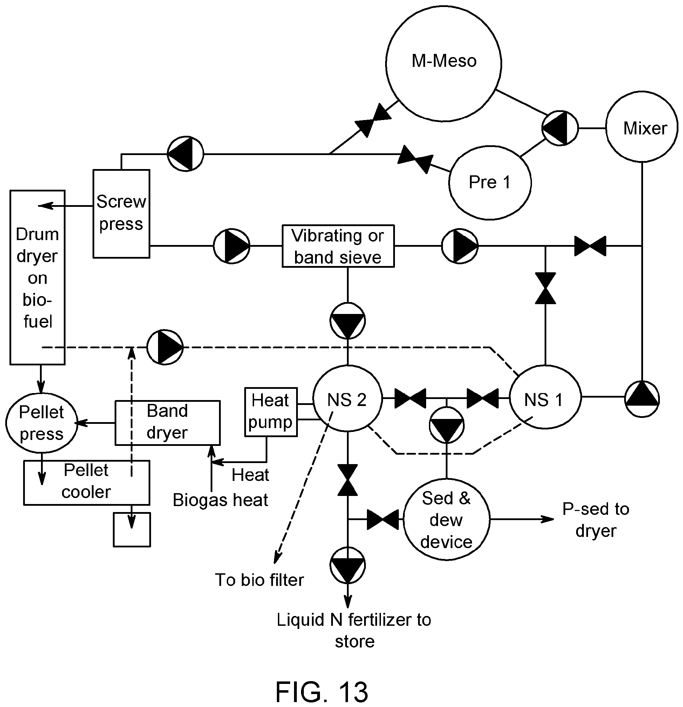

Fig. 13 shows an overview on flow and interaction between treatment steps and technologies. The starting point is that partly degasified biomass is taken from the biogas reactors - shown as M-meso and Pre1. M-Meso is a normal biogas reactor operated at 35°-38° or 48° - 51°C, dry matter content of 12 - 15% and average retention time of 12-15 days. Pre 1 is a group of smaller batch reactors operated at 35°-38° or 48° - 51°C, dry matter content 15-18% and average retention time of 8-10 days + 1 day for "killing and empty" and 1 day for "reloading".
   From pre-reactors and the M-Meso reactor the partly degasified biomass is pumped to the Sep & Sed department and here it is by use of separation and sedimentation technologies in more steps split into fibrous solid, concentrate, permeate and dewatered P-sed.
   In the first step - the screw press - the partly degasified biomass is split into:
   a) a fibrous solid fraction with more than 30% dry matter and
   b) a fibrous liquid fraction with less than 5% dry matter.

   In the second step the fibrous liquid fraction is by use of vibrating or band sieves split into:
   a) fibrous liquid fraction or concentrate with more than 9% dry matter and
   b) an essentially non-fibrous liquid fraction or permeate with less than 2% dry matter.

   The fibrous solid fraction is dried in a drum dryer or band dryer. The drum dryer is fuelled with low quality wood chips and/or wood waste and band dryer is fuelled with recovered process heat (heat pump) and biogas heat. Combustion air is taken from the production area and a slightly under pressure is created in order to reduce or preferable eliminate emission of smells and odorants to the surrounding environment.
   By drying of the fibrous solid fraction water and evaporation of inorganic N thereby is generated:
   a) a dry fibrous solid substrate having a reduced amount of inorganic N and dry matter content of 85% or more, and
   b) a first gaseous fraction or N-steam 1 comprising nitrogen containing compounds, including ammonia, CO₂ and water having a temperature of at least 80°C and preferably 90°C.

   Gaseous fractions are depicted as dotted lines.
   The dry fibrous solid substrate is led to a pellet press and compacted in pellets. After cooling and packaging the substrate pellets can be stored or shipped to final destination and used as basic mushroom substrate, as litter for horses, poultry or cattle and/or dry fibre.
   Concentrate - high in NH⁴⁺-N content - is led to N-strippers (NS1) or to the Mixer. In NS1 the concentrate is heated up to more than 80°C by injection of the said first gaseous fraction from drying of fibre and pH is adjusted to 9.5 or more by adding CaO (limestone). In NS1 effective stripping of ammonia can take place due the combination of high temperature and high pH that shift the balance from ammonium to ammonia and sedimentation of inorganic solids with high content of phosphor will take place and thereby is generated:
   a) a N-reduced and heated biogas substrate low in NH⁴⁺-N content and low in inorganic solid content that through the Mixer is recycled back to the biogas reactors.
   b) a phosphor (P) comprising fraction or sediment containing mainly inorganic solids and phosphor containing compounds, and
   c) a second gaseous fraction or N-steam 2 comprising nitrogen containing compounds, including ammonia, CO₂ and water having a temperature of at least 70°C.

   Permeate, i.e. the essentially non-fibrous liquid fraction - high in NH⁴⁺-N content but low in Org N and P content, is led to NS2. In NS2 the permeate is heated by injection of the said second gaseous fraction from NS1 and cooled by use of a heat pump and pH is adjusted to 6.5 or lower by adding acid.
   In NS2 absorption of ammonia can take place due the combination of low temperature and lower pH that shift the balance from ammonia to ammonium. In parallel sedimentation of inorganic solids will take place and thereby is generated:
   a) a cooled permeate or N-enriched liquid fertilizer high in NH⁴⁺-N content and low in inorganic solid content that can be stored until final use as fertilizer in agriculture,
   b) a sediment containing mainly inorganic solids and phosphor containing compounds, and
   c) a third gaseous fraction or N-steam 3 comprising nitrogen containing compounds, including ammonia, CO₂ and water having a temperature of 30°C or less that can be finally treated in a bio-filter.

   Sediments from NS1 and NS2 with high content of P and low in volatile dry matters are pumped to a sedimentation and dewatering device (Sed & dew device) and there are generated:
   a) a P-sediment or dewatered P-sed with dry matter content of 25% or more,
   b) a liquid fraction or permeate that is pumped to and incorporated in the N-enriched liquid fertilizer.

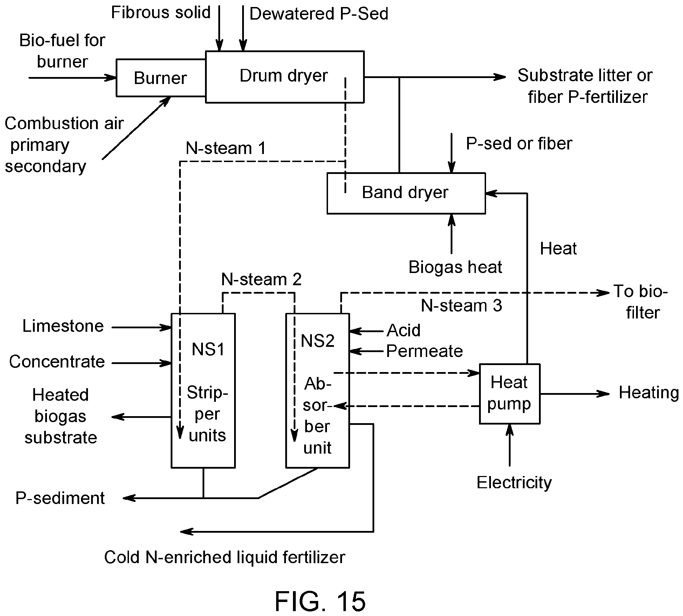

   P-sediment is finally dried in the drum dryer and/or band dryer and converted to high valuable dry P-fertilizer.

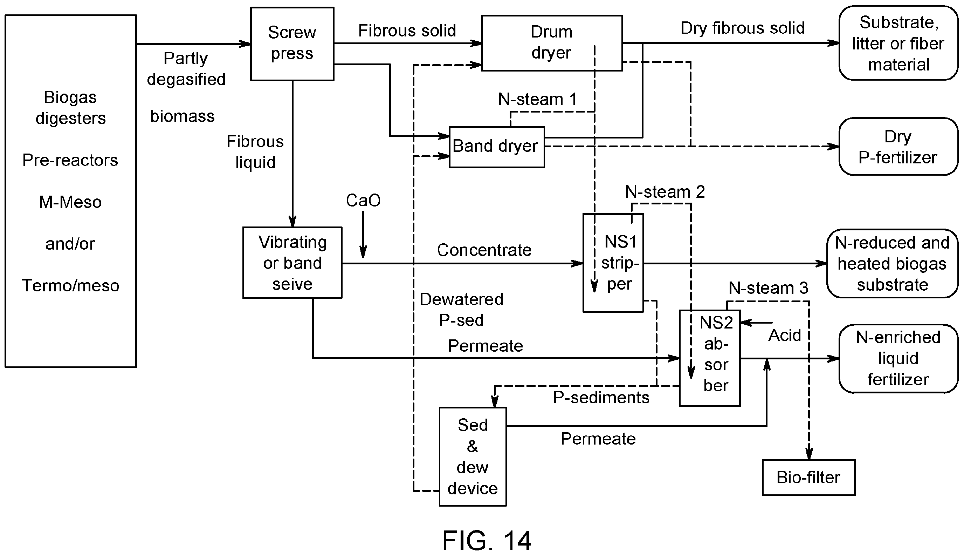

Fig. 14 shows production flow from degasified material to final products. The figure basically shows the same flow as figure 13 but the focus is different. In figure 14 the focus is on illustration of the flow related to the used terms and the shift in terms from partly degasified biomass to the final products:
   a) substrate, litter or dry fiber materials
   b) dry P-fertilizer
   c) N-reduced and heated biogas substrate
   d) N-enriched liquid fertilizer
Fig. 15 shows the energy flow - drying, N-stripping, N-absorption and drying. The figure shows how the energy obtained from burning bio-fuel in the burner for drum dryer is reused for heating up N-strippers and recovered from N-absorbers by heat pump and then recycled back for drying purposes in the band dryer or used for other heating purposes.
   Bio-fuels like wood chips or wood waste are burned in the bio-fuel burner as heating source for the drum dryer. The energy needed is directly related to the quantity of water to be evaporated and the type of dryer. In a drum dryer will be needed between 1.0 and 1.1 MWh per ton of water evaporated and for a band dryer the need is from 1.1 to 1.2 MWh per ton water evaporated.
   A part of the energy is lost during drying and another part will follow the dried material - substrate, litter, fibre and P-fertilizer that will leave the dryer with a temperature of 80 to 90°C.
   The main part of the energy - 70 to 75% - will be in the exhaust steam or in this context the N-steam 1 that is injected and used for heating up the concentrate in NS1. Here again a part of energy will be lost during N-stripping and another part will follow the heated biogas substrate and used for heating up biomasses and biogas reactors. The rest - 60 to 70% - will be in the N-steam 2 that is transferred to NS2. In NS2 a bigger part of the energy will be recovered by use of heat pump and reused for drying purposes or for other heating purposes. A part will be transferred with N-steam 3 to the bio-filter and lost.

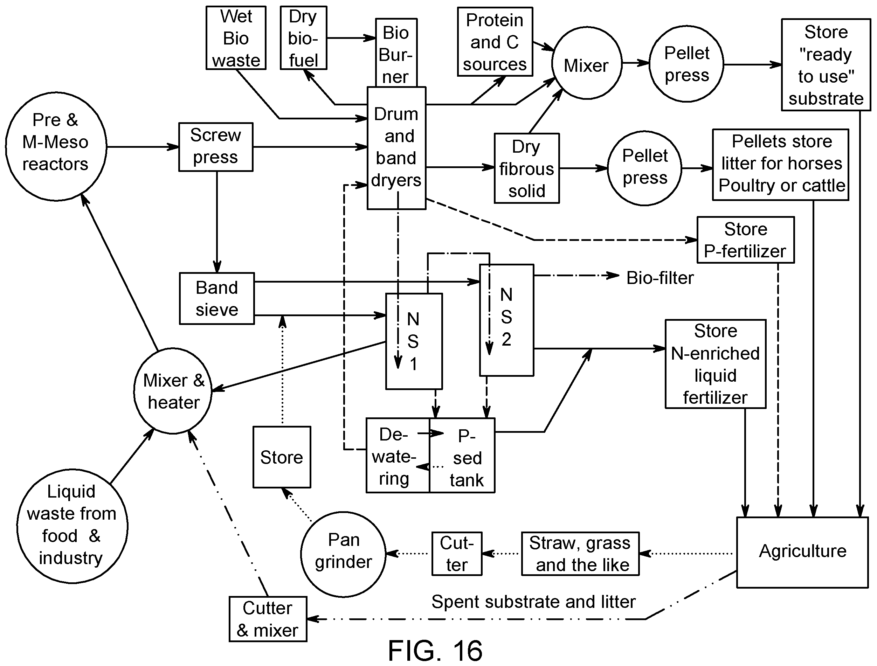

Fig 16 is an overview on circular system with focus on efficient utilization of energy and nutrients potentials in biomass. The figure illustrates the circular system or value chain based on high efficiency in utilization of energy and nutrient potentials in biomass and organic waste products from agriculture and food industry. The figure has to be seen as extension of the contents in figures 13-15 and focus in the following is therefore on what is outside of the marked grey zone.

Starting in the left upper corner is shown a box with "Wet bio waste". The term "wet bio waste" covers biomass with content of macromolecular constituents, including cellulose, hemicellulose and lignin. A part of the "wet bio waste" - saw dust and wood chips - is after drying suitable to be stored and used as bio-fuel for drying or used as "C source" in production of "ready to use substrate" for mushroom production. Other sources like residues from production of sugar, vegetable oils and the like can after drying be stored and used as "protein and C source" in production of "ready to use substrate" for mushroom production.

In the upper right corner focus is on production of a "ready to use" substrate e.g. for production of more types of mushroom. The "ready to use" term is to be understood as a substrate that can be stored until time of use and when used it is easy to handle - just add water and mycelium.

After production the spent mushroom substrate with its content of organic dry matter and nutrients can be recycled back and used as feed stock for biogas production as shown in the bottom of the figure.

Returning back to the upper right corner of the figure is shown another potential use of the dry fibrous solid for production of litter pellets for animals such as horses, poultry or cattle. Here the raw material is 100% dry fibre solids from partly degasified biomass. After use in agriculture the litter pellets with additional manure and urine from animals can be recycled back and used as feed stock for biogas in parallel with spent mushroom substrate.

Other types of residues from agriculture - straw, grass and the like - are also integrated as inputs in the value chain. Special attention in relation to this is the combined pre-treatment method based on cutting, grinding and cooking that will open up cellulose, hemicellulose and lignin and increase efficiency in utilization of energy potentials and allow higher dry matter content in the mix of biomasses to be added to the M-Meso and/or Pre reactors.

The cutting takes place in a standard cutter adjusted to a maximum length after cutting of 5 cm and in this way a part of structure in the added biomass is maintained.

The grinding takes place in a standard pellet press and die with 16 mm or bigger holes and a press way that secures a density of the granulate of 250 to 300 kg per m3 and a moisture content of 75 to 85%.

The granulate can be stored in big bags, in a silo or just on a flour and a time of use added to the concentrate and pumped to NS1. In NS1 it will be heated up to 80°C or more in a fibrous liquid with pH of 9.5 or more and content of both CO₂ and ammonium.

### Detailed description

The present invention is as defined in the claims.

The present disclosure relates to a fibrous solid substrate in pellet form obtained from a biomass material from a biogas fermenter following an anaerobic fermentation and biogas production.

Specifically, the fibrous solid substrate in pellet form is obtained by a method comprising the steps of
a. providing a biomass material comprising solid and liquid parts from a biogas fermenter following an anaerobic fermentation and biogas production,
b. subjecting the biomass material of step a. to one or more separation steps resulting in the provision of i) a fibrous solid fraction comprising organic and inorganic nitrogen parts and having a reduced content (w/w) of water, and comprising one or more macromolecular nutrient constituents selected from the group consisting of cellulose, hemicellulose, lignin and lignocellulose, and ii) at least one liquid fraction comprising solid and liquid organic and inorganic phosphor-containing parts,
c. subjecting the fibrous solid fraction of step b. to a sanitation treatment comprising one or more sanitation steps,
   wherein said sanitation treatment i) reduces or eliminates viable microorganisms present in the fibrous solid fraction, and/or ii) reduces the contents of volatile nitrogen-containing compounds and/or precursor volatile compounds present in the fibrous solid fraction, and
d. obtaining a fibrous solid fraction comprising organic and inorganic nitrogen parts having a reduced content of volatile nitrogen-containing compounds,
e. optionally subjecting said fibrous solid fraction to one or more of heating, drying, evaporation, pressure, and/or alkaline pH conditions,
f. optionally adding to said fibrous solid fraction one or more solid and/or liquid supplemental nutrient substrate compositions, and
g. compressing said fibrous solid fraction of step d., e. and/or f.,
   thereby generating a fibrous solid substrate in pellet form.

In one embodiment according to the present disclosure no non-fermented biomass and/or water is added to the fibrous solid fraction of step c. In one embodiment according to the present disclosure the fibrous solid fraction of step c. is not allowed to compost.

In one embodiment according to the present disclosure said one or more sanitation steps of step c. comprises i) heating the fibrous solid fraction to a temperature of more than 70°C, optionally under alkaline pH conditions, and optionally ii) subjecting the fibrous solid fraction comprising organic and inorganic nitrogen parts to a pressure of more than 1 bar.

In one embodiment according to the present disclosure the fibrous solid substrate in pellet form is compressed to achieve a specified density and having a certain diameter and moisture content.

Compressing the fibrous solid substrate decreases the volume of the fibrous solid substrate with associated advantages with respect to transportation, handling and storage as compared to a non-compressed fibrous solid substrate, and provided a ready-to-use substrate for many applications.

The compressed pellets disclosed herein can be used in many areas of agriculture e.g. as litter for animals or as a substrate for cultivating mushrooms.

Also disclosed herein are methods for processing a partly degasified biomass obtained from an anaerobic biogas fermentation, i.e. a spent biomass material, into solid and liquid fractions and further processing said liquid fractions into high value fertilizer products. In particular are disclosed combined methods for stripping ammonia N from one or more fractions and absorbing ammonia N in a liquid fraction obtained from separation of spent biomass material thereby generating one or more N and/or P-fertilizers.

In one aspect of the present disclosure there is provided a method for fractionating a partly degasified biomass material and obtaining a) a fibrous solid fraction comprising solid and liquid parts, said fibrous solid fraction further comprising organic and inorganic nitrogen parts, b) a fibrous liquid fraction or concentrate comprising solid and liquid parts and further comprising organic and inorganic nitrogen parts, c) a non-fibrous liquid fraction or permeate comprising mainly inorganic nitrogen parts and d) a phosphor (P) comprising fraction or sediment.

In a further aspect of the present disclosure there is provided a method for increasing the relative amount of inorganic nitrogen content of a liquid fraction comprising organic and inorganic nitrogen parts and providing an N enriched liquid fertilizer.

In a still further aspect of the present disclosure there is provided a method for reducing the content of water and inorganic nitrogen compounds in a phosphor (P) fraction or sediment comprising organic and inorganic nitrogen parts of a biomass material comprising organic and inorganic nitrogen parts and providing a dry P fertilizer.

In one aspect according to the present disclosure, there is provided a method for generating one or more fertilizers, such as one or more N or P-fertilizers comprising the steps of:
i) providing a partly degasified biomass material comprising solid and liquid nitrogen (N) and phosphor (P) containing parts from a biogas fermenter following an anaerobic fermentation and biogas production,
ii) subjecting the partly degasified biomass material to one or more separation steps resulting in the provision of
   a) a fibrous solid fraction comprising organic and inorganic nitrogen (N) parts, and
   b) at least one liquid fraction comprising solid and liquid organic and inorganic nitrogen (N) and phosphor (P) containing parts,
i) subjecting said liquid fraction to one or more separation steps, resulting in the provision of
   a) a fibrous liquid fraction or concentrate, and
   b) an essentially non-fibrous liquid fraction or permeate,
ii) subjecting the essentially non-fibrous liquid fraction or permeate to a gaseous fraction comprising nitrogen containing volatile compounds, wherein the pH and temperature are individually adjusted to shift the balance from ammonia to ammonium, thereby generating
   a) an N-comprising fraction or N-fertilizer in liquid form,
   b) a P-comprising fraction or sediment,
   c) a heating source suitable for drying and other heating purposes,
iii) optionally subjecting the P-comprising fraction or sediment to a heating and drying treatment sufficient to evaporate water and volatile nitrogen (N) containing compounds, and thereby generating
   a) a P-comprising fraction or P-fertilizer.

Processing of biomass materials prior to anaerobic biogas fermentation and stripping of ammonia N prior to performing the biogas fermentation is not always sufficient to preclude an undesirable inhibition of biogas-producing bacteria by ammonia not stripped during the pre-treatment step.

There is a need for improving the processing and removal of volatile solids from biomasses prior to such biomasses being subjected to pre-treatment steps e.g. involving thermo-chemical processing steps, such as e.g. lime pressure cooking.

There is also a need for novel and innovative methods for stripping ammonia N from different biomasses such as biomass materials having a high N content.

Furthermore, there is a need for improving the efficiency of anaerobic biogas fermentations and their capability of utilizing macromolecular nutrient constituents selected in particular from cellulose, hemicellulose, lignin and lignocellulose.

The present disclosure facilitates efficient biomass processing and an increased production of renewable energy from processing and anaerobic fermentation of a wide variety of biomass materials.

Many types of biomass/ organic materials have a high energy potential which may be exploited by processing the biomass material. One form of processing an organic material is by performing an anaerobic fermentation resulting in the production of biogas. This process represents a conversion of an energy potential to a readily usable energy source.

There are four key biological and chemical stages of an anaerobic fermentation: Hydrolysis; acidogenesis; acetogenesis; and methanogenesis. In order for bacteria under anaerobic conditions to exploit the energy potential of the organic materials used as substrates, macromolecules present in the biomass materials must initially be broken down into their smaller constituent parts. The process of breaking the macromolecular structures initially involves a hydrolysis and/or an oxidation of the macromolecular structures. Breaking down macromolecular constituents present in the biomass materials can advantageously take place e.g. during a pre-treatment processing step prior to anaerobic fermentation and biogas production.

The resulting constituent parts, including monomers and oligomers, such as degraded constituents comprising sugar and/or amino acid residues, can be more readily metabolized by microbial organisms involved in one or more of the steps of acidogenesis; acetogenesis; and methanogenesis.

Acetate and hydrogen produced in the first stages of an anaerobic fermentation can be used directly by methanogens. Other molecules, such as volatile fatty acids (VFAs) with a chain length that is greater than that of acetate, must first be catabolised into compounds that can be directly metabolised by methanogens.

The biological process of acidogenesis further breaks down remaining components by acidogenic (fermentative) bacteria. Here, VFAs are created along with ammonia, carbon dioxide, and hydrogen sulfide, as well as other by-products.

The third stage of an anaerobic fermentation is acetogenesis. Here, simple molecules created through the acidogenesis phase are further digested by acetogens to produce largely acetic acid as well as carbon dioxide and hydrogen.

The final stage of an anaerobic fermentation is that of methanogenesis. Methanogens metabolise intermediate compounds formed during the preceding stages of the anaerobic fermentation, and these compounds are metabolised into methane, carbon dioxide, and water. The afore-mentioned compounds are the major constituents of a biogas. Methanogenesis is sensitive to both high and low pHs - and methanogenesis generally occurs between pH 6.5 and pH 8.

Remaining, non-digestible organic material that the microbes present in the biogas fermenter may not metabolise, along with any dead bacterial remains, constitutes the digestate from the fermentation in the form of a fibrous solid fraction which can be further separated from fermentations liquids and processed as disclosed herein below.

Apart from having a high energy potential, many biomass materials also have a high content of nitrogen (N). When such organic materials are used as substrates for converting organic materials into bio energy in a bio energy plant, in particular biogas in a biogas plant, the organic N will gradually be converted to ammonia.

The formation of ammonia in a bio energy plant - especially at high levels - represents a problem as many biogas producing bacteria are sensitive to high levels of ammonia - and high ammonia levels in a biogas fermenter will thus reduce or inhibit the production of methane. Ultimately, the formation of high levels of ammonia will kill biogas producing bacteria and inhibit any further biogas formation.

The inhibitory levels of ammonia in a biogas fermenter depend on the conditions used. Under thermophilic fermentation conditions, approx. 3.8 to 4.2 kg ammonia per ton of biomass such as 4.0 kg is considered inhibitory if the biomass has been thermo- chemically pre-treated with added limestone, else in absence of any thermo-chemically pre-treatment the inhibitory level is approx. 3.4 to 3.6 kg ammonia per ton of the biomass such as 3.5 kg. Under mesophilic fermentation conditions the figure is approx. 5.5 to 6.0 kg ammonia per ton of biomass such as 5.8 kg is considered inhibitory if the biomass has been thermo-chemically pre-treated with added limestone, else in absence of any thermo-chemically pre-treatment the inhibitory level is approx. 4.4 to 5.0 kg ammonia per ton of biomass such as 4.7 kg. The biogas fermentation process may be expected to be completely inhibited at ammonia levels of approx. 7.0 kg to 7.5 kg ammonia per ton of biomass such as 7.2 kg ammonia per ton of biomass.

The above-cited ammonia inhibition threshold values are generally taken into consideration when operating commercial biogas plants using conventional organic materials as substrates for the biogas producing bacteria. Many such plants are operated according to a two-step strategy initially adopting thermophile digestion conditions in a first step and mesophile digestion conditions in a separate and subsequent, second step.

The conversion of organic N to ammonia N progresses during an anaerobic fermentation process - i.e. during the process of generating biogas by anaerobic fermentation - and a conversion of as much as approx. 60 to 70 % of organic N to ammonia N may be expected.

Particular challenges arise when it is desirable to process organic materials having a particularly high organic N content - as inhibitory levels of ammonia during biogas fermentation can be expected to occur relatively early on in the fermentation process due to the high levels of organic N and protein in the organic material to be processed. Problems related to high organic N content in the basic raw materials are also well known in connection with production of mushroom substrate and normally this is solved by composting and mixing. Doing this means loose of valuable C content from the easy degradable part of volatile solids and in parallel part of N is lost.

Conventional production of substrate is based on the use of poultry manure, deep litter, straw, saw dust and similar types of complex organic waste with high cellulose, hemicellulose, lignin and / or organic N content.

Use of spent mushroom substrate as main feed stock in a conventional biogas plant is due to the high organic N content not possible, but in combination with other types of waste and biomasses, and when using the technologies and processes of the present disclosure, spent mushroom substrate is a valuable feed stock. This is illustrated in the examples.

Examples of organic materials high in N are e.g. sludge from cheese and offal stomach and gut products, and other tabled waste categories/ types. However, in order to perform such a mixture one needs to have access to organic materials low in N - and this poses a particular challenge with respect to mixing facilities and the availability in general of particular types of organic materials which will need to be available for "dilution" of organic materials containing high levels of organic N and / or protein. Consequently, it is not always possible to perform such a mixture of different organic materials and even if it should be possible, it imposes certain practical restrictions on the organic materials to be processed by anaerobic fermentation.

The present disclosure further provides technical solutions to the problem of how to adjust and control moisture content and content of inorganic N as well as organic N and in parallel it provides technical solutions on the problem of how to improve biogas production in a commercial biogas plant. The solutions involves novel and inventive methods for reducing organic N and protein in an organic material further comprising at least one carbon (C) source, either prior to performing an anaerobic fermentation resulting in the production of biogas, or during the progress of an anaerobic fermentation.

The anaerobic fermentation resulting in the production of biogas is followed by one or more processing steps aimed to strip ammonia N from the organic material after to the biogas production from pre-digestion in fermentation facilities, heating and drying. This is illustrated in the examples.

The present disclosure in a first aspect generally relates to a combined method for manufacturing and recycling a fibrous solid fraction obtained from an anaerobic biogas fermentation for cultivating Basidiomycete cells, and subsequently using the spent Basidiomycete substrate as a feed stock biomass material in the anaerobic biogas fermentation from which the fibrous solid fraction used for the cultivation of the basidiomycete cells was obtained.

Once the fibrous solid substrate has been obtained from an anaerobic biogas production, one can contact the fibrous solid substrate suitable for cultivating Basidiomycete cells with one or more species of Basidiomycete cells, or spores, and cultivate said Basidiomycete cells, or spores, in said fibrous solid substrate in accordance - in one embodiment according to the present disclosure - with state-of-the-art cultivation protocols. Examples of such protocols are presented herein elsewhere.

It may be required to add to said thus obtained fibrous solid fraction comprising organic and inorganic nitrogen parts one or more solid and/or liquid supplemental nutrient substrate compositions, thereby generating a final, ready-to-use fibrous solid substrate suitable for cultivating Basidiomycete cells. Also, an adjustment of the pH of the liquid parts of the final, ready-to-use fibrous solid substrate suitable for cultivating Basidiomycete cells may be required, said adjustment resulting in a final pH value of from about 5.0 to about 7.5.

Accordingly, in one embodiment according to the present disclosure, the above-cited method further comprises the steps of adding to said fibrous solid fraction comprising organic and inorganic nitrogen parts one or more solid and/or liquid supplemental nutrient substrate compositions, thereby generating a final, ready-to-use fibrous solid substrate suitable for cultivating Basidiomycete cells, and/or adjusting the pH of the liquid parts of the final, ready-to-use fibrous solid substrate suitable for cultivating Basidiomycete cells may be required, said pH adjustment resulting in a final pH value of from about 5,0 to about 7.5.

The fibrous solid substrate suitable for cultivating Basidiomycete cells preferably comprises one or more macromolecular nutrient constituents selected from the group consisting of cellulose, hemicellulose and lignin. Lignocellulose in the form of a feed stock biomass material collectively comprising cellulose, hemicellulose and lignin as macromolecular constituents is another example of biomass materials and macromolecular nutrient constituents difficult for microbial organisms involved in one or more stages of an anaerobic fermentation and biogas production to digest.

The early stages of a biogas fermentation includes an initial stage of hydrolysis of macromolecular nutrient constituents into their basic constituents, or into nutrient constituents which can more readily be metabolized and fermented by the microbial organisms involved in one or more stages of an anaerobic fermentation and biogas production.

Metabolism of nutrient constituents is essential for the production of biogas as no fermentable and energy generating microbial activities can be carried out in the absence of such metabolism.

It is a particular challenge during an anaerobic biogas fermentation that no, or an insufficient hydrolysis takes place of macromolecular nutrient constituents into their basic constituents, or into nutrient constituents which can more readily be metabolized and fermented by the microbial organisms involved in one or more stages of an anaerobic fermentation and biogas production.

Accordingly, the fibrous solid substrate suitable for cultivating Basidiomycete cells with one or more species of Basidiomycete cells or spores will contain - as a result of an insufficient hydrolysis of macromolecular nutrient constituents into their basic constituents, or into nutrient constituents which can more readily be metabolized and fermented by the microbial organisms involved in one or more stages of an anaerobic fermentation and biogas production - one or more macromolecular nutrient constituents preferably selected from the group consisting of cellulose, hemicellulose and lignin, as well as lignocellulose, a biomass material collectively comprising cellulose, hemicellulose and lignin, as such macromolecular nutrient constituents are difficult, if not impossible, for many, if not all, microbial organisms involved in one or more stages of an anaerobic fermentation and biogas production to digest.

However, many fungal organisms, including many Basidiomycetes, are capable of digesting macromolecular nutrient constituents preferably selected from the group consisting of cellulose, hemicellulose and lignin, as well as lignocellulose, as many fungal organisms produce and secrete extracellular enzymes for which said macromolecular nutrient constituents form a substrate.

Accordingly, the Basidiomycete cell can be selected from any of the subclasses of Agaricomycetidae, Exobasidiomycetidae, Tremellomycetidae and Ustilaginomycetidae, as long as the Basidiomycete cell in question is able to degrade or digest one or more macromolecular nutrient constituents preferably selected from the group consisting of cellulose, hemicellulose and lignin, as well as lignocellulose.

Preferred Basidiomycete cells are those which are edible, such as for example a cell selected from the genera of Agaricus, Lentinula (Lentinus), Flammulina, Pleurotus; and Lyophyllum. Additionally, and more specifically, the Basidiomycete cell can be selected from the species of Lentinula (Lentinus) edodes; edible Agaricus species, such as e.g. Agaricus blazei Murill (AbM), a.k.a. Agaricus subrufescens Peck, a.k.a. Agaricus brasiliensis Wasser, and Agaricus bisporus, Flammulina velutipes (Enokitake), Pleurotus eryngii (Eryngii), Pleurotus ostreatus; and Lyophyllum shimeji (Shimeji).

Hence, by recycling once, or more than once, in any order, a) anaerobic biogas fermentation methods exploiting, as a feed stock biomass material, spent fungal substrate, and b) Basidiomycete cultivation methods using a fibrous solid fraction from spent, anaerobically fermented biomass material as a substrate for cultivation of said Basidiomycetes; one can effectively recycle and utilize more efficiently all of the above-mentioned macromolecular nutrient constituents present in a biomass material to be used for both anaerobic biogas fermentation and the cultivation of fungal species.

Suitable fungal organisms can be selected from organisms constituting the phylum Basidiomycota of the kingdom Fungi, or, in older classification schemes, the class Basidiomycetes of the kingdom Plantae, i.e. fungal organisms characterized by bearing the spores on a basidium, including the edible mushrooms described herein elsewhere in more detail. Preferred Basidiomycetes are those genera and species producing extracellular enzymes capable of digesting one or more macromolecular nutrient constituents selected from cellulose, hemicellulose, lignin and lignocellulose. Among the genera and species of preferred Basidiomycetes, genera and species of Basidiomycetes which are edible are particularly preferred.

The combined and sequential re-use of degassed biomass materials and spent mushroom substrates, respectively, is disclosed in various aspects of the present disclosure as will be clear from the below disclosure of the present disclosure. Spent fungal substrate diverted to an anaerobic biogas fermentor as described above is supplemented with additional organic waste biomass materials prior to biogas fermentation in order to provide a more optimal feed stock biomass material and in order to ensure continued utilization of suitable and preferred sources of biodegradable and fermentable biomass materials. The solid feed stock biomass materials entering an anaerobic biogas fermenter in this way may be diluted to a suitable content of total solids by adding to said solid feed stock biomass materials any suitable liquid dilution or suspension source, such as e.g. liquids obtained when fragmenting and draining a spent, fermented and degassed biomass material in order to obtain a fibrous solid fraction. Slurries of manures can also be accepted for this purpose.

The fibrous solid substrate having been obtained from anaerobic biogas fermentation preferably comprises one or more macromolecular nutrient constituents selected from the group consisting of cellulose, hemicellulose and lignin, or in the form of lignocellulose. The fibrous solid substrate can also comprise one or more of such macromolecular constituents, preferably more than one macromolecular constituent, such as two or three macromolecular constituents selected from the group consisting of cellulose, hemicellulose and lignin, wherein said macromolecular constituents are not, or only partly, metabolized by anaerobic bacteria involved in the production under anaerobic fermentation conditions of biogas, and can be at least partly metabolized by said Basidiomycete cells contacted with the fibrous solid substrate.

The digestion by in particular extracellular Basidiomycete enzymes of said macromolecular constituents results in a hydrolysis and/or an oxidation of at least part of said macromolecular constituents, wherein said hydrolysis and/or oxidation of at least part of said macromolecular constituents in turn generates a substrate capable of being fermented by microbial organisms involved in one or more stages of a biogas fermentation, wherein said microbial organisms involved in said one or more stages of a biogas fermentation preferentially metabolises the hydrolysis and/or oxidation products resulting from the hydrolysis and/or oxidation of said macromolecular constituents, and less preferentially metabolises said macromolecular constituents, including cellulose, hemicellulose and lignin, or wherein said microbial organisms involved in said one or more stages of a biogas fermentation are essentially unable to metabolise said macromolecular constituents, including cellulose, hemicellulose and lignin.

The general lack of hydrolysis of said macromolecular constituents, including cellulose, hemicellulose and lignin, by methanogenic and other anaerobic bacteria involved in the production of biogas under anaerobic fermentation conditions, will result in said macromolecular constituents being present during an anaerobic biogas fermentation during one or more stages of the anaerobic biogas fermentation, including the stages selected from acidogenesis, acetogenesis and methanogenesis.

A method for manufacturing a fibrous solid substrate suitable for cultivating fungal cells In one aspect of the present disclosure there is provided a method for manufacturing a fibrous solid substrate suitable for cultivating fungal cells, such as Basidiomycetes, said method comprising the steps of
i) providing a biomass material comprising solid and liquid parts from a biogas fermenter following an anaerobic fermentation and biogas production (a degassed or partly degassed biomass),
ii) subjecting the biomass material to one or more separation steps resulting in the provision of a fibrous solid fraction comprising organic and inorganic nitrogen parts and at least one liquid fraction, said liquid fraction optionally comprising solid and liquid organic and inorganic phosphor (P) containing parts,
iii) subjecting the fibrous solid fraction to a sanitation treatment comprising one or more sanitation steps, wherein said sanitation treatment a) reduces or eliminates viable microorganisms present in the fibrous solid fraction, and/or b) reduces the contents of volatile nitrogen-containing compounds and/or precursor volatile compounds present in the fibrous solid fraction,
iv) obtaining a fibrous solid fraction having a reduced content of nitrogen-containing compounds (and/or an ammonia-stripped and/or sanitised fibrous solid fraction) suitable for use as a fibrous solid substrate for cultivating fungal (such as Basidiomycete) cells, and
v) optionally adding to said fibrous solid fraction one or more solid and/or liquid supplemental nutrient substrate compositions, thereby generating a fibrous solid substrate suitable for cultivating fungal (such as Basidiomycete) cells.

In one embodiment according to the present disclosure 'a biomass material comprising solid and liquid parts from a biogas fermenter following an anaerobic fermentation and biogas production' according to the disclosure is a degassed or partly degassed biomass, such as a biomass material which has been subject to anaerobic fermentation thereby producing a biogas and a degassed biomass material comprising organic and inorganic nitrogen parts.

### A method for cultivating fungal cells and/or spores on a fibrous solid substrate

In another aspect of the present disclosure there is provided a method for cultivating fungal cells, including Basidiomycete cells, and/or spores, on a fibrous solid substrate, said method comprising the steps of
i) providing fungal (such as Basidiomycete) cells and/or spores,
ii) providing a fibrous solid substrate for cultivating fungal (such as Basidiomycete) cells and/or spores obtained by any of the methods of the present disclosure disclosed herein,
iii) contacting the fungal (such as Basidiomycete) cells and/or spores provided in step i) with the fibrous solid substrate provided in step ii),
iv) cultivating the fungal (such as Basidiomycete) cells and/or spores in said substrate,
v) obtaining spent fungal substrate (or spent fungal substrate biomass material), and
vi) optionally collecting the spent fungal substrate, wherein said spent fungal substrate is at least partially digested by the cultivation of the fungal cells and is suitable as a feed stock for an anaerobic fermentation and biogas production.

A method for recycling biomass materials comprising organic and inorganic nitrogen parts selected from spent fungal substrate biomass materials and degassed, fermented biomass materials

In yet another aspect of the present disclosure there is provided a method for recycling biomass materials comprising organic and inorganic nitrogen parts selected from 1) spent fungal substrate biomass materials and 2) degassed fermented biomass materials, wherein said method is for
a) recycling, more than once, spent fungal substrate biomass material from a fungal cultivation to, and reusing said substrate biomass material in an anaerobic biogas fermentation taking place in an anaerobic biogas fermenter, said fermentation resulting in the production of biogas and a degassed, fermented biomass material for recycling, and/or
b) recycling, more than once, a fibrous solid fraction of a degassed fermented biomass material from said anaerobic biogas fermenter to a fungal cultivation facility, and reusing said degassed, fermented biomass material fibrous solid fraction from said anaerobic biogas fermenter to cultivate said fungus, said fungus cultivation resulting in the provision of fungus and a spent fungal substrate biomass material for recycling,
said method comprising the steps of
i) cycling, more than once, spent fungal substrate biomass material from a fungal cultivation to, and reusing said substrate biomass material in, an anaerobic biogas fermentation taking place in an anaerobic biogas fermenter,
   said fermentation resulting in the production of biogas and a degassed fermented biomass material suitable for use as a substrate for cultivating fungal cells and/or spores, and
ii) cycling, more than once, a fibrous solid fraction of a degassed fermented biomass material from said anaerobic biogas fermenter to a fungal cultivation facility, and reusing said degassed fibrous solid fraction from said anaerobic biogas fermenter to cultivate said fungal cells and/or spores,
   said fungal cultivation resulting in the provision of fungal cells and/or spores and a spent fungal substrate biomass material suitable for use as a feed stock biomass material in an anaerobic biogas fermentation,
iii) fractionating the degassed fermented biomass material by subjecting the degassed fermented biomass material to one or more separation steps as disclosed herein elsewhere,
thereby obtaining a) a fibrous solid fraction comprising solid and liquid parts, said fibrous solid fraction further comprising organic and inorganic nitrogen parts, and b) at least one liquid fraction comprising solid and liquid parts,
wherein the anaerobic biogas fermenter is optionally supplemented by addition of further anaerobically fermentable organic waste biomass materials, such as in the form of supplementary feed stock biomass materials.

A method for controlling the composition of nutrients and/or the moisture content of a fibrous solid fraction comprising organic and inorganic nitrogen parts suitable for cultivating fungal cells

In a still further aspect of the present disclosure there is provided a method for controlling the composition of nutrients and/or the moisture content of a fibrous solid fraction comprising organic and inorganic nitrogen parts suitable for cultivating fungal such as Basidiomycete cells and/or spores, said method comprising the steps of
i) providing a biomass material comprising solid and liquid parts from a biogas fermenter following an anaerobic fermentation and biogas production,
ii) subjecting the biomass material to one or more separation steps resulting in the provision of a fibrous solid fraction comprising organic and inorganic nitrogen parts and at least one liquid fraction,
iii) evaporating, under predetermined conditions, from said fibrous solid fraction an aqueous gas further comprising one or more volatile compounds,
iv) obtaining a fibrous solid fraction comprising organic and inorganic nitrogen parts from which said one or more volatile compounds have been removed by evaporation,
   wherein the fibrous solid fraction is suitable for use as a fibrous solid substrate for cultivating fungal such as Basidiomycete cells, and
v) optionally adding to the fibrous solid fraction comprising organic and inorganic nitrogen parts obtained in step iv) one or more solid and/or liquid supplemental nutrient substrate compositions, thereby generating a fibrous solid substrate suitable for cultivating fungal such as Basidiomycete cells,
   wherein at least one of said one or more nutrients can be stripped as volatile compounds from said fibrous solid fraction comprising organic and inorganic nitrogen parts by evaporation under said predetermined conditions,
   wherein said one or more nutrients are converted into one or more volatile compounds, and
vi) controlling the composition of nutrients and/or the moisture content of the fibrous solid substrate by converting said one or more nutrients into one or more volatile compounds and evaporating said one or more volatile compounds as aqueous gasses from said fibrous solid fraction comprising organic and inorganic nitrogen parts under predetermined evaporation conditions.

A method for separating and drying a biomass material comprising organic and inorganic nitrogen parts, and providing a fibrous solid substrate suitable for cultivating fungal cells

The above-cited aspects of the present disclosure all employ the step of providing a fibrous solid fraction from a degassed, fermented biomass material having been fermented under anaerobic fermentation conditions.

Accordingly, the present disclosure in yet another aspect provides a method for separating and drying a biomass material comprising organic and inorganic nitrogen parts, and providing a fibrous solid substrate suitable for cultivating fungal such as Basidiomycete cells, said method comprising the steps of
i) providing a biomass material comprising solid and liquid parts from a biogas fermenter following an anaerobic fermentation and biogas production,
ii) subjecting the biomass material to one or more separation steps resulting in the provision of a fibrous solid fraction comprising organic and inorganic nitrogen parts and at least one liquid fraction,
iii) subjecting said fibrous solid fraction comprising organic and inorganic nitrogen parts to heating sufficient to evaporate, under predetermined evaporation conditions, volatile compounds present in the fibrous solid fraction comprising organic and inorganic nitrogen parts as volatile compounds, or sufficient to converting volatile precursor compounds into volatile compounds capable of being evaporated under predetermined conditions,
iv) evaporating said one or more volatile compounds under predetermined evaporation conditions characterized at least by heating the fibrous solid fraction to a temperature of at least 70°C under alkaline pH conditions and at a pressure sufficient to evaporate said volatile compounds, and a. providing (obtaining) a) a fibrous solid fraction comprising organic and inorganic nitrogen parts and having an increased content (w/w) of organic nitrogen compounds, a reduced content (w/w) of volatile compounds, or volatile precursor compounds, and a reduced content (w/w) of water, and b) an aqueous gas further comprising one or more volatile compounds, including ammonia,
thereby separating and drying a biomass biomaterial and providing a fibrous solid substrate suitable for cultivating fungal cells.

A method for reducing the content of inorganic nitrogen compounds in a fibrous solid fraction comprising organic and inorganic nitrogen parts

In a still further aspect of the present disclosure there is provided a method for reducing the content of inorganic nitrogen compounds in a fibrous solid fraction comprising
organic and inorganic nitrogen parts of a biomass material comprising organic and inorganic nitrogen parts and providing a fibrous solid substrate suitable for cultivating fungal such as Basidiomycete cells, said method comprising the steps of
i) providing a biomass material comprising solid and liquid parts from a biogas fermenter following an anaerobic fermentation and biogas production,
ii) subjecting the biomass material to one or more separation steps resulting in the provision of a fibrous solid fraction comprising organic and inorganic nitrogen parts and at least one liquid fraction,
iii) subjecting said fibrous solid fraction comprising organic and inorganic nitrogen parts to heating sufficient to evaporate volatile inorganic nitrogen compounds present in the fibrous solid fraction comprising organic and inorganic nitrogen parts as volatile inorganic nitrogen compounds, or in the form of volatile inorganic nitrogen precursor compounds capable of being evaporated under predetermined conditions,
iv) converting said inorganic nitrogen compounds to gaseous nitrogen containing volatile compounds, including ammonia,
v) evaporating said gaseous nitrogen containing volatile compounds, including ammonia,
wherein said conversion and evaporation generates a fibrous solid fraction comprising organic and inorganic nitrogen parts and having a reduced content of inorganic nitrogen compounds.

A method for increasing the relative amount of organic nitrogen content of a fibrous solid fraction comprising organic and inorganic nitrogen parts of a biomaterial following fermentation and biogas production

In a still further aspect of the present disclosure there is provided a method for increasing the relative amount of organic nitrogen content of a fibrous solid fraction comprising organic and inorganic nitrogen parts of a biomass material following fermentation and biogas production, said method comprising the steps of
i) providing a biomass material comprising solid and liquid parts from a biogas fermenter following an anaerobic fermentation and biogas production,
ii) subjecting the biomass material to one or more separation steps resulting in the provision of a fibrous solid fraction comprising organic and inorganic nitrogen parts and at least one liquid fraction,
iii) subjecting said fibrous solid fraction comprising organic and inorganic nitrogen parts to heating sufficient to evaporate volatile inorganic nitrogen compounds present in the fibrous solid fraction comprising organic and inorganic nitrogen parts as volatile inorganic nitrogen compounds, or in the form of volatile inorganic nitrogen precursor compounds capable of being evaporated under predetermined conditions,
iv) converting said inorganic nitrogen compounds to gaseous nitrogen containing volatile compounds, including ammonia,
wherein said conversion and evaporation generates a fibrous solid fraction comprising organic and inorganic nitrogen parts and having an increased, relative amount of organic nitrogen.

A method for fractionating a biomass material and obtaining a) a fibrous solid fraction comprising solid and liquid parts, said fibrous solid fraction further comprising organic and inorganic nitrogen parts, b) at least one liquid fraction comprising solid and liquid parts, and c) a phosphor-containing fraction or sediment

In yet another aspect of the present disclosure there is provided a method for fractionating a biomass material and obtaining a) a fibrous solid fraction comprising solid and liquid parts, said fibrous solid fraction further comprising organic and inorganic nitrogen parts, b) at least one liquid fraction comprising solid and liquid parts, and c) a phosphor-containing fraction or sediment, said method comprising the steps of
i) providing a biomass material comprising solid and liquid nitrogen (N) and phosphor-containing parts from a fermenter following an anaerobic fermentation,
ii) subjecting the biomass material to one or more separation steps and obtaining a fibrous solid fraction comprising solid and liquid organic and inorganic nitrogen containing parts, and at least one liquid fraction comprising solid and liquid organic and inorganic phosphor-containing parts,
iii) separating solid and liquid parts of the at least one liquid fraction by fractionation and/or sedimentation, and
iv) obtaining a) a fibrous solid fraction comprising solid and liquid parts comprising organic and inorganic nitrogen parts, b) a first solid, phosphor-containing fraction or sediment suitable for being used as, or added to, a phosphor-containing agricultural fertilizer, and c) a first liquid permeate fraction comprising solid and/or liquid nitrogen and/or phosphor-containing parts.

### A method for producing a biogas by anaerobic fermentation of a biomass material

In a still further aspect of the present disclosure there is provided a method for producing a biogas by anaerobic fermentation of a biomass material comprising the steps of
i) providing a biomass material suitable for anaerobic fermentation and biogas production,
ii) fermenting the biomass material under anaerobic fermentation conditions, thereby producing a biogas and a degassed biomass material comprising organic and inorganic nitrogen parts,
iii) collecting and/or storing the biogas,
   said method optionally comprising the further steps of
iv) fractionating the degassed biomass material and obtaining a fibrous solid fraction comprising solid and liquid parts, said fibrous solid fraction further comprising organic and inorganic nitrogen parts, at least one liquid fraction comprising solid and liquid parts, and a phosphor-containing fraction or sediment, said method comprising the further steps of
v) separating solid and liquid parts of the at least one liquid fraction comprising solid and liquid parts, and
vi) obtaining a) a fibrous solid fraction comprising solid and liquid parts,
wherein the fibrous solid fraction further comprises organic and inorganic nitrogen parts, b) a first solid, phosphor-containing fraction or sediment suitable for being used as, or added to, a phosphor-containing agricultural fertilizer, and c) a first liquid permeate fraction comprising solid and liquid parts.

### A method for producing biogas and gasses comprising volatile nitrogen containing compounds by sequential, anaerobic fermentations and ammonia stripping

In a yet further aspect of the present disclosure there is provided a method for producing biogas and gasses comprising volatile nitrogen containing compounds by sequential, anaerobic fermentations and stripping at least partly said volatile nitrogen containing compounds, including ammonia, from the fermented biomass materials comprising organic and inorganic parts, said method comprising the steps of
i) providing a first biomass material comprising organic and inorganic nitrogen parts,
ii) performing an initial, anaerobic fermentation of the first biomass material in an anaerobic fermenter,
iii) producing biogas and volatile nitrogen-containing compounds and a fermented, first biomass material under said anaerobic fermentation conditions,
   wherein said initial fermentation of the first biomass material under anaerobic fermentation conditions results in at least partly converting organic nitrogen parts into inorganic nitrogen parts,
   wherein said inorganic nitrogen parts comprise, or are converted into, gasses comprising volatile nitrogen-containing compounds during said initial,
   anaerobic fermentation,
iv) diverting the first fermented biomass material, and said gasses comprising volatile nitrogen containing compounds formed during said initial, anaerobic fermentation, to a stripper and sanitation tank,
v) stripping at least part of said gasses comprising volatile nitrogen-containing compounds from the first, fermented biomass material in the stripper and sanitation tank by heating to a temperature of at least 70oC at a pressure sufficient to strip said volatile compounds, thereby obtaining a second biomass material comprising a reduced amount of inorganic nitrogen parts,
vi) diverting said second biomass material having a reduced amount of inorganic nitrogen parts to a further anaerobic fermenter and subsequently fermenting said second biomass material under anaerobic conditions,
vii) producing under anaerobic fermentation conditions biogas and volatile nitrogen-containing compounds and a fermented, second biomass material comprising organic and inorganic nitrogen parts,
viii) wherein said subsequent anaerobic fermentation of the second biomass material results in at least partly converting organic nitrogen parts into inorganic nitrogen parts,
   wherein said inorganic nitrogen parts comprise, or are converted into, gasses comprising volatile nitrogen containing compounds during said subsequent, anaerobic fermentation,
ix) subjecting said fermented, second biomass material to one or more separation steps resulting in the formation of a fibrous solid fraction comprising organic and inorganic nitrogen parts and at least one liquid fraction,
x) subjecting said fibrous solid fraction to a temperature of at least 70°C at a pressure sufficient to strip said volatile compounds, and stripping volatile nitrogen containing compounds present in the solid fibrous fraction, thereby generating a fibrous solid fraction having a reduced amount of volatile nitrogen containing compounds and inorganic nitrogen precursor volatile compounds, including ammonium,
xi) forming a gaseous fraction comprising nitrogen containing volatile compounds, including ammonia, and having a temperature of at least 70°C, and
xii) diverting said gaseous fraction comprising nitrogen containing volatile compounds, and having a temperature of at least 70°C, to the stripper and sanitation tank of step iv) for stripping of said nitrogen containing volatile compounds,
wherein the diverted gaseous fraction comprising nitrogen containing volatile compounds, including ammonia, and having a temperature of at least 70°C, contributes to heating the fermented, first biomass material in
the stripper and sanitation tank, or a further first biomass material having been diverted to the stripper and sanitation tank from an anaerobic fermenter, and
wherein said volatile compounds diverted to said stripper and sanitation tank are converted into solid forms and stored until further use.

A method for producing biogas and reducing or eliminating the emission from a biogas fermentation facility of undesirable odorants in the form of gasses comprising volatile nitrogen containing compounds, and optionally also sulphur containing compounds,

In accordance with this aspect of the present disclosure there is provided a method for producing biogas and reducing or eliminating the emission from a biogas fermentation facility of undesirable odorants in the form of gasses comprising volatile nitrogen containing compounds, and optionally also sulphur containing compounds, by sequential, anaerobic fermentations and stripping at least partly said volatile nitrogen containing compounds, and optionally also said volatile sulphur containing compounds, including ammonia and, when present, hydrogen sulphide, from the fermented biomass materials comprising organic and inorganic parts, said method comprising the steps of
i) providing a first biomass material comprising organic and inorganic nitrogen parts and optionally also sulphur parts,
ii) performing an initial, anaerobic fermentation of the first biomass material in an anaerobic fermenter,
iii) producing biogas and volatile nitrogen and sulphur containing compounds, and a fermented, first biomass material,
   wherein said initial anaerobic fermentation of the first biomass material results in at least partly converting organic nitrogen parts into inorganic nitrogen parts,
   wherein said inorganic nitrogen parts comprise, or are converted into gasses comprising volatile nitrogen containing compounds during said initial anaerobic fermentation,
   wherein said sulphur parts, when present, comprise, or are converted into, gasses comprising volatile sulphur containing compounds during said initial, anaerobic fermentation,
iv) diverting the first fermented biomass material, and said gasses comprising volatile nitrogen and optionally sulphur containing compounds formed during said initial, anaerobic fermentation, to a stripper and sanitation tank for stripping of said volatile compounds,
v) stripping at least part of said gasses comprising volatile nitrogen and optionally sulphur containing compounds by heating, to a temperature of at least 70°C at a predetermined pressure sufficient stripping said volatile compounds, the contents of the stripper and sanitation tank, thereby obtaining a second biomass material comprising a reduced amount of inorganic nitrogen and optionally sulphur parts,
vi) diverting said second biomass material having a reduced amount of inorganic nitrogen and optionally sulphur parts to a further anaerobic fermenter and subsequently fermenting said second biomass material under anaerobic conditions,
vii) producing biogas and volatile nitrogen and optionally also sulphur containing compounds, and a fermented, second biomass material comprising organic and inorganic nitrogen parts under said anaerobic fermentation conditions,
   wherein said subsequent anaerobic fermentation of the second biomass material results in at least partly converting organic nitrogen parts into inorganic nitrogen parts,
   wherein said inorganic nitrogen parts comprise, or are converted into, gasses comprising volatile nitrogen containing compounds during said subsequent, anaerobic fermentation,
   wherein said sulphur parts, when present, comprise, or are converted into, gasses comprising volatile sulphur containing compounds during said subsequent, anaerobic fermentation,
viii) subjecting said fermented, second biomass material to one or more separation steps resulting in the formation of a fibrous solid fraction comprising organic and inorganic nitrogen parts and at least one liquid fraction,
ix) subjecting said fibrous solid fraction to a heating and drying treatment by heating said solid fraction to a temperature of at least 70°C at a pressure sufficient to strip said volatile compounds, and stripping volatile nitrogen containing compounds, and optionally also sulphur containing compounds, present in the solid fibrous fraction,
   thereby generating a dried fibrous solid fraction having a reduced amount of volatile nitrogen containing compounds, including ammonium and inorganic nitrogen precursor volatile compounds, and optionally also a reduced amount of sulphur containing volatile compounds, e.g. hydrogen sulphide, wherein the heating and drying treatment exploits primary and secondary combustion air sources, including exhaust air sources, present in or generated in the biogas fermentation facility as a result of performing said heating and drying process, wherein said primary and secondary combustion air sources are also diverted to said stripper and sanitation tank for conversion and/or collection as solids,
   wherein the exploitation of primary combustion air sources from the biogas fermentation facility results in generating a negative pressure in the biogas fermentation facility space, which negative pressure prevents or contributes to presenting any undesirable odorants from escaping the biogas fermentation facility,
x) forming a gaseous fraction comprising nitrogen containing volatile compounds, including ammonia, and having a temperature of at least 70oC at a pressure sufficient to form said gaseous fraction,
xi) diverting said gaseous fraction comprising nitrogen containing volatile compounds, including ammonia, and optionally also sulphur containing volatile compounds, and having a temperature of at least 70°C at said predetermined pressure, to the stripper and sanitation tank of step iv) for stripping of said nitrogen containing volatile compounds and optionally also said sulphur containing volatile compounds,
   wherein the diverted gaseous fraction having a temperature of at least 70°C contributes to heating the fermented first biomass material in the stripper and sanitation tank, and/or the further first biomass material having been diverted to the stripper and sanitation tank, and
   wherein said volatile compounds diverted to said stripper and sanitation tank are converted into solid forms and stored until further use, and
xii) reducing or eliminating the emission from a biogas fermentation facility of undesirable odorants in the form of gasses comprising volatile nitrogen containing compounds, and optionally also volatile sulphur containing compounds, by performing said sequential anaerobic fermentations in a closed system and stripping at least partly said volatile nitrogen containing compounds, and optionally also said volatile sulphur containing compounds, such as ammonia and hydrogen sulphide respectively, from the fermented biomass materials comprising organic and inorganic parts,
   wherein the conversion into solid forms of said volatile compounds diverted to said stripper and sanitation tank contributes to the reduction or elimination of the emission from the biogas fermentation facility of undesirable odorants in the form of gasses comprising said volatile compounds.

### A method for sequential fermentation of a biomass material

In yet another aspect of the present disclosure there is provided a method for sequential fermentation of a biomass material, said method comprising the steps of
i) fermenting a biomass material by anaerobic batch fermentation, wherein the anaerobic batch fermentation results in the production of nitrogen containing volatile compounds, including ammonia,
ii) removing said nitrogen containing volatile compounds from the batch fermented biomass material, thereby reducing the contents of nitrogen containing volatile precursor compounds capable of being converted into nitrogen containing volatile compounds during said anaerobic batch fermentation, and generating an anaerobic batch fermented biomass material having a reduced amount of inorganic nitrogen precursor volatile compounds,
iii) obtaining an anaerobic batch fermented biomass material having a reduced content of nitrogen containing volatile compounds and comprising lignocellulose and additional macromolecular constituents not digested during said anaerobic batch fermentation,
iv) diverting the anaerobic batch fermented biomass material from the batch fermentation facility to a fungal cultivation facility and employing the batch fermented biomass material, or a solid fibrous fraction thereof having a reduced amount of inorganic nitrogen precursor volatile compounds, as a substrate for cultivating one or more fungal species,
v) cultivating said one or more fungal species in said substrate, wherein said cultivation results in the hydrolysis and/or oxidation of at least part of said lignocellulose and/or said additional macromolecular constituents not digested during said batch fermentation and the formation of a spent fungal substrate material,
vi) generating a spent fungal substrate material comprising macromolecular hydrolysis and/or oxidation products obtained by fungal digestion of said lignocellulose and/or said additional macromolecular constituents not digested during said anaerobic batch fermentation,
vii) diverting the spent fungal substrate material from the facility for cultivating one or more fungal species to a facility for continuous, anaerobic biogas fermentation and employing the macromolecular hydrolysis and/or oxidation products in said spent fungal substrate material obtained by fungal digestion of said lignocellulose and/or said additional macromolecular constituents not digested during said anaerobic batch fermentation as a substrate for microbial organisms involved in the continuous anaerobic biogas fermentation, and
viii) performing a continuous, anaerobic biogas fermentation using said spent fungal substrate material supplemented with one or more further biomass materials as a substrate for producing said biogas during a continuous, anaerobic biogas fermentation.

### A method for obtaining a feed stock biomass material suitable for use in anaerobic biogas fermentation

In a further aspect of the present disclosure there is provided a method for obtaining a feed stock biomass material suitable for use in anaerobic fermentation and biogas production, said method comprising the steps of
i) providing a first fermented biomass material as a substrate for cultivating one or more fungal species, such as Basidiomycetes, wherein said first fermented biomass material comprises one or more macromolecular nutrient constituents selected from the group consisting of cellulose, hemicellulose, and lignin,
ii) cultivating said one or more fungal species in said substrate,
   wherein said fungal species cultivation converts the macromolecular nutrient constituents present in said substrate to lower molecular weight nutrient constituents,
   wherein the cultivation of said fungal species in said substrate generates a first spent fungal species substrate,
iii) diverting said first spent fungal species substrate to an anaerobic biogas fermenter as a contribution to a feed stock biomass material,
iv) diverting one or more further biomass materials to said anaerobic biogas fermenter, such as organic waste biomass materials comprising one or more macromolecular nutrient constituents selected from the group consisting of cellulose, hemicellulose and lignin,
v) performing an anaerobic biogas fermentation by using said first spent fungal species substrate and said one or more further biomass materials as a feed stock biomass material, and
vi) producing biogas by fermentation of said feed stock under anaerobic fermentation conditions,
wherein said anaerobic biogas fermentation generates a fermented, second biomass material.

The method in one embodiment according to the present disclosure comprises the further step of fractionating said fermented, second biomass material into solid and liquid fractions and obtaining a solid fibrous fraction comprising solid and liquid parts.

The method in one embodiment according to the present disclosure further comprises the additional steps of
i) providing said second fermented biomass material, or a solid fibrous fraction thereof, as a substrate for cultivating one or more fungal species, wherein said second fermented biomass material, or a solid fibrous fraction thereof, comprises one or more macromolecular nutrient constituents selected from the group consisting of cellulose, hemicellulose, and lignin
ii) cultivating said one or more fungal species in the substrate provided in step i), wherein said cultivation converts, by hydrolysis, oxidation, or otherwise, macromolecular nutrient constituents present in said substrate to lower molecular weight nutrient constituents
   wherein said conversion of said macromolecular nutrient constituents is obtained when said fungal species are metabolizing said macromolecular constituents,
   wherein the cultivation of said fungal species in said substrate provided in step i) generates a second spent fungal species substrate having a different composition compared to the composition of the substrate provided in step i),
iii) diverting said second spent fungal species substrate to an anaerobic biogas fermenter as a contribution to a feed stock biomass material,
iv) diverting one or more further biomass materials, preferably organic waste biomass comprising one or more macromolecular nutrient constituents selected from the group consisting of cellulose, hemicellulose and lignin, to said anaerobic biogas fermenter as a further contribution to the formation of a feed stock biomass material suitable as a substrate for producing said biogas during an anaerobic biogas fermentation,
v) performing an anaerobic biogas fermentation by using said second spent fungal species substrate and said one or more further biomass materials, preferably organic waste biomass materials, as a feed stock biomass material, and
vi) producing biogas by fermentation of said feed stock under anaerobic fermentation conditions,
wherein said anaerobic biogas fermentation additionally generates a fermented, third biomass material.

The method in one embodiment according to the present disclosure further comprises the additional further steps of
i) providing said third fermented biomass material, or a solid fibrous fraction thereof, as a substrate for cultivating one or more fungal species,
   wherein said third fermented biomass material, or a solid fibrous fraction thereof, comprises one or more macromolecular nutrient constituents selected
   from the group consisting of cellulose, hemicellulose, and lignin,
ii) cultivating said one or more fungal species in the substrate provided in step i), wherein said cultivation converts, by hydrolysis, oxidation, or otherwise macromolecular nutrient constituents present in said substrate to lower molecular weight nutrient constituents,
   wherein said conversion of said macromolecular nutrient constituents is obtained when said fungal species are metabolizing said macromolecular constituents,
   wherein the cultivation of said fungal species in said substrate provided in step i) generates a third spent fungal species substrate having a different composition compared to the composition of the substrate provided in step i),
iii) diverting said third spent fungal species substrate to an anaerobic biogas fermenter as a contribution to a feed stock biomass material,
iv) diverting one or more further biomass materials, preferably organic waste biomass comprising one or more macromolecular nutrient constituents selected from the group consisting of cellulose, hemicellulose and lignin, to said anaerobic biogas fermenter as a further contribution to the formation of a feed stock biomass material suitable as a substrate for producing said biogas during an anaerobic biogas fermentation,
v) performing an anaerobic biogas fermentation by using said third spent fungal species substrate and said one or more further biomass materials, preferably organic waste biomass materials, as a feed stock biomass material, and
vi) producing biogas by fermentation of said feed stock under anaerobic fermentation conditions,
wherein said anaerobic biogas fermentation additionally generates a fermented, fourth biomass material.

The cyclical reuse of a) spent fermentation substrate in the form of fermented biomass material, or a solid fibrous fraction thereof, for cultivation of fungal species, and b) spent fungal species substrate for performing an anaerobic biogas fermentation, respectively, can be further repeated one or more times,
wherein, preferably, the fermented biomass material, or a solid fibrous fraction thereof, provided step i) of said different cycles of the method as a substrate for cultivating one or more fungal species is obtained from different batch fermentations, and wherein said different batch fermentations further comprise stripping by evaporation volatile nitrogen containing compounds, including ammonia, from said batch fermented biomass materials, thereby providing a substrate for cultivating said one or more fungal species which has a lower amount of inorganic nitrogen compounds compared to a substrate from which no volatile nitrogen containing compounds had been removed prior to cultivation of said fungal species.

The anaerobic biogas fermentation to which the spent fungal species substrates and the one or more further biomass materials, preferably organic waste biomass materials, are diverted, is preferably the same, continuous anaerobic biogas fermentation, and preferably the spent anaerobic fermentation biomass materials from said continuous anaerobic biogas fermentation are being continuously diverted from said anaerobic biogas fermenter and combined with spent biomass materials from said batch fermentations prior to said spent biomass materials being used a substrates for fungal cell cultivation.

In one embodiment according to the present disclosure the spent biomass materials is separated, dried and/or fractionated by a method for separation and drying of a biomass material according to the present disclosure.

### A method for sequentially and differentially fermenting a biomass material comprising different bioenergy sources

In a still further aspect of the present disclosure there is provided a method for sequentially and differentially fermenting a biomass material comprising different bioenergy sources, said method comprising the steps of
i) fermenting one or more first fermentable bioenergy sources forming part of a fermentable biomass material further comprising one or more additional fermentable bioenergy sources,
ii) producing a) one or more fermentation products comprising, or selected from, biogas and gasses comprising volatile nitrogen containing compounds, and b) a first fermented biomass material, by preferentially fermenting said one or more first, fermentable bioenergy sources,
   wherein said first fermented biomass material comprises a reduced amount of said one or more first, fermentable bioenergy sources, and essentially all, or at least the majority of, said one or more additional fermentable bioenergy sources,
iii) diverting said first, fermented biomass material to a fungal cultivation facility and cultivating fungal cells in said first fermented biomass material,
iv) producing a) fungal cell biomass, and b) a spent fungal substrate biomass material, by metabolizing said one or more additional, fermentable bioenergy sources and any remaining, first fermentable bioenergy source, wherein said spent fungal substrate biomass material comprises a reduced amount of said one or more additional, fermentable bioenergy source and first, fermentable bioenergy sources generated by fungal cell metabolism of said one or more additional, fermentable bioenergy sources,
v) harvesting said fungal cell biomass,
vi) diverting said spent fungal substrate biomass material to an anaerobic biogas fermenter as a feed stock biomass material,
vii) supplementing the feed stock biomass materials in said anaerobic biogas fermenter with one or more organic waste biomass materials,
   wherein said one or more organic waste biomass materials comprises fermentable bioenergy sources selected from first bioenergy sources and/or
   one or more additional bioenergy sources,
viii) fermenting said combined feed stock biomass materials under anaerobic fermentation conditions, and
ix) producing a) one or more fermentation products comprising, or selected from, biogas and gasses comprising volatile nitrogen containing compounds, and b) a further first fermented biomass material, by preferentially fermenting said one or more first fermentable bioenergy sources present in the combined feed stock biomass materials,
wherein said further first, fermented biomass material comprises a reduced amount of said one or more first, fermentable bioenergy sources, and essentially all, or at least the majority of, said one or more additional fermentable bioenergy sources.

The method in one embodiment according to the present disclosure further comprises the additional steps of
x) producing a) additional fungal cell biomass, and b) additional spent fungal substrate biomass material, by metabolizing said one or more additional, fermentable bioenergy sources and any remaining, first fermentable bioenergy source,
   wherein said additional spent fungal substrate biomass material comprises a reduced amount of said one or more additional, fermentable bioenergy source and first, fermentable bioenergy sources generated by fungal cell metabolism of said one or more additional, fermentable bioenergy sources,
xi) harvesting said additional fungal cell biomass,
xii) diverting said additional spent fungal substrate biomass material comprising first, fermentable bioenergy sources generated by fungal cell metabolism of said one or more additional, fermentable bioenergy sources to an anaerobic biogas fermenter as a feed stock biomass material,
xiii) producing a) one or more further fermentation products comprising, or selected from, biogas and gasses comprising volatile nitrogen containing compounds, and b) additional first fermented biomass material, by preferentially fermenting said one or more first, fermentable bioenergy sources present in the combined feed stock biomass materials,
wherein said additional first, fermented biomass material comprises a reduced amount of one or more first, fermentable bioenergy sources, and essentially all, or at least the majority of, one or more additional fermentable bioenergy sources.

The fermentable biomass material provided in step i) is in one embodiment according to the present disclosure a partly degassed biomass material obtained by performing an initial, anaerobic fermentation resulting in the production of a gaseous fraction comprising ammonia and biogas. Accordingly, the fermentable biomass material is preferably obtained from different batch fermentations, and the method may comprise the further step of stripping by evaporation volatile nitrogen containing compounds, including ammonia, from said fermented biomass materials, thereby providing a substrate for cultivating said one or more fungal species having a lower amount of inorganic nitrogen compounds compared to a substrate from which no volatile nitrogen containing compounds have been removed prior to cultivation of said fungal species.

The anaerobic biogas fermentation to which the spent fungal species substrates and the one or more further biomass materials, preferably organic waste biomass materials, are diverted, is preferably the same, continuous anaerobic biogas fermentation, and preferably the spent anaerobic fermentation biomass materials from said continuous anaerobic biogas fermentation are being continuously diverted from said anaerobic biogas fermenter and combined with spent biomass materials from said batch fermentations prior to said spent biomass materials being used a substrates for fungal cell cultivation.

In one embodiment according to the present disclosure the spent biomass materials is separated, dried and/or fractionated by a method for separation and drying of a biomass material according to the present disclosure.

### A method for producing and collecting first and second volatile compounds through sequential fermentations of a fermentable biomass material

It is an aspect of the present disclosure to provide a method for producing and collecting first and second volatile compounds through sequential fermentations of a fermentable biomass material, said method comprising the steps of
i) performing a first anaerobic fermentation of a first fermentable biomass material, such as in one or more pre-fermentation facility or first fermentation facility unit(s), thereby obtaining a first fermented biomass material, and producing and collecting first volatile nitrogen-containing compounds,
ii) separating at least partly the first fermented biomass material from the first volatile nitrogen-containing compounds and obtaining a separated, first fermented biomass having a reduced content of first volatile nitrogen-containing compounds, and/or a reduced content of carbon and nitrogen-containing precursor compounds capable of being converted into first volatile carbon and nitrogen containing compounds during a fermentation,
iii) diverting the separated, first fermented biomass to a second fermentation facility for producing second volatile methane-containing compounds, and
iv) performing a second anaerobic fermentation of the separated, first fermented biomass, optionally supplemented with additional organic waste biomass material, in the second fermentation facility, thereby obtaining a second fermented biomass material, and producing and collecting at least second volatile methane-containing compounds.

It is understood that the method for producing and collecting first and second volatile compounds through sequential fermentations of a fermentable biomass material, as outlined herein above, can be combined with any of the other methods defined according to the disclosure.

In one embodiment according to the present disclosure at least some of said first volatile nitrogen-containing compounds produced from the fermentation of the first fermentable biomass material have an inhibitory effect on the formation of second volatile methane-containing compounds during the fermentation of the first fermentable biomass material in said one or more pre-fermentation facility or first fermentation facility unit(s), and
an increased amount of second volatile methane-containing compounds are produced from the anaerobic fermentation of the separated, first fermented biomass in the second anaerobic fermentation facility due to the stripping of first volatile nitrogen-containing compounds from said first fermentable biomass material in said one or more pre-fermentation facility or first fermentation facility unit(s).

In one embodiment according to the present disclosure the first volatile compounds comprises gaseous ammonia.

In one embodiment according to the present disclosure the second volatile compounds, the second volatile methane-containing compounds, collectively form a gas comprising methane, such as a gas comprising more than 50% methane, such as more than 60% methane, such as more than 70% methane, such as more than 80% methane, such as more than 90% methane, such as more than 95% methane, or such as more than 99% methane, such as a biogas.

In one embodiment according to the present disclosure,
a) the formation of first volatile nitrogen-containing compounds inhibits the formation of second volatile methane-containing compounds during the first and/or second fermentation , and/or
b) at least about 20%, such as 30%, such as 40%, such as at least about 50%, of organic N in the biomass is converted to ammonia N during the first anaerobic fermentation, and/or
c) ammonia N stripped from the organic material in the pre-fermentors/ pre-reactors is diverted to a stripper and sanitation tank and/or to an absorption column for absorption of the stripped ammonia N, and/or
d) ammonia N stripped from the organic material in the pre-fermentors/ pre-reactors is diverted to an N-steamer for treatment of complex biomasses such as straw, grass and the like, as described in figure 12; and optionally subsequently diverted to a stripper and sanitation tank and/or to an absorption column.

In one embodiment according to the present disclosure,
a) the method further comprises the step of mixing the complex biomass in a first mixing tank prior to the first fermentation in the pre-fermentation or first facility unit(s), wherein said mixing in one embodiment according to the present disclosure further comprises addition of lime, and/or
b) the sequential fermentation of the fermentable biomass material comprises at least three separate fermentation steps, and/or
c) the first fermentation is conducted under thermophilic conditions or mesophilic conditions, and/or
d) the temperature for the fermentation of the first fermentable biomass material is in the range of from 25°C to 55°C, such as from 25°C to 28°C, 28°C to 30°C, 30°C to 32°C, 32°C to 35°C, 35°C to 38°C, 38°C to 42°C, 42°C to 45°C, 45°C to 48°C, 48°C to 52°C, 52°C to 55°C, and/or
e) the fermentation of the first fermentable biomass material is performed until a substantial amount of first volatile compounds is produced, wherein a substantial amount of first volatile compounds is an amount which has an inhibitory effect on the microbial organisms performing the fermentation and effectively inhibits growth and/or metabolism of said microbial organisms.

The separation of first fermented biomass and first volatile nitrogen-containing compounds may be performed in various ways according to the present disclosure.

In one embodiment according to the present disclosure the step of separating first volatile compounds from the first, fermentable biomass material includes diverting the first fermented biomass to a stripper and sanitation tank for stripping said first volatile compounds, and heating the first fermented biomass material, thereby producing a separated, first biomass material. The stripping-off of the first volatile compound includes ammonia stripping.

In one embodiment according to the present disclosure
a) the separated, first fermented biomass is diverted to a pressure unit and subjected to a thermal hydrolysis, thereby producing an at least partly hydrolysed, fermentable biomass, and/or
b) the first fermented biomass is diverted to a fermentation facility comprising thermophilic and/ or mesophilic fermenters for biogas production, and/or
c) the method comprises the further step of diverting non-complex biomass material to the pressure unit and subjecting the combined biomass materials in the pressure unit to a thermal hydrolysis, thereby producing an at least partly hydrolysed fermentable biomass, and diverting the at least partly hydrolysed, fermentable biomass to the second fermentation facility, and/or
d) the method further comprises the step of at least partly stripping-off first volatile compounds formed during the thermal hydrolysis.

In one embodiment according to the present disclosure the thermal hydrolysis occurs under predetermined alkaline conditions, optionally achieved by addition of sufficient lime to reach a pH in the range of from about 9 to about 12.

In one embodiment according to the present disclosure the hydrolysis of the biomass material in the pressure unit is performed at a temperature in the range of from 100°C to preferably less than 250°C, under a pressure of from about 2 to preferably less than 20 bar, and with an operation time ranging of preferably less than 60 minutes, or until the biomass is suitably hydrolysed.

In one embodiment according to the present disclosure the at least partly hydrolysed biomass from the pressure unit is diverted to the pre-fermentation or first fermentation facility unit(s) for further fermentation. In one embodiment according to the present disclosure said at least partly hydrolysed and further fermented biomass is further diverted to ammonia stripping in a stripper and sanitation unit.

In one embodiment according to the present disclosure the further fermentation is followed by a further thermal hydrolysis in the pressure unit for producing a further and even more hydrolysed biomass.

In one embodiment according to the present disclosure the method further comprises fermenting the at least partly hydrolysed and optionally further fermented biomass in the second fermentation facility for producing a biogas comprising second volatile compounds.

In one embodiment according to the present disclosure fermentation in the second fermentation facility comprises i) subjecting the at least partly hydrolysed biomass or the further hydrolysed biomass to first fermentation conditions resulting in the formation of a first conditioned fermented biomass; and ii) subjecting the first conditioned fermented biomass to a fermentation under a second set of conditions for producing a fermentation biomass and second volatile compounds. In one embodiment according to the present disclosure the first conditions comprise thermophilic conditions and the second conditions comprise mesophilic conditions. In one embodiment according to the present disclosure the first conditions comprises mesophilic conditions and the second conditions comprises thermophilic conditions.

In one embodiment according to the present disclosure at least part of the first conditioned, fermented biomass is diverted to a first mixing unit and/or to the pre-fermentation facility units. In one embodiment according to the present disclosure at least part of the fermented biomass is diverted to a first mixing unit and/ or to one or more of the pre-fermentation facility units.

Also provided is a method for producing a first volatile compound through fermentation of a biomass material comprising solid and/ or liquid parts, the method comprising i) receiving the biomass material in one or more pre-fermentation facility unit(s); and ii) performing a first fermentation of the biomass material in the one or more pre-fermentation facility unit(s) for producing a first fermented biomass material and at least the first volatile compound until a substantial amount of the first volatile compound is produced.

Also provided is a method for identifying a complex biomass, the method comprising computing content of organic Nitrogen in relation to the total content of the biomass material; and categorizing the biomass material as a complex biomass if the biomass material comprises a high percentage of organic Nitrogen.

The disclosure in a further aspect is directed to a sequential fermentation facility plant for fermenting a fermentable biomass and suitably adapted for generating at least first and second volatile compounds, said fermentation facility plant comprising
i) one or more pre-fermentation facility unit(s) for performing a first fermentation of the fermentable biomass material,
ii) a separation unit operably connected to the pre-fermentation facility unit(s) for separating, at least partly, first fermented biomass material from first volatile compounds, thereby obtaining a separated, first fermented biomass comprising a reduced content of first volatile compounds and/or a reduced content of carbon and nitrogen containing precursor compounds capable of being converted during a fermentation to first volatile compounds; and
iii) a second fermentation facility operably connected to the separation unit for performing a fermentation of the separated, first fermented biomass, thereby producing a further fermented biomass material and second volatile compounds.

In one embodiment according to the present disclosure,
- said separation unit comprises an N-stripper unit for stripping first volatile compounds from the fermented biomass material fermented in the one or more pre-fermentation facility unit(s), and/or
- said N-stripper unit is connected to an absorption unit for absorbing and condensing first volatile compounds stripped from the first fermented biomass material, and/or
- said separation unit comprises a sanitation unit for sanitizing the first fermented biomass material, and/or
- said pre-fermentation facility unit(s) and the separation unit are connected so that the first fermented biomass material is diverted from the pre-fermentation facility unit(s) to the separating unit, and/or
- said fermentation facility plant further comprises a pressure unit adapted to perform a thermal hydrolysis, optionally under predetermined alkaline conditions, of the separated, first fermented biomass material, wherein the pressure unit is operably connected to both the separation unit and to the second fermentation facility, and/or
- said fermentation facility plant further comprises a stripper tank connected to the pressure unit for stripping first volatile compounds from hydrolysed biomass, wherein the stripper tank is connected to an absorption unit for absorbing and condensing stripped first volatile compound, and/or
- said separation unit is connected to the pressure unit and/ or to the second fermentation facility so that the first fermented biomass material stripped off the first volatile compounds can be diverted to the pressure unit and/or to the second fermentation facility, and/or
- said fermentation facility plant further comprise connections between the pre-fermentation facility unit(s), the separation unit and the pressure unit,
- said pressure unit is connected with the second fermentation facility for diverting at least partly hydrolysed biomass at least partly stripped off first volatile compounds to the second fermentation facility, and/or
- a plurality of pre-fermentation facility unit(s) are operably connected and wherein the second fermentation facility comprises a further plurality of operably connected fermentation units, and/or
- said fermentation facility units are connected to the pre-fermentation facility unit(s) and/ or to a first mixing tank for allowing diversion of the first fermented biomass from the pre-fermentation facility and/ or diversion of biomass from the first mixing tank, and/or
- the fermentation facility is adapted to receive biomass from a reception tank using first reception connectors, the pressure unit is adapted to receive biomass from the reception tank using second reception connectors, and the pre-fermentation facility unit is adapted to receive biomass from the reception tank / the first mixing tank using third reception connector, and/or
- the pre-fermentation facility unit(s) is adapted to produce maximum amount of the first volatile compound even at the expense/ with lower amount of the second volatile compound production during the first fermentation; and the fermentation unit is adapted to produce maximum amount of the second volatile compound from the biomass material that has been stripped off the first volatile compound during the pre-treatment stage in the first fermentation unit(s) and/ or the pressure unit.

In one embodiment according to the present disclosure the fibrous solid substrate is derived from a degassed biomass from biogas production, which degassed biomass is separated into a liquid fraction and a fibrous solid fraction. The separated fibrous solid fraction is sanitized to reduce the content of volatile nitrogen-containing compounds such as ammonia, and to eliminate viable microorganisms.

In one embodiment according to the present disclosure the fibrous solid fraction is not supplemented with non-fermented biomass. In one embodiment according to the present disclosure the fibrous solid fraction is not supplemented with water.

In one embodiment according to the present disclosure the fibrous solid fraction will not compost and/or develop heat from composting of non-fermented biomass and water. In one embodiment according to the present disclosure the volatile nitrogen-containing compounds such as ammonia of the fibrous solid fraction is not removed by evaporation from composting of non-fermented biomass and water.

### Biomasses and fermentation

An anaerobic fermentation (or first or second fermentation) can be conducted under thermophilic conditions and/or under mesophilic conditions. Accordingly, an anaerobic fermentation can be performed either by performing an anaerobic fermentation under thermophilic conditions, or under mesophilic conditions, or performing an anaerobic fermentation under thermophilic conditions followed by an anaerobic fermentation under mesophilic conditions, or by performing an anaerobic fermentation under mesophilic conditions followed by an anaerobic fermentation performed under thermophilic conditions.

Theromophilic conditions comprise temperatures ranging from 42°C to 70°C, such as 45°C to 60°C, preferably from 48°C and 52°C. Mesophilic conditions comprise temperatures from 21°C to 42°C, such as 21°C to 25°C, for example 25°C to 30°C, such as 30°C to 35°C, for example 35°C to 40°C, such as 40°C to 42°C.

The thermophilic anaerobic fermentation and/or the mesophilic anaerobic fermentation is in one embodiment according to the present disclosure performed for about 5 to 15 days, such as 5 to 7 days, for example 7 to 10 days, such as 10 12 days, for example 12 to 15 days.

The pH for an anaerobic fermentation of a fermentable biomass material is in one embodiment according to the present disclosure in the range of from about 7 to 8.5, preferably between 7.4 and 8.4 and more preferably between 7.8 and 8.

Biomaterials according to the present disclosure in one embodiment comprise one or more carbon sources and one or more nitrogen sources. Carbon sources are typically polysaccharides or polymers which comprise polysaccharides. The polysaccharides are hydrolysed and/or oxidized into smaller constituents, such as e.g. oligosaccharides and/or monosaccharides. Exemplary polysaccharides are cellulose, hemicellulose, lignin and lignocellulose.

Fermentable biomass materials according to the present disclosure in one embodiment contain a maximum of 50% solid parts, such as a maximum of 40% solid parts, for example a maximum of 30% solid parts, such as a maximum of 20% solid parts by weight.

The fermentable biomass materials according to the present disclosure in one embodiment have a content of fibres of preferably more than 5% (w/w), for example more than 10% (w/w), such as more than 20% (w/w), and preferably less than 40% (w/w).

The biomass materials according to the disclosure is in one embodiment any organic waste biomass materials and in one embodiment according to the present disclosure selected from organic waste biomass materials and manures from domestic animals, including pigs, cattle, and domestic avian species.

Biomass material and fermentable biomass material may be used interchangeably herein.

In one embodiment according to the present disclosure,
a) the biomass material comprises spent mushroom substrate,
b) the biomass material is selected from the group consisting of biomasses comprising manures and slurries thereof, biomasses comprising crop residues, biomasses comprising silage crops, biomasses comprising animal carcasses and fractions thereof, slaughterhouse waste products, dairy waste products, meat and bone meal, and animal category 2 waste products, including any combination thereof, and/or
c) the fermentable biomass material comprises one or more complex biomasses (or complex waste), and/or
d) the biomass material comprises one or more complex biomasses each comprising a biomass material having a high percentage of organic Nitrogen, such as at least 5 kg, 6 kg, 7 kg, 8 kg, 10 kg, 15 kg, 20 kg, 25 kg or 30 kg of organic Nitrogen per ton of the biomass material, and/or
e) the biomass material is complex agricultural waste, such as crop residues, specifically straw, grass and the like, and/or
f) the biomass material is a complex biomass comprising protein, oily substances and fats, and/or
g) the biomass material is a complex biomass selected from the group consisting of organic municipal waste, foodstuff waste, fermentable organic industrial waste products, fish waste products, slaughterhouse waste; deep litter or manure from animals, especially from cattle, pigs and poultry holdings; animal carcasses and/or fractions thereof, meat and bone meal, blood plasma and any produce originating from animals, straw, fibres and sawdust including any combination thereof, and/or
h) the biomass material contains a maximum of 50% solid parts, such as a maximum of 40%, 30%, 20% or 10% solid parts, and/or
i) the biomass material is in a fluid condition and comprises a maximum of 10% solid parts, and/or
j) the biomass material is a liquid slurry obtained by the addition of water and/or water containing a low concentration of organic material, preferably less than 10% solid parts.

In one embodiment according to the present disclosure the biomass material is fermentable. In one embodiment according to the present disclosure the biomass material is pre-treated in order to increase the fermentation potential, the digestion potential and/or the potential as substrate.

In one embodiment according to the present disclosure the biomass material used in biogas production comprises a pre-treated complex agricultural waste, such as straw and the like, which has been treated with warm and moist steam comprising N, in one embodiment according to the present disclosure from the dryer via the N-steamer.

### Fibrous solid fraction / Fractionating spent biomass material

In one embodiment of the disclosure the fermented biomass material (such as the first and/or second fermented biomass material) is fractionated into solid and liquid parts, thereby generating a fibrous solid fraction comprising solid and liquid parts and at least one liquid fraction. In one embodiment according to the present disclosure the fibrous solid fraction comprises one or more macromolecular constituents selected from the group consisting of cellulose, hemicellulose and lignin. The fractionation (or separation) in one embodiment according to the present disclosure is performed prior to being diverted to a fungal cultivation facility.

In one embodiment of the disclosure the fibrous solid fraction is subject to one or more additional treatments. The further steps are suitable for generating a final and ready-to-use fibrous solid substrate suitable for cultivating Basidiomycete cells, and having a reduced content of inorganic nitrogen compounds.

A sanitation treatment reduces or eliminates undesirable, viable microorganisms present in the fibrous solid fraction, and/or reduces the contents of undesirable, inorganic nitrogen-containing volatile compounds present in the fibrous solid fraction.

The sanitation treatment in one embodiment according to the present disclosure strips or removes by evaporation aqueous ammonia gas from the fibrous solid fraction. It follows that said stripping reduces the amount of nitrogen-containing volatile compounds and/or inorganic nitrogen precursor volatile compounds, such as ammonium and ammonium salts, in the fibrous solid fraction comprising organic and inorganic nitrogen parts.

In one embodiment of the present disclosure the fibrous solid fraction obtained from the degassed biomass material is subjected to a sanitation treatment, in one embodiment according to the present disclosure prior to cultivation of Basidiomycete species in said fibrous solid fraction.

In one embodiment according to the present disclosure a sanitation treatment comprises evaporating one or more volatile compounds under predetermined evaporation conditions characterized at least by heating the fibrous solid fraction to a temperature of at least 70°C under alkaline pH conditions and at a pressure sufficient to evaporate said volatile compounds.

A sanitation treatment according to the disclosure in one embodiment comprises a) heating the fibrous solid fraction comprising organic and inorganic nitrogen parts to a temperature of more than 70°C, and optionally further comprises b) subjecting the fibrous solid fraction comprising organic and inorganic nitrogen parts to a pressure of more than 1 bar.

In one embodiment according to the present disclosure said fibrous solid fraction comprising organic and inorganic nitrogen parts is subjected to heating sufficient to evaporate volatile inorganic nitrogen compounds present in the fibrous solid fraction thus converting said inorganic nitrogen compounds to gaseous nitrogen containing volatile compounds, including ammonia.

The sanitation treatment in one embodiment according to the present disclosure comprises the step of heating the fibrous solid fraction comprising organic and inorganic nitrogen parts to a temperature of from 70°C to 500°C, such as 70 to 80 °C, for example 80 to 90 °C, such as 90 to 100 °C, for example 100 to 110 °C, such as 110 to 120 °C, for example 120 to 130 °C, such as 130 to 140 °C, for example 140 to 150 °C, such as 150 to 160 °C, for example 160 to 170 °C, such as 170 to 180 °C, for example 180 to 190 °C, such as 190 to 200 °C, for example 200 to 250 °C, such as 250 to 300 °C, for example 300 to 350 °C, such as 350 to 400 °C, for example 400 to 450 °C, such as 450 to 500 °C.

In one embodiment according to the present disclosure the heating of the fibrous solid fraction comprising organic and inorganic nitrogen parts to a temperature of from 70°C to 500°C, preferably 70°-200°C, results in the formation of an aqueous ammonia gas having a temperature of more than 70°C.

Independently of the above disclosed steps, the fibrous solid fraction comprising organic and inorganic nitrogen parts is in one embodiment according to the present disclosure subjected to a pressure of from 1 to 10 bar; such as 1 to 2 bar, for example 2 to 3 bar, such as 3 to 4 bar, for example 4 to 5 bar, such as 5 to 6 bar, for example 6 to 7 bar, such as 7 to 8 bar, for example 8 to 9 bar, such as 9 to 10 bar.

In one embodiment according to the present disclosure the fibrous solid fraction comprising organic and inorganic nitrogen parts is heated and dried, such as heated and dried in a dryer, such as a drum dryer.

In one embodiment according to the present disclosure the pH value of the fluid or liquid parts of said fibrous solid fraction is preferably above pH = 7.5, for example above pH 8.0, such as above pH 8.5, at least during said sanitation treatment.

The fibrous solid fraction, after stripping of aqueous ammonia gas, preferably contains a reduced amount of inorganic nitrogen precursor volatile compounds, such as less than about 70% of the amount of inorganic nitrogen precursor volatile compounds present in the fibrous solid fraction comprising organic and inorganic nitrogen parts prior to stripping aqueous ammonia gas from the fibrous solid fraction; such as from about 20% to 25%, 25 to 30%, 30% to 40%, 40% to 50%, 50% to 55%, 55% to 60%, 60% to 65%, or 65 to 70% of the amount of said inorganic nitrogen precursor volatile compounds present in the fibrous solid fraction comprising organic and inorganic nitrogen parts prior to stripping aqueous ammonia gas from the fibrous solid fraction.

The inorganic nitrogen precursor volatile compounds in one embodiment according to the present disclosure comprise ammonium compounds capable of being converted into ammonia gas during said sanitation treatment.

The fibrous solid fraction is preferably suitable for cultivating Basidiomycete cells and capable of holding a desired content of water. The fibrous solid substrate in one embodiment of the disclosure comprises fibrous particles having an average particle size of more than 100 micron (µ), such as more than 200 µ, for example more than 300 µ, such as more than 400 µ, for example more than 500 µ, such as more than 600 µ, for example more than 700 µ, such as more than 800 µ, for example more than 900 µ, such as more than 1000 µ.

The fibrous solid substrate in one embodiment according to the present disclosure comprises fibrous particles having an average particle size of from 200 µ to 300 µ, such as 300 to 400 µ, for example 400 to 500 µ, such as 500 to 600 µ, for example 600 to 700 µ, such as 700 to 800 µ, for example 800 to 900 µ, such as 900 to 1000 µ, for example 1000 to 1200 µ, such as 1250 to 1500 µ, for example 1500 to 2000 µ.

The fibrous solid substrate in one embodiment according to the present disclosure is able to hold a minimum content of water, calculated by mass, which is at least in the range of from about 10% to about 80%, such as 10% to 20%, for example 20% to 30%, such as 30% to 40%, for example 40% to 50%, such as 50% to 60%, for example 60% to 70%, such as 70% to 80%,

In one embodiment according to the present disclosure the fibrous solid fraction is drained, separated and/or ammonia stripped.

In one embodiment according to the present disclosure, the disclosure comprises a further step of draining liquid parts from the fibrous solid fraction comprising solid and liquid parts and obtaining a fibrous solid fraction comprising organic and inorganic nitrogen parts and having a total dry matter content of more than about 25% (w/w), such as more than 30% (w/w), for example more than 35% (w/w), and a residual liquid fraction.

In one embodiment according to the present disclosure the fibrous solid fraction is supplemented with one or more solid and/or liquid supplemental nutrient substrate compositions.

In order to obtain a "fluffy" consistency of the fibrous solid substrate suitable for cultivating Basidiomycete cells, the majority, and in some cases essentially all, inorganic solids present in the biomass material are separated from the fibrous solid fraction. The presence of inorganic solids in excessive and undesirable amounts will reduce the water holding capacity of the fibrous solid substrate.

The stripping of aqueous ammonia gas resulting in a reduction of the amount of inorganic nitrogen precursor volatile compounds present in the fibrous solid fraction generate an ammonia stripped, fibrous solid fraction comprising organic and inorganic nitrogen parts. In one embodiment according to the present disclosure fibrous solid fraction contains from about 0.5 kg to about 4.0 kg inorganic nitrogen (NH₄ - N) per ton of fibrous solid fraction, such as 0.5 to 1.0 kg, for example 1.0 to 1.2 kg, such as 1.2 to 1.4 kg, for example 1.4 to 1.5 kg, such as 1.5 to 1.6 kg, for example 1.6 to 1.8 kg, such as 1.8 to 2 kg, for example 2 to 2.5 kg, such as 2.5 to 3 kg, for example 3 to 3.5 kg, such as 3.5 to 4 kg inorganic nitrogen (NH₄ - N) per ton of fibrous solid fraction.

In one embodiment according to the present disclosure the ammonia stripped, fibrous solid fraction comprising organic and inorganic nitrogen parts contains from about 3 kg to about 80 kg, such as from about 3 kg to about 80 kg, organic nitrogen per ton of fibrous solid fraction, for example 3 to 5, such as 5 to 10, for example 10 to 11, such as 11 to 12, for example 12 to 13, such as 13 to 14, for example 14 to 15, such as 15 to 16, for example 16 to 17, such as 17 to 18, for example 18 to 19, such as 19 to 20, for example 20 to 25, such as 25 to 30 kg, for example 25 to 30, such as 30 to 35 kg for example 35 to 40, such as 40 to 50 kg for example 50 to 60, such as 60 to 70 kg for example 70 to 80 kg organic nitrogen per ton of fibrous solid fraction.

In one embodiment according to the present disclosure the ammonia stripped, fibrous solid fraction comprising organic and inorganic nitrogen parts contains less than 2.0 kg NH₃ per ton fibrous solid fraction, such as less than 1.8 kg, for example less than 1.6 kg, such as less than 1.4 kg, for example less than 1.2 kg, such as less than 1.0 kg, for example less than 0.8 kg, such as less than 0.6 kg. In a preferred embodiment according to the present disclosure the fibrous solid fraction contains less than 1.2 kg NH₃ per ton fibrous solid fraction.

In one embodiment according to the present disclosure the ammonia stripped, fibrous solid fraction comprising organic and inorganic nitrogen parts contains 0.1 - 0.4 kg NH₃ per ton fibrous solid fraction, such as 0.4 - 0.6 kg NH₃, for example 0.6 - 0.8 kg NH₃, such as 0.8 - 1.0 kg NH₃, for example 1.0 - 1.2 kg NH₃.

It may be desirable to adjust the pH of the fibrous solid substrate. The pH of the fibrous solid substrate is in one embodiment according to the present disclosure adjusted by adding substrate compositions to the fibrous solid fraction to obtain a fibrous solid substrate with liquid parts having, or being adjusted to have, a pH value of from 5.0 to 7.5

Once the nutrient composition of the fibrous solid substrate suitable for cultivating Basidiomycete cells has been controlled and a final, fibrous solid substrate has been manufactured, one can contact the fibrous solid substrate with one or more species of Basidiomycete cells, or spores, and cultivate said Basidiomycete cells, or spores, in said fibrous solid substrate.

By subjecting the biomass material to one or more separation steps resulting in the provision of a fibrous solid fraction comprising organic and inorganic nitrogen parts and at least one liquid fraction, and by evaporating from said fibrous solid fraction comprising organic and inorganic nitrogen parts an aqueous gas further comprising one or more volatile compounds, and obtaining a fibrous solid fraction comprising organic and inorganic nitrogen parts from which said one or more volatile compounds have been removed by evaporation, the present disclosure provides a method for controlling the composition of nutrients of a fibrous solid substrate suitable for cultivating Basidiomycete cells.

At least one of said one or more nutrients can be stripped as volatile compounds from said fibrous solid fraction by evaporation under predetermined conditions, wherein said one or more nutrients are converted into one or more volatile compounds which are stripped and removed from the composition of the final, fibrous solid substrate. The nutrient is in one embodiment according to the present disclosure evaporable or strippable inorganic nitrogen and/or sulphur containing nutrient compounds. Such compounds can be stripped as their gaseous counterparts, such as e.g. ammonia and hydrogen sulphide. Accordingly, the evaporable or strippable inorganic nitrogen and/or sulphur containing nutrient compounds can be considered precursor volatile compounds in this respect.

Evaporation of said one or more volatile compounds in one embodiment according to the present disclosure comprises a heating and drying treatment of the fibrous solid fraction comprising the steps of heating the fibrous solid fraction to a temperature of at least 70°C under alkaline pH conditions, i.e. a pH above 7, such as above 7.5, for example a pH of above 8.0, and at a pressure sufficient to evaporate said volatile compounds.

Any of the above mentioned treatments including evaporation, stripping, separation and/or sanitation will serve to provide A) a fibrous solid fraction comprising organic and inorganic nitrogen parts and having i) an increased content (w/w) of organic nitrogen compounds, ii) a reduced content (w/w) of volatile compounds, or volatile precursor compound, and iii) a reduced content (w/w) of water, and B) an aqueous gas further comprising said one or more volatile compounds, including ammonia.

In one embodiment of the disclosure a volatile nitrogen-containing compounds is selected from the group consisting of gaseous ammonia, ammonia, inorganic nitrogen; an aqueous gas comprising ammonia; an aqueous gas comprising ammonia and volatile sulphur-containing compounds.

In one embodiment according to the present disclosure a volatile nitrogen-containing compound further comprise volatile sulphur-containing compounds, including hydrogen sulphide.

In one embodiment of the disclosure a precursor volatile compound is selected from ammonium and ammonium salts.

In one embodiment of the disclosure a volatile methane-containing compound collectively form a gas comprising methane, such as a gas comprising more than 50% methane, such as more than 60% methane, such as more than 70% methane, such as more than 80% methane, such as more than 90% methane, such as more than 95% methane, or such as more than 99% methane, such as a biogas.

### A fibrous solid substrate in pellet form

It is a further aspect of the present disclosure to provide a fibrous solid substrate in pellet form obtained by a method comprising the steps of
a. providing a biomass material comprising solid and liquid parts from a biogas fermenter following an anaerobic fermentation and biogas production,
b. subjecting the biomass material to one or more separation steps resulting in the provision of a) a fibrous solid fraction comprising organic and inorganic nitrogen parts and b) at least one liquid fraction comprising solid and liquid organic and inorganic phosphor-containing parts,
c. subjecting the fibrous solid fraction to a sanitation treatment comprising one or more sanitation steps,
   wherein said sanitation treatment a) reduces or eliminates viable microorganisms present in the fibrous solid fraction, and/or b) reduces the contents of volatile nitrogen-containing compounds and/or precursor volatile compounds present in the fibrous solid fraction, and
d. obtaining a fibrous solid fraction comprising organic and inorganic nitrogen parts having a reduced content of volatile nitrogen-containing compounds,
e. optionally subjecting said fibrous solid fraction to one or more of heating, drying, evaporation, pressure, and/or alkaline pH conditions,
f. optionally adding to said fibrous solid fraction one or more solid and/or liquid supplemental nutrient substrate compositions, and
g. compressing said fibrous solid fraction, thereby generating a fibrous solid substrate in pellet form.

In one embodiment according to the present disclosure no non-fermented biomass and/or water is added to the fibrous solid fraction. In one embodiment according to the present disclosure the fibrous solid fraction is not allowed to compost.

In one embodiment according to the present disclosure the sanitation of step c. does not comprise composting.

In one embodiment according to the present disclosure the sanitation of step c. does not comprise addition of non-fermented biomass and/or water to the fibrous solid fraction.

In one embodiment according to the present disclosure said one or more sanitation steps of step c. comprises i) heating the fibrous solid fraction to a temperature of more than 70°C, optionally under alkaline pH conditions, and optionally ii) subjecting the fibrous solid fraction comprising organic and inorganic nitrogen parts to a pressure of more than 1 bar.

In one embodiment according to the present disclosure there is provided a fibrous solid substrate in pellet form obtained by a method comprising the steps of
a. providing a biomass material comprising solid and liquid parts from a biogas fermenter following an anaerobic fermentation and biogas production,
b. subjecting the fermented biomass material of step a. to one or more separation steps resulting in the provision of
   i) a fibrous solid fraction comprising organic and inorganic nitrogen parts and having a reduced content (w/w) of water, and comprising one or more macromolecular nutrient constituents selected from the group consisting of cellulose, hemicellulose, lignin and lignocellulose, and
   ii) at least one liquid fraction comprising solid and liquid organic and inorganic phosphor-containing parts,
c. subjecting the fibrous solid fraction of step b. to a sanitation and N-stripping treatment comprising the steps of
   i) heating the fibrous solid fraction to a temperature of more than 70°C, optionally under alkaline pH conditions, and optionally ii) subjecting the fibrous solid fraction comprising organic and inorganic nitrogen parts to a pressure of more than 1 bar,
      wherein said treatment i) reduces or eliminates viable microorganisms present in the fibrous solid fraction, and/or ii) reduces the contents of volatile nitrogen-containing compounds and/or precursor volatile compounds present in the fibrous solid fraction, and
d. obtaining a fibrous solid fraction comprising organic and inorganic nitrogen parts having a reduced content of volatile nitrogen-containing compounds,
e. optionally subjecting said fibrous solid fraction to one or more of heating, drying, evaporation, pressure, and/or alkaline pH conditions,
f. optionally adding to said fibrous solid fraction one or more solid and/or liquid supplemental nutrient substrate compositions, and
g. compressing said fibrous solid fraction of step d., e. and/or f., thereby generating a fibrous solid substrate in pellet form.

Obtained by is identical to obtainable by. In this respect it is understood that the step of compressing said fibrous solid fraction into pellets may be obtained by any conventional method known to the skilled person.

Compressing may be used herein interchangeably with the terms densifying and/or compacting, whereby the fibrous solid fraction is compressed, densified and/or compacted.

The fibrous solid fraction comprises organic and inorganic nitrogen parts obtained according to the disclosure has i) an increased content (w/w) of organic nitrogen compounds, ii) a reduced content (w/w) of volatile compounds, or volatile precursor compound, and iii) a reduced content (w/w) of water.

The fibrous solid fraction of steps d), e) and f) may be further processed by any means and methods as disclosed herein, prior to compression in step g).

In one embodiment according to the present disclosure the fibrous solid fraction is compressed to pellet form. In one embodiment according to the present disclosure the fibrous solid fraction is compressed using a pellet press (also known as a pellet mill). Two main types of pellet mills are the Flat Die pellet mill and the Ring Die pellet mill, which are both encompassed.

In one embodiment according to the present disclosure the fibrous solid substrate in pellet form has a density which is about 4 to 20 times higher than before the compression, such as 4 to 6 times, such as 6 to 8 times, for example 8 to 10 times, such as 10 to 12 times, for example 12 to 14 times, such as 14 to 16 times, for example 16 to 18 times, such as 18 to 20 times higher than before the compression.

In one embodiment according to the present disclosure the density of the fibrous solid substrate in pellet form is about 200 kg/m3 to about 800 kg/m3, such as from about 300 kg/m3 to about 700 kg/m3, for example from about 400 kg/m3 to about 600 kg/m3, such as from about 450 kg/m3 to about 550 kg/m3.

In one embodiment according to the present disclosure the density of the fibrous solid substrate in pellet form is less than 800 kg/m3, such as less than 700 kg/m3, for example less than 650 kg/m3, such as less than 600 kg/m3, for example less than 550 kg/m3, such as less than 500 kg/m3, for example less than 450 kg/m3, such as less than 400 kg/m3.

In one embodiment according to the present disclosure the density of the fibrous solid substrate in pellet form is 200 kg/m3 to 250 kg/m3, such as 250 kg/m3 to 300 kg/m3, for example 300 kg/m3 to 350 kg/m3, such as 350 kg/m3 to 400 kg/m3, for example 400 kg/m3 to 450 kg/m3, such as 450 kg/m3 to 500 kg/m3, for example 500 kg/m3 to 550 kg/m3, such as 550 kg/m3 to 600 kg/m3, for example 600 kg/m3 to 650 kg/m3, such as 650 kg/m3 to 700 kg/m3, for example 700 kg/m3 to 750 kg/m3, such as 750 kg/m3 to 800 kg/m3.

In one embodiment according to the present disclosure the diameter of the fibrous solid substrate in pellet form is about 6 to 20 mm, such as 6 to 8 mm, for example 8 to 10 mm, such as 10 to 12 mm, for example 12 to 14 mm, such as 14 to 16 mm, for example 16 to 18 mm, such as 18 to 20 mm.

In one embodiment according to the present disclosure the moisture content of the fibrous solid fraction of step d) and/or e) is adjusted, such as by drying and/or heating.

In one embodiment according to the present disclosure the fibrous solid fraction of step d) and/or e) is adjusted by adding one or more binders and/or one or more lubricants prior to compression.

In one embodiment according to the present disclosure the compression occurs at an appropriate temperature, such as a temperature which is higher than room temperature.

In one embodiment according to the present disclosure the compression occurs at an appropriate pressure, such as a pressure which is suitable for obtaining the required density of the pellet.

The pressure and the temperature in the pellet press during compression will vary according to the desired density and diameter of the pellet to be produced, and the skilled person will know how to achieve these parameters.

In one embodiment according to the present disclosure the pressure and temperature is lower when the pellets produced are intended for substrate for fungal production, that when the pellets are intended for use as animal litter and/or fertilizer.

In one embodiment according to the present disclosure the temperature during compression is 50 to 99°C, such as 55 to 60°C, for example 60 to 65°C, such as 65 to 70°C, for example 70 to 75°C, such as 75 to 80°C, for example 80 to 95°C, such as 95 to 99°C.

In one embodiment according to the present disclosure the pressure during compression is 10 to 60 Bar, such as 10 to 15, for example 15 to 20, such as 20 to 25, for example 25 to 30, such as 30 to 35, for example 35 to 40, such as 40 to 45, for example 45 to 50, such as 50 to 55, for example 55 to 60 Bar.

For example, for production of pellets having a diameter of 6mm and a density of 600 kg/m3 thee pressure may reach 50 bar and the temperature 90°C. For example, for production of pellets having a diameter of 12mm and a density of 450 kg/m3 thee pressure may be approximately 20 bar and the temperature 80°C.

In one embodiment according to the present disclosure the fibrous solid substrate in pellet form has a dry content of at least 75%, such as at least 80%, for example at least 85%, such as at least 90%, for example at least 95%.

In one embodiment according to the present disclosure the fibrous solid substrate in pellet form has a dry content of 75 to 80%, such as 80 to 85%, for example 85 to 90%, such as 90 to 95%.

In one embodiment according to the present disclosure the fibrous solid substrate in pellet form has a moisture content of less than or equal to 25%, such as less than or equal to 20%, for example less than or equal to 15%, such as less than or equal to 10%, for example less than or equal to 8%.

In one embodiment according to the present disclosure the fibrous solid substrate in pellet form has a moisture content of 2 to 3%, such as 3 to 4%, for example 4 to 5%, such as 5 to 6%, for example 6 to 7%, such as 7 to 8%, for example 8 to 9%, such as 9 to 10%, for example 10 to 12%, such as 12 to 14%, for example 14 to 15%, such as 15 to 16%, for example 16 to 18%, such as 18 to 20%. In one embodiment according to the present disclosure the fibrous solid substrate in pellet form has a moisture content of below 10%, such as 5 to 8%.

In one embodiment according to the present disclosure the sanitation treatment of step c) comprises one or more steps of a) heating the fibrous solid fraction comprising organic and inorganic nitrogen parts to a temperature of more than 70°C, optionally under alkaline pH conditions, and/or b) subjecting the fibrous solid fraction comprising organic and inorganic nitrogen parts to a pressure of more than 1 bar.

In one embodiment according to the present disclosure the fibrous solid fraction of step d) and/or e) is heated to a temperature of from 70°C to 300°C, such as 70 to 80 °C, for example 80 to 90 °C, such as 90 to 100 °C, for example 100 to 110 °C, such as 110 to 120 °C, for example 120 to 130 °C, such as 130 to 140 °C, for example 140 to 150 °C, such as 150 to 160 °C, for example 160 to 170 °C, such as 170 to 180 °C, for example 180 to 190 °C, such as 190 to 200 °C, for example 200 to 250 °C, such as 250 to 300 °C.

In one embodiment according to the present disclosure the fibrous solid fraction of step d) and/or e) is subjected to a pressure of from 1 to 10 bar; such as 1 to 2 bar, for example 2 to 3 bar, such as 3 to 4 bar, for example 4 to 5 bar, such as 5 to 6 bar, for example 6 to 7 bar, such as 7 to 8 bar, for example 8 to 9 bar, such as 9 to 10 bar.

In one embodiment according to the present disclosure the fibrous solid fraction of step d) and/or e) is subjected to alkaline pH conditions, i.e. a pH above 7, such as above 7.5, for example a pH of above 8.0.

In one embodiment according to the present disclosure the fibrous solid fraction of step d) and/or e) is subjected to a heating and drying treatment by heating said solid fraction to a temperature of at least 70°C at a pressure sufficient to strip said volatile compounds, and stripping volatile nitrogen containing compounds, and optionally also sulphur containing compounds, present in the solid fibrous fraction,
thereby generating a dried fibrous solid fraction having a reduced amount of volatile nitrogen containing compounds, including ammonium and inorganic nitrogen precursor volatile compounds, and optionally also a reduced amount of sulphur containing volatile compounds, e.g. hydrogen sulphide.

In one embodiment according to the present disclosure the fibrous solid substrate in pellet form obtained in step g) is cooled, either by active cooling in a cooling facility or by passive cooling by allowing the pellet to reach ambient temperature.

Heating and drying may be performed using any conventional methods known to the skilled person, such as in a dryer, such as a drum dryer.

In one embodiment according to the present disclosure the liquid parts are further drained from the fibrous solid fraction comprising solid and liquid parts to obtain a fibrous solid fraction comprising organic and inorganic nitrogen parts and having a total dry matter content of more than 25% (w/w), such as more than 30% (w/w), for example more than 35% (w/w), and a residual liquid fraction.

When intended for use as animal litter, in one embodiment according to the present disclosure the density of the fibrous solid substrate in pellet form is up to 600 kg/m3, and/or the diameter of the pellet is 6-12 mm.

When intended for use in production of mushrooms, in one embodiment according to the present disclosure the density of the fibrous solid substrate in pellet form is in one embodiment according to the present disclosure 6 to 8 times higher than before the compression, and/or the density of the pellet is 450 to 550 kg/m3, and/or the diameter of the pellet is 12-16 mm.

In one embodiment according to the present disclosure the fibrous solid substrate in pellet form are packaged in bags.

In one embodiment according to the present disclosure the fibrous solid substrate in pellet form contains from about 0.2 kg to about 4.0 kg inorganic nitrogen (NH₄ - N) per ton, for example 0.2 to 0.5 kg, such as 0.5 to 1.0 kg, for example 1.0 to 1.2 kg, such as 1.2 to 1.4 kg, for example 1.4 to 1.5 kg, such as 1.5 to 1.6 kg, for example 1.6 to 1.8 kg, such as 1.8 to 2 kg, for example 2 to 2.5 kg, such as 2.5 to 3 kg, for example 3 to 3.5 kg, such as 3.5 to 4 kg inorganic nitrogen (NH₄ - N) per ton fibrous solid substrate in pellet form.

In one embodiment according to the present disclosure the fibrous solid substrate in pellet form contains from about 3 kg to about 80 kg organic nitrogen per ton fibrous solid substrate, for example 3 to 5, such as 5 to 10, for example 10 to 11, such as 11 to 12, for example 12 to 13, such as 13 to 14, for example 14 to 15, such as 15 to 16, for example 16 to 17, such as 17 to 18, for example 18 to 19, such as 19 to 20, for example 20 to 25, such as 25 to 30 kg, such as 30 to 35 kg for example 35 to 40, such as 40 to 50 kg for example 50 to 60, such as 60 to 70 kg for example 70 to 80 kg organic nitrogen per ton fibrous solid substrate in pellet form.

In one embodiment according to the present disclosure the fibrous solid substrate in pellet form contains less than 1.0 kg NH3 per ton fibrous solid substrate in pellet form, such as less than 0.8 kg, for example less than 0.7 kg, such as less than 0.6 kg, for example less than 0.5 kg, such as less than 0.4 kg, for example less than 0.3 kg, such as less than 0.2 kg. In a preferred embodiment according to the present disclosure the fibrous solid substrate in pellet form contains less than 0.5 kg NH3 per ton fibrous solid substrate in pellet form.

In one embodiment according to the present disclosure the fibrous solid substrate in pellet form contains 0.01 - 0.05 kg NH3 per ton fibrous solid substrate in pellet form, such as 0.05 - 0.1 kg NH3, for example 0.1 - 0.2 kg NH3, such as 0.2 - 0.3 kg NH3, for example 0.3 - 0.4 kg NH3, such as 0.4 - 0.5 kg NH3 per ton fibrous solid substrate in pellet form.

In one embodiment according to the present disclosure the fibrous solid substrate in pellet form comprises one or more solid and/or liquid supplemental nutrient substrate compositions. This is especially relevant when intended for use in productions facilities, such as mushroom production, as well as manure/fertilizer. In one embodiment according to the present disclosure no supplemental nutrient substrate compositions are added when intended for use as animal litter.

In one embodiment according to the present disclosure the fibrous solid fraction is supplemented with one or more solid and/or liquid supplemental nutrient substrate compositions.

In one embodiment according to the present disclosure the biomass material is pre-treated prior to anaerobic fermentation and biogas production to increase the fermentation potential of and biogas production from said biomasses.

In one embodiment according to the present disclosure the biomass material for anaerobic fermentation and biogas production is supplemented with a pre-treated complex agricultural waste, such as straw and the like, which has been treated with warm and moist steam comprising N, in one embodiment according to the present disclosure from the dryer via the N-steamer. In one embodiment according to the present disclosure said pre-treated biomass material is grinded prior to anaerobic fermentation and biogas production.

In one embodiment according to the present disclosure the one or more solid and/or liquid supplemental nutrient substrate compositions are individually selected from the group consisting of protein, C, N, P, and K.

It is understood that the composition of the fibrous solid substrate in pellet form may be adjusted and optimized according to the intended use, such as by adding nutrients of value for production of mushrooms (generally or for specific types of mushroom), or adding nutrients of value as a fertilizer.

In one embodiment according to the present disclosure the fibrous solid substrate in pellet form comprising one or more solid and/or liquid supplemental nutrient substrate compositions, wherein the fibrous solid substrate is obtained by controlling the nutrient composition and/or the moisture content by converting said one or more supplemental nutrient substrate compositions into one or more volatile compounds and evaporating said one or more volatile compounds from said fibrous solid fraction.

In one embodiment according to the present disclosure the fibrous solid fraction in pellet form is supplemented with a pre-treated complex agricultural waste, such as straw and the like, which has been treated with warm and moist steam comprising N, such as via an N-steamer. In one embodiment according to the present disclosure said pre-treated complex agricultural waste is grinded and/or dried prior to compression into a pellet.

The fibrous solid substrate in pellet form according to the disclosure is a ready-to-use substrate. In one embodiment according to the present disclosure the fibrous solid substrate in pellet form is used directly.

When used for animal litter the fibrous solid substrate in pellet form will be able to absorb liquids such as urine and faeces.

When used for fertilizer/soil conditioner the fibrous solid substrate in pellet form will be able to transport the nutrients and dissolve when in contact with the soil.

When used for a substrate for fungal growth, the fibrous solid substrate in pellet form will be wetted by addition of water prior to and/or simultaneously with fungal production, to obtain a wetted fibrous substrate.

### Additional method steps

The biomass fermentation preferably results in the production of ammonia. Warm and moist steam comprising N, such as from the dryer (drum or band dryer), can be collected in one or more NS-units (N-stripping and sanitation units). Alternatively, or additionally, the warm and moist N-steam can be diverted to an "N-steamer" (cf. figure 12).

The N-steamer utilises warm and moist steam comprising N from the dryer to treat or pre-treat materials such as complex waste, complex agricultural waste, and some category II waste, including crop residues, straw, grass and the like. By this treatment, effectively ammonia treatment, the materials will be rendered usable as i) valuable biomasses for biogas production, as ii) fibre material for adding to the fibrous solid substrate in pellet form, as iii) a substrate directly utilisable for cultivating fungal cells and/or spores, or iv) a substrate directly utilisable as animal feed.

The warm and moist N-steam from the dryer is in one embodiment according to the present disclosure diverted to the N-steamer (and/or NS1), together with the complex waste such as straw, grass and the like, which has first been cutted and/or grinded to a desired length. Factors such as retention time, temperature, pH and moisture can be adjusted. From the N-steamer, the treated material can be diverted to one or more of a) a grinder and subsequently a biogas reactor, b) a conditioning and/or watering device and c) a grinder and/or dryer and subsequently a pellet press. The N-steam is diverted to NS1 for stripping and sanitation (to reduce the N-load on the system), and/or NS2 if the N-steam is no longer very warm.

The methods of the present disclosure in one embodiment according to the present disclosure comprise a pre-treatment anaerobic fermentation step followed be either a) a pressure cooking step and an ammonia stripping step, which steps are performed prior to at least one subsequent anaerobic fermentation step for biogas generation at the fermentation facility, or b) an ammonia stripping step performed prior to the at least one subsequent anaerobic fermentation step.

The pre-treatment fermentation step preferably results in the production of ammonia that is collected and thus not diverted to a further biogas fermenter where the production of predominantly biogas in the further fermentation step takes place.

Preferably, the pre-treatment fermentation step according to the first aspect of the present disclosure is performed in combination with an ammonia stripping and/or an ammonia collection step. By initially removing ammonia in a pre-treatment fermentation step it is possible to increase the production of biogas in a further fermentation step in a second fermentation facility. In one embodiment according to the present disclosure, the initial pre-treatment fermentation step does not involve thermophilic fermentation.

Thus, according to one embodiment of the present disclosure there is provided a method for producing first volatile nitrogen-containing compounds collectively forming gaseous ammonia and second volatile methane-containing compounds collectively forming a biogas through sequential fermentations of a biomass material comprising solid and/ or liquid parts

In one embodiment according to the present disclosure the methods of the disclosure further comprises subjecting the biomass material, such as an at least partly degassed biomass material, to one or more separation steps resulting in the provision of a) a fibrous solid fraction comprising organic and inorganic nitrogen parts and b) at least one liquid fraction comprising solid and liquid organic and inorganic phosphor-containing parts.

In one embodiment according to the present disclosure the methods of the disclosure further comprises separating solid and liquid parts of the at least one liquid fraction comprising solid and liquid organic and inorganic phosphor-containing parts by fractionation and/or sedimentation, and obtaining a) a fibrous solid fraction, b) a first solid, phosphor-containing fraction or sediment suitable for being used as, or added to, a phosphor-containing agricultural fertilizer, and c) a first liquid permeate fraction comprising solid and/or liquid nitrogen- and/or phosphor-containing parts.

In one embodiment of the disclosure the first solid, phosphor-containing fraction or sediment and the first liquid permeate fraction of the disclosure is obtained by passing the biomass material comprising solid and liquid parts over a first sieve membrane allowing the first solid, phosphor-containing fraction, or sediment, and the first liquid permeate fraction to pass through the membrane, while the fibrous solid fraction is retained, and thereby separated from the first solid, phosphor-containing fraction, or sediment, and the first liquid permeate fraction.

In one embodiment according to the present disclosure the methods of the disclosure comprise the further step of draining liquid parts from the fibrous solid fraction comprising solid and liquid parts and obtaining a residual liquid fraction.

In one embodiment according to the present disclosure the methods of the disclosure comprise the further step of combining the residual liquid fraction and the first liquid permeate fraction into a combined liquid fraction comprising solid and liquid parts, and subjecting said combined liquid fraction comprising solid and liquid parts to further separation of solid and liquid parts contained therein.

The combined liquid fraction comprising solid and liquid parts is in one embodiment according to the present disclosure passed over a second or further sieve membrane having a smaller pore size than the first sieve membrane, and the combined liquid fraction comprising solid and liquid parts separated into:
a) a second solid, phosphor-containing fraction or sediment,
b) a second liquid permeate fraction, and
c) a solid fraction concentrate comprising solid and liquid parts.

In one embodiment according to the present disclosure the separation comprises diverting the combined liquid fraction comprising solid and liquid parts over or through the second or further sieve membrane, retaining the solid fraction concentrate comprising solid and liquid parts and separating said solid fraction concentrate from the second solid, phosphor- comprising fraction or sediment, and the second liquid permeate fraction.

In a still further step, the said first and second solid, phosphor-containing fractions or sediments are dried.

In one embodiment according to the present disclosure the gaseous ammonia generated during the initial, anaerobic fermentation is stripped from the partly degassed biomass material by heating the partly degassed biomass material in a designated stripper and sanitation tank to a temperature of at least about 70°C at a pressure sufficient to strip said volatile compounds, and collecting and/or storing the stripped, gaseous ammonia, preferably storing the stripped ammonia by converting the ammonia gas to a solid ammonium salt compound by a reaction with a suitable acid.

In one embodiment according to the present disclosure the temperature in the stripper and sanitation tank is 75°C to 95°C, such as 78°C to 90°C, such as to 80°C to 88°C, such as 82°C to 85°C.

In one embodiment according to the present disclosure the pH in the stripper and sanitation tank is maintained from 9 to 12, such as 9.5 to 11.8, such as 10 to 11.3, such as 10.5 to 11. In one embodiment according to the present disclosure the pH is controlled by addition of lime to the stripper and sanitation tank.

In one embodiment according to the present disclosure said sanitation treatment that reduces the contents of volatile nitrogen-containing compounds and/or precursor volatile compounds present in the fibrous solid fraction, produces and/or evaporates volatile nitrogen-containing compounds and/or precursor volatile compounds.

In one embodiment according to the present disclosure said volatile nitrogen-containing compounds and/or precursor volatile compounds are diverted to and/or collected in a stripper and sanitation tank.

In one embodiment according to the present disclosure said volatile nitrogen-containing compounds and/or precursor volatile compounds are diverted to an N-steamer, and optionally subsequently to a stripper and sanitation tank.

In one embodiment according to the present disclosure said volatile nitrogen-containing compounds comprise gaseous ammonia which ammonia gas is converted to a solid ammonium salt compound by reaction with an acid, such as an inorganic or organic acid.

The stripped ammonia gas can be converted to a solid ammonium salt compound by a reaction with an acid.

In one embodiment according to the present disclosure the solid ammonium salt compound is stored. In one embodiment according to the present disclosure the solid ammonium salt compound is used to enrich a composition, such as an agricultural fertilizer.

According to the disclosure any stripped ammonia gas is in one embodiment according to the present disclosure collected and/or converted into a solid ammonium compound fraction comprising one or more inorganic, ammonium salt compounds, such as for example ammonium sulphate, following a reaction with an acid, such as for example sulphuric acid, or any other, suitable inorganic or organic acid.

In one embodiment according to the present disclosure the methods according to the disclosure further comprise one or more of the steps of
i) heating the fibrous solid fraction to a temperature of more than 70°C, wherein said heating results in the formation of an aqueous ammonia gas having a temperature of more than 70°C, and/or
ii) stripping said aqueous ammonia gas having a temperature of more than 70°C from the drained, fibrous solid fraction, and/or
iii) diverting the aqueous ammonia gas having a temperature of more than 70°C to the stripper and sanitation tank, and/or
iv) using the aqueous ammonia gas having a temperature of more than 70°C for heating the partly degassed biomass material present in the stripper and sanitation tank, and/or
v) optionally heating the partly degassed biomass material present in the stripper and sanitation tank by one or more additional heating sources.

In one embodiment according to the present disclosure the sanitation treatment exploits primary and secondary combustion air sources, including exhaust air sources, present in or generated in the biogas fermentation facility as a result of performing said sanitation, wherein said primary and secondary combustion air sources are diverted to a stripper and sanitation tank for conversion and/or collection as solids.

In one embodiment according to the present disclosure the exploitation of primary combustion air sources from the biogas fermentation facility results in generating a negative pressure in the biogas fermentation facility space, which negative pressure prevents or contributes to preventing any undesirable odorants from escaping the biogas fermentation facility, wherein said odorants comprise one or more volatile nitrogen-containing compounds and/or volatile sulphur-containing compounds.

In one embodiment according to the present disclosure the (first) biomass material suitable for anaerobic fermentation and biogas production is a partly degassed biomass material obtained by performing an initial, anaerobic fermentation resulting in the production of a gaseous fraction comprising ammonia and biogas.

In one embodiment of the present disclosure, the first biomass material suitable for anaerobic fermentation and biogas production is a partly degassed biomass material obtained by performing an initial, anaerobic batch fermentation resulting in the production of a gaseous fraction comprising ammonia and biogas.

In one embodiment according to the present disclosure the continuous, anaerobic biogas fermentation generates a fermented, degassed biomass material suitable for use as a substrate for cultivating one or more fungal species, and the fermented, degassed biomass material obtained from the continuous, anaerobic biogas fermentation and the anaerobic batch fermented biomass material obtained from the batch fermentation are combined and used as a substrate for cultivation of one or more fungal species.

The further biomass materials is in one embodiment according to the present disclosure any organic waste biomass materials and in one embodiment according to the present disclosure selected from organic waste biomass materials and manures from domestic animals, including pigs, cattle, and domestic avian species.

### Basidiomycete

Basidiomycete, Basidiomycetes and Basidiomycete cells and/or spores are used interchangeably herein.

Many fungal organisms including the Basidiomycetes produce and secrete extracellular enzymes capable of degrading or digesting the macromolecular nutrient constituents comprised in the fibrous solid substrate, including cellulose, hemicellulose, lignin and lignocellulose.

Hence, by performing, sequentially in any order, anaerobic biogas fermentations and Basidiomycete cultivation methods using a fibrous solid fraction from the spent biomass as a substrate for the cultivation of Basidiomycetes the macromolecular nutrient constituents present in a biomass material can be more efficiently utilized.

The digestion by extracellular Basidiomycete enzymes of said macromolecular nutrient constituents results in a hydrolysis and/or an oxidation of at least part of said macromolecular constituents In one embodiment according to the present disclosure said hydrolysis and/or oxidation of at least part of said macromolecular constituents generates a spent fungal substrate capable of being fermented by microbial organisms involved in one or more stages of a biogas fermentation. In one embodiment according to the present disclosure said microbial organisms involved in said one or more stages of a biogas fermentation metabolises the hydrolysis and/or oxidation products resulting from the hydrolysis and/or oxidation of said macromolecular constituents.

Suitable fungal organisms according to the disclosure comprise organisms constituting the phylum Basidiomycota of the kingdom Fungi, or, in older classification schemes, the class Basidiomycetes of the kingdom Plantae, i.e. fungal organisms characterized by bearing the spores on a basidium, including edible mushrooms.

In one embodiment according to the present disclosure the Basidiomycetes are selected from the group of Basidiomycetes belonging to any of the subclasses of Agaricomycetidae, Exobasidiomycetidae, Tremellomycetidae and Ustilaginomycetidae, wherein said Basidiomycete is able to degrade or digest macromolecular nutrient constituents including cellulose, hemicellulose, lignin, and lignocellulose.

Preferred Basidiomycete cells are those which are edible, in one embodiment according to the present disclosure including a fungus or fungal cell selected from the genera of Agaricus, Lentinula (Lentinus), Flammulina, Pleurotus; and Lyophyllum. In one embodiment according to the present disclosure the Basidiomycete cell is selected from the species of Lentinula (Lentinus) edodes (shiitake); edible Agaricus species (e.g. Agaricus bisporus, Agaricus campestris, Agaricus subrufescens), Flammulina velutipes (Enokitake), Pleurotus eryngii (Eryngii), Pleurotus ostreatus; and Shimeji (e.g. Lyophyllum shimejl, Buna-shimeji, Bunapi-shimeji, Hatake-shimeji, shirotamogidake, velvet pioppino).

The cultivation of the Basidiomycete cell in one embodiment according to the present disclosure takes place at a temperature of from 15°C to 35°C, for example 15°C to 17°C, such as 17°C to 20°C, for example 20°C to 22°C, such as 22°C to 25°C, for example 25°C to 30°C, such as 30°C to 35°C. The cultivation of the Basidiomycete cell in one embodiment according to the present disclosure takes place in a fibrous solid substrate having a moisture content of from 50% by mass to 70 % by mass, such as a moisture content of about 60% by mass.

Conventional fungal cultivation protocols can be followed as long as the substrate for the cultivation is a fibrous solid substrate isolated from a spent, at least partly degassed biomass material following anaerobic biogas fermentation.

### Methods of use of the fibrous solid substrate in pellet form

It is an aspect of the present disclosure to provide a method for cultivating fungal cells and/or spores comprising the steps of
a. providing fungal cells and/or spores,
b. providing the fibrous solid substrate in pellet form according to the disclosure,
   wherein the fibrous solid substrate in pellet form optionally comprises one or more supplemental nutrient substrate compositions suitable for fungal growth,
c. adding water to the fibrous solid substrate in pellet form to obtain a wetted fibrous substrate,
d. contacting the fungal cells and/or spores with the wetted fibrous substrate,
e. cultivating the fungal cells and/or spores in said substrate, and
f. optionally obtaining a spent fungal substrate.

In one embodiment according to the present disclosure the moisture content of the wetted fibrous substrate is about 55% w/w to about 80% w/w, such as about 60% w/w to about 75% w/w, for example about 65% w/w to about 70% w/w.

Also disclosed is the use of the fibrous solid substrate in pellet form according to the disclosure for cultivating fungal cells and/or spores, including but not limited to Basidiomycetes.

In one embodiment according to the present disclosure the fibrous solid substrate in pellet form comprises one or more supplemental nutrient substrate compositions suitable for fungal growth, such as nutrients known to be of value for cultivating fungal cells and/or spores; either generally, or those nutrients that are of value for specific types of fungal cells and/or spores.

Also disclosed is the use of the fibrous solid substrate in pellet form as disclosed herein as litter for animals, such as household animals, such as domesticated animals, such as horses, cows, poultry, pigs and the like.

Also disclosed is the use of the fibrous solid substrate in pellet form as disclosed herein as feed or fodder for animals, such as household animals, such as domesticated animals, such as horses, cows, poultry, pigs and the like.

Also disclosed is the use of the fibrous solid substrate in pellet form as disclosed herein as manure or fertilizer, such as household fertilizer, or a soil improver.

In one embodiment according to the present disclosure the fibrous solid substrate in pellet form comprises one or more supplemental nutrient substrate compositions suitable for plant/crop growth, such as nutrients known to be of value in fertilizers; either generally, or those nutrients that are of value for specific types of plants/crops.

### Macromolecular nutrient constituents

The fibrous solid substrate suitable for cultivating Basidiomycete cells in a preferred embodiment according to the present disclosure comprises one or more macromolecular nutrient constituents selected from the group consisting of cellulose, hemicellulose lignin and lignocellulose. The fibrous solid substrate in one embodiment according to the present disclosure comprises more than one macromolecular constituent, such as two or three macromolecular constituents selected from the group consisting of cellulose, hemicellulose and lignin.

The macromolecular nutrient constituents are difficult for the microbial organisms such as anaerobic bacteria involved in one or more stages of an anaerobic fermentation and biogas production to digest.

Lignocellulose in the form of a feed stock biomass material comprises cellulose, hemicellulose and lignin as macromolecular constituents and lignocellulose or lignocellulose-containing material is an example of a biomass material used for biogas production.

The early stages of a biogas fermentation includes an initial stage of hydrolysis of macromolecular nutrient constituents into their basic constituents, or into nutrient constituents which can more readily be metabolized and fermented by the microbial organisms involved in one or more stages of an anaerobic fermentation and biogas production. Metabolism of nutrient constituents is essential for the production of biogas as no fermentable and energy generating microbial activities can be carried out in the absence of such metabolism.

It is a particular challenge during an anaerobic biogas fermentation that no, or an insufficient hydrolysis takes place of macromolecular nutrient constituents into their basic constituents, or into nutrient constituents which can more readily be metabolized and fermented by the microbial organisms involved in one or more stages of an anaerobic fermentation and biogas production. Accordingly, the fibrous solid substrate according to the present disclosure comprises one or more macromolecular nutrient constituents which are difficult, if not impossible, for many microbial organisms involved in one or more stages of an anaerobic fermentation and biogas production to digest.

The general lack of hydrolysis of said macromolecular constituents, including cellulose, hemicellulose and lignin, by methanogenic and other anaerobic bacteria involved in the production of biogas under anaerobic fermentation conditions, will result in said macromolecular constituents being present during an anaerobic biogas fermentation during one or more stages of the anaerobic biogas fermentation, including the stages selected from acidogenesis, acetogenesis and methanogenesis.

In one embodiment according to the present disclosure the fibrous solid substrate suitable for cultivating fungal such as Basidiomycete cells comprising one or more macromolecular nutrient constituents selected from the group consisting of cellulose, hemicellulose lignin and lignocellulose are at least partly digested by the cultivation of fungal cells, leaving a spent fungal substrate which, optionally supplemented with further biomass, can be used as a feed stock for a new round of anaerobic fermentation and biogas production.

### Methods for pre-treatment of biomass materials prior to anaerobic fermentation

The methods of the present disclosure in one embodiment according to the present disclosure comprise a pre-treatment step prior to anaerobic fermentation and biogas production from biomasses. Such pre-treatment may increase the fermentation potential of and biogas production from said biomasses.

In one embodiment according to the present disclosure a biomass material is pre-treated prior to fermentation, such as by ammoniation, in a particular embodiment according to the present disclosure by utilising warm and moist steam comprising N, such as from a dryer (drum or band dryer). This is feasible especially for complex waste, complex agricultural waste, and some category II waste, including crop residues, straw, grass and the like.

The above-cited macromolecular constituents can be degraded enzymatically as well as by mechanical and/or chemical treatments of biomass materials containing such macromolecular constituents prior to anaerobic fermentation thereof.

In one embodiment according to the present disclosure a biomass material according to the present disclosure is subjected to one or more pre-treatment processing steps in any suitable way or combination prior to performing an anaerobic fermentation on the pre-treated biomass material.

The pre-treatment is in one embodiment according to the present disclosure carried out before enzymatic hydrolysis and/or oxidation of the biomaterial components forming part of the biomaterial, and in another embodiment according to the present disclosure at the same time as an enzymatic hydrolysis and/or oxidation takes place.

The enzymatic hydrolysis and/or oxidation is in one embodiment according to the present disclosure catalyzed by endogeneous microbial organisms present in the biomaterial to be subjected to anaerobic fermentation. In another embodiment according to the present disclosure the enzymes are exogeneously added, for example as bulk enzymes produced by a producer of industrial enzymes. In one embodiment according to the present disclosure the microbial organism is Basidiomycetes.

A pre-treatment according to the disclosure in one embodiment reduce the size of the solids and macromolecular constituents making up the biomass material. The pre-treatment thus increase or supplement the rate of hydrolysis of the biomass materials prior to - or during - anaerobic biogas fermentation. A pre-treatment according to the disclosure in one embodiment promotes the separation and/or release of cellulose, hemicellulose and/or lignin.

Pre-treatment processes, such as wet-oxidation, steam explosion and alkaline pre-treatment steps, will preferably target lignin, while dilute acid and auto-hydrolysis will preferably target hemicellulose containing materials.

The pre-treatment step is in one embodiment according to the present disclosure a conventional pre-treatment step using techniques well known in the art. In one embodiment according to the present disclosure pre-treatment takes place in a slurry of lignocellulose-containing material and water. The lignocellulose-containing material during pre-treatment is in one embodiment according to the present disclosure present in an amount between 10-80 wt.-%, such as 10-20 wt.-%, for example 20-30 wt.-%, such as 30-40 wt.-%, for example 40-50 wt.-%, such as 50-60 wt.-%, for example 60-70 wt.-%, such as 70-80 wt.-%, such as around 50 wt-%.

The biomass material or lignocellulose-containing material according to the disclosure is in one embodiment according to the present disclosure chemically, mechanically and/or biologically pre-treated before, before and during, or during, hydrolysis or fermentation. Mechanical pre-treatment may be carried out alone or combined with chemical or biological pre-treatment processes

The pre-treated biomass material preferably has a neutral to basic pH value before anaerobic fermentation / when it is added to the biogas digester. An acidic biomass may slow down or complicate the biogas conversion process due to inhibition of methanogenic microorganisms. The pH-value of the biomass entering the anaerobic digester is preferably between 7 and 10, such as from 7.2 to 10; for example from 7.4 to 10, such as from 7.6 to 10, for example from 7.8 to 10, such as from about 8 to 10, for example around pH 8.5. The pH may be adjusted using NaOH, Na₂CO₃, NaHCO₃, Ca(OH)₂, CaO lime hydrate, ammonia and/or KOH or the like.

In one embodiment according to the present disclosure the pre-treatment includes subjecting a biomass material to thermo-chemical treatment in a lime pressure cooker. The pressure cooking pre-treatment breaks down complex macromolecular structures of the biomass material and also contributes to and results in stripping of ammonia from the biomass.

In another embodiment according to the present disclosure, instead of or in addition to a thermo-chemical pre-treatment step, the biomass material can be subjected to a pre-fermentation in the pre-fermenters with N-stripping.

### Chemical pre-treatment

In one embodiment according to the present disclosure a biomass material according to the present disclosure is subjected to one or more chemical pre-treatment steps.

A chemical pre-treatment include treatment with; for example, dilute acid, lime, lime hydrate, alkaline, NaOH, Na₂CO₃, NaHCO₃, Ca(OH)₂, organic solvent, cellulose solvent, ammonia, KOH, sulfur dioxide, carbon dioxide, enzymatic hydrolysis. Pre-treatment processes using ammonia are described in, e.g., WO 2006/110891, WO 2006/11899, WO 2006/11900, WO 2006/110901.

Further, wet oxidation and pH-controlled hydro-thermolysis are also considered chemical pre-treatment. Wet oxidation techniques involve use of oxidizing agents, such as: sulphite based oxidizing agents or the like. Examples of solvent pre-treatments include treatment with DMSO (Dimethyl Sulfoxide) or the like. Other examples of suitable pre-treatment processes are described by Schell et al. (2003) Appl. Biochem and Biotechn. Vol. 105-108, p. 69-85, and Mosier et al. Bioresource Technology 96 (2005) 673-686, and US publication no. 2002/0164730.

### Mechanical pre-treatment

In one embodiment according to the present disclosure a biomass material according to the present disclosure is subjected to one or more mechanical pre-treatment steps, or homogenization.

The term "mechanical pre-treatment" refers to any mechanical (or physical) pre-treatment which promotes the separation and/or release of cellulose, hemicellulose and/or lignin from lignocellulose-containing material. For example, mechanical pre-treatment includes various types of milling, irradiation, steaming/steam explosion, and hydrothermolysis.

Mechanical pre-treatment includes comminution (mechanical reduction of the size). Comminution includes dry milling, wet milling, vibratory ball milling and grinding. Mechanical pre- treatment may involve high pressure and/or high temperature (steam explosion). In an embodiment of the disclosure high pressure means pressure in the range from 300 to 600 psi, preferably 400 to 500 psi, such as around 450 psi. In an embodiment of the disclosure high temperature means temperatures in the range from about 100 to 300°C., preferably from about 140 to 235°C. In a preferred embodiment according to the present disclosure mechanical pre- treatment is carried out as a batch-process, in a steam gun hydrolyzer system which uses high pressure and high temperature as defined above. A Sunds Hydrolyzer (available from Sunds Defibrator AB (Sweden) may be used.

In one embodiment according to the present disclosure the lignocellulose-containing material is subjected to a irradiation pre-treatment. The term "irradiation pre-treatment" refers to any pre- treatment by microwave e.g. as described by Zhu et al. "Production of ethanol from microwave-assisted alkali pre-treated wheat straw" in Process Biochemistry 41 (2006) 869-873 or ultrasonic pre-treatment, e.g., as described by e.g. Li et al. "A kinetic study on enzymatic hydrolysis of a variety of pulps for its enhancement with continuous ultrasonic irradiation", in Biochemical Engineering Journal 19 (2004) 155-164.

In one embodiment according to the present disclosure the lignocellulose-containing material is subjected to both chemical and mechanical pre-treatment. For instance, the pre-treatment step may involve dilute or mild acid treatment and high temperature and/or pressure treatment. The chemical and mechanical pre-treatments may be carried out sequentially or simultaneously, as desired.

In an embodiment according to the present disclosure the pre-treatment is carried out as a dilute and/or mild acid steam explosion step. In another preferred embodiment according to the present disclosure pre-treatment is carried out as an ammonia fiber explosion (or AFEX pre-treatment step).

In yet another embodiment according to the present disclosure, a base is added to the lignocellulose-containing material or the slurry prior to or while it is being homogenized; preferably the base is NaOH, Na₂CO₃, NaHCO₃, Ca(OH)₂, lime hydrate, ammonia and/or KOH or the like.

### Biological pre-treatment

In one embodiment according to the present disclosure a biomass material according to the present disclosure is subjected to one or more biological pre-treatment steps. The term "biological pre-treatment" refers to any biological pre-treatment which promotes the separation and/or release of cellulose, hemicellulose, and/or lignin from the lignocellulose-containing material.

In one embodiment according to the present disclosure the biological pre-treatment technique involve applying lignin-solubilizing microorganisms.

### Enzymatic pre-treatment

In one embodiment according to the present disclosure a biomass material according to the present disclosure is subjected to one or more enzymatic pre-treatment steps. Before the pre-treated lignocellulose-containing material is fermented it can preferably be hydrolysed enzymatically to break down especially hemicellulose and/or cellulose into fermentable sugars.

According to the disclosure enzymatic hydrolysis is performed in several steps. The lignocellulose-containing material to be hydrolysed in one embodiment according to the present disclosure constitutes above 2.5% wt-% DS (dry solids), preferably above 5% wt-% DS, preferably above 10% wt-% DS, preferably above 15 wt-% DS, preferably above 20 wt.-% DS, more preferably above 25 wt-% DS of the slurry of step a).

In one embodiment according to the present disclosure the lignocellulose-containing material is subjected to the action of one or more enzyme activities of enzymes selected from the group consisting of an amylolytic enzyme (amylase), a lipolytic enzyme (lipase), a proteolytic enzyme (protease), a hemicellulase, a pectinolytic enzyme (pectinase), a cellulolytic enzyme (cellulase), an oxidoreductase and a plant cell-wall degrading enzyme.

In one embodiment according to the present disclosure, the one or more enzyme for enzymatic pre-treatment is selected from the group consisting of aminopeptidase, alpha-amylase, amyloglucosidase, arabinofuranosidase, arabinoxylanase, beta-glucanase, carbohydrase, carboxypeptidase, catalase, cellobiohydrolase, cellulase, chitinase, cutinase, cyclodextrin glycosyltransferase, ferulic acid esterase, deoxyribonuclease, endo-cellulase, endo-glucanase, endo- xylanase, esterase, galactosidase, beta-galactosidase, glucoamylase, glucose oxidase, glucosidase, haloperoxidase, hemicellulase, invertase, isomerase, laccase, ligase, lipase, lyase, mannanase, mannase, mannosidase, oxidase, pectate lyase, pectin lyase, pectin trans- eliminase, pectin ethylesterase, pectin methylesterase, pectinolytic enzyme, peroxidase, protease, phytase, phenoloxidase, polygalacturonase, polyphenoloxidase, proteolytic enzyme, rhamnogalacturonan lyase, rhamnoglucanase rhamnogalacturonase, ribonuclease, SPS-ase, transferase, transglutaminase, xylanase and xyloglucanase.

The enzymatic activities listed is in one embodiment according to the present disclosure provided by Basidiomycete cells producing extracellular enzymes having said activates, or in another embodiment according to the present disclosure by other microbial organisms. In yet another embodiment according to the present disclosure endogenic enzymes are provided e.g. in a bulk enzyme preparation e.g. to a biomass material prior to anaerobic biogas fermentation.

Hydrolytic enzymes, proteases and oxidases produced by Basidiomycetes and other fungal species are preferred as these are present in the spent mushroom substrate which constitutes an input biomaterial for pre-treated and anaerobic fermentation.

### N-steamer

Ammonia treatment of straw, grass and the like (complex biomasses high in C) is well known and used as a treatment method for increasing digestibility and value of said e.g. straw as feed stock for cattle. By ammonia treatment of e.g. straw it is possible to increase the content of raw protein and increase the digestible energy content. For example, it is possible to increase the content of raw protein (dry matter) from 3.3% to 8% in wheat straw, from 4% to 6.9% in barley straw and from 4.2% to 11 % in rice straw, and to increase the digestible energy content from 7.5 to 9.5 MJ/kg of dry matter.

The efficiency of ammonia treatment depends on various factors such as temperature, pH, moist content and retention time.

With the "N-steamer" the inventor is introducing an innovative method for treatment of e.g. straw with ammonia N. The choice and the design of the technology and process utilizes the N and energy sources in a new manner, and enables a broad final use of the N-treated biomasses such as straw (see figure 12). In connection with the production of N-treated straw as "ready to use" substrate for fungal cells and/or spores (mushroom), the method is very efficient compared with traditional ways of producing substrate for mushroom.

The present disclosure provides a solution that enables complex biomasses high in C, such as straw, grass and the like, to become readily useable via ammonia treatment using warm and moist steam comprising N generated during anaerobic fermentation and biogas production. The ammonia treatment of said complex biomasses occurs in an N-steamer which comprises
a) warm and moist steam comprising N generated during biogas production and drying of degasified biomasses (N-steam), and
b) complex biomasses high in C, such as straw, grass and the like, which has been cutted and/or grinded into smaller pieces.

In one embodiment according to the present disclosure there is provided a method for ammonia treatment of complex biomasses high in C, such as straw, grass and the like, said method comprising the steps of
a) providing complex biomasses high in C, such as straw, grass and the like,
b) optionally cutting and/or grinding said complex biomasses into smaller pieces,
c) providing warm and moist steam comprising N generated during biogas production and drying of degasified biomasses (N-steam),
d) allowing for ammonia treatment of said complex biomasses with said N-steam.

In one embodiment according to the present disclosure the ammonia treatment using N-steam has a temperature of about 60 to 95°C, such as 60 to 65°C, such as 65-70°C, such as 70 to 75°C, such as 75-80°C, such as 80-85°C, such as 85-90°C.

In one embodiment according to the present disclosure the ammonia treatment using N-steam has a neutral or weakly basic pH. In one embodiment according to the present disclosure the ammonia treatment using N-steam has a pH of about 7 to 9, such as 7 - 7.5, such as 7.5 - 8, such as 8 - 8.5, such as 8.5 - 9.

In one embodiment according to the present disclosure the ammonia treatment using N-steam has a moisture content of about 30 to 50%, such as 30 to 35%, such as 35 to 40%, such as 40 to 45%, such as 45 to 50%.

In one embodiment according to the present disclosure the ammonia treatment using N-steam has a retention time of about 20 minutes to 12 hours, such as 20 to 30 minutes, such as 30 to 45 minutes, such as 45 to 60 minutes, such as 1 hour to 2 hours, such as 2 to 3 hours, such as 3 to 4 hours, such as 4 to 5 hours, such as 5 to 6 hours, such as 6 to 7 hours, such as 7 to 8 hours, such as 8 to 9 hours, such as 9 to 10 hours, such as 10 to 11 hours, such as 11 to 12 hours.

In one embodiment according to the present disclosure the N-steam is taken from the back end of the dryer and sucked through the cutted straw in the N-steamer, and then led to NS1 or NS2 for N-stripping / absorption.

IN one embodiment according to the present disclosure the complex biomasses such as wheat comprises wheat straw, barley straw and rice straw.

In one embodiment according to the present disclosure the N-steam treated complex biomasses are diverted to one or more of
i) a grinder and subsequently a biogas reactor (for biogas feed stock),
ii) a grinder and subsequently a pellet press, optionally through a dryer (for supplement to the fibrous solid substrate in pellet form, usable e.g. for mushroom production or animal feed/litter), and/or
iii) a conditioning and/or watering device, for generating substrates directly utilisable for cultivating fungal cells and/or spores or as animal feed.

In one embodiment according to the present disclosure the N-steam treated complex biomasses is used as
a) biomasses for biogas production (biogas feed stock),
b) fibre material for adding to the fibrous solid substrate in pellet form, as defined herein,
c) a substrate directly utilisable for cultivating fungal cells and/or spores, and/or
d) a substrate directly utilisable as animal feed/fodder.

Also provided is an N-steamer for ammonia N treatment of complex biomasses, such as straw, grass and the like rich in C, wherein said N-steamer receives warm and moist steam comprising N generated during biogas production and drying of degasified biomasses (N-steam).

In one embodiment according to the present disclosure the N-steam is taken from the back end of the dryer and sucked through the cutted straw comprised in the N-steamer, and subsequently led to one or more stripper and sanitation tanks for N-stripping / absorption.

In one embodiment according to the present disclosure the N-steam treated complex biomasses of the N-steamer are diverted to one or more of
i) a grinder and subsequently a biogas reactor,
ii) a grinder and/or a dryer and subsequently a pellet press, and/or
iii) a conditioning and/or watering device.

### Production of fertilizers

In one embodiment according to the present disclosure the present disclosure relates to further fractionating and processing liquid fractions obtained from spent biomass materials into high value N (Nitrogen) and P (Phosphor)-containing fertilizer products. Spent biomass material from biogas production plants contains high amounts of liquid. The liquid from spent biomass material can be used directly as fertilizer but the nutrients content is not optimal in part due to the large volume of the liquid in the spent biomass. The present disclosure provides methods for optimizing the nutrients content in the liquid fraction, thereby generating fertilizer products having improved nutrients content. The resulting fertilizer products also have a decreased volume as compared to the liquid part of the spent biomass material thus making them easier to transport and store.

Thus, in one embodiment according to the present disclosure the present disclosure relates to a method for fractionating a partly degasified biomass material and obtaining
a) a fibrous solid fraction comprising solid and liquid parts, said fibrous solid fraction further comprising organic and inorganic nitrogen parts,
b) a fibrous liquid fraction or concentrate comprising solid and liquid parts and further comprising organic and inorganic nitrogen parts,
c) an essentially non-fibrous liquid fraction (permeate) comprising mainly inorganic nitrogen parts, and
d) a phosphor (P) comprising fraction or sediment.

An essentially non-fibrous liquid fraction, i.e. the permeate, is a liquid fraction having a low solids content, such as containing less than about 10% dry matter, for example less than about 5% dry matter, such as less than 2% dry matter. In one embodiment according to the present disclosure the permeate contains less than about 2% dry matter.

In one embodiment according to the present disclosure, there is provided a method for fractionating a partly degasified biomass material, said partly degasified biomass material being a biomass material comprising solid and liquid parts, comprising the steps of:
i) providing a partly degasified biomass material comprising solid and liquid nitrogen (N) and phosphor (P) containing parts from a biogas fermenter following an anaerobic fermentation and biogas production,
ii) subjecting the partly degasified biomass material to one or more separation steps resulting in the provision of
   a) a fibrous solid fraction comprising organic and inorganic nitrogen (N) parts, and
   b) at least one liquid fraction comprising solid and liquid organic and inorganic nitrogen and phosphor (P) containing parts,
iii) subjecting said fibrous solid fraction to a heating and drying treatment, such as by heating said solid fraction to a temperature of at least 80°C and preferable 90°C and adjusting the pH to alkaline conditions, such as a pH of at least 9.0 or more such as 10.5, such as by adding CaO sufficient to strip volatile nitrogen containing compounds, and thereby generating:
   e) a dry fibrous solid fraction or substrate having a reduced amount of volatile nitrogen containing compounds, including ammonium and inorganic nitrogen precursor volatile compounds,
   f) a first gaseous fraction comprising nitrogen containing volatile compounds, including ammonia, CO₂ and water and preferably having a temperature of at least 80°C and preferable about 90°C,
iv) subjecting said liquid fraction to one or more separation steps, resulting in the provision of
   a) a fibrous liquid fraction or concentrate having a reduced amount of volatile nitrogen containing compounds, including ammonium and inorganic nitrogen precursor volatile compounds, and
   b) an essentially non-fibrous liquid fraction or permeate having an increased amount of volatile nitrogen containing compounds and a reduced amount of organic nitrogen compounds,
v) subjecting the said concentrate to a N-stripping and sedimentation tank wherein the pH is adjusted to alkaline conditions, such as at least pH 9.0 or more, such as about pH 10.5 for example by adding limestone and heating said fibrous liquid fraction to a temperature sufficient to strip volatile nitrogen containing compounds, such as a temperature of at least 70°C and preferable 80°C by injection of a heated gaseous fraction comprising nitrogen containing volatile compounds, including ammonia, CO₂ and water, such as by injection of the first gaseous fraction, thereby generating:
   a) a fibrous concentrate or substrate suitable as substrate for biogas production, wherein said fibrous concentrate or substrate is preferably heated to a temperature of at least 70°C and wherein said concentrate or substrate has a reduced amount of volatile nitrogen containing compounds, including ammonium and inorganic nitrogen precursor volatile compounds, and a reduced amount of inorganic solid and phosphor (P) containing compounds,
   b) a second gaseous fraction comprising nitrogen containing volatile compounds, including ammonia, CO₂ and water and preferably having a temperature of at least 70°C,
   c) a phosphor (P) comprising fraction or sediment containing mainly inorganic solid and phosphor (P) containing compounds,
vi) subjecting the essentially non-fibrous liquid fraction or permeate to a second N-stripping and sedimentation tank, inject a gaseous fraction comprising nitrogen containing volatile compounds, including ammonia, CO₂ and water, such as the second gaseous fraction, adjust the pH and temperature to shift the balance from ammonia to ammonium, such as by adding an acid to reduce the pH to about 6.5 or less and preferable about 6.0 and by reducing the temperature to about 15°C or less, such as about 10°C, thereby generating
   a) a liquid N-fertilizer fraction having an increased amount of volatile nitrogen containing compounds, including ammonium and a reduced amount of solid and phosphor (P) containing compounds,
   b) a phosphor (P) comprising fraction or sediment containing mainly inorganic solid and phosphor (P) containing compounds
   c) a heating source suitable for drying and other heating purposes
vii) subjecting the said phosphor (P) comprising fractions or sediments to a heating and drying treatment such as by heating said sediments to a temperature of at least 70°C sufficient to evaporate water and volatile nitrogen containing compounds, and thereby generating
   a) a dry phosphor (P) comprising fraction or P-fertilizer containing mainly inorganic solid and phosphor (P) containing compounds.

A partly degasified biomass material is used interchangeably herein with a biomass material comprising solid and liquid parts obtained from a biogas fermenter following an anaerobic fermentation and biogas production.

The above method thus generates the following usable products:
a) a fibrous solid fraction
b) a N-fertilizer
c) a P-fertilizer, and
d) a heating source.

The fibrous solid fraction can e.g. be used as substrate for mushroom production and thereafter re-used as substrate for further biogas production according to the disclosure herein and as disclosed in PCT/DK2014/050220 (WO 2015/007290).

The fertilizers obtained by the methods disclosed herein can be used in agriculture.

The heat generated by the method can be recycled in the process steps of the method via heat pumps or used as a separate heating source.

In one embodiment according to the present disclosure, there is provided a method for generating one or more fertilizers, such as one or more N or P-fertilizers comprising the steps of:
i) providing a partly degasified biomass material comprising solid and liquid nitrogen (N) and phosphor (P) containing parts from a biogas fermenter following an anaerobic fermentation and biogas production,
ii) subjecting the partly degasified biomass material to one or more separation steps resulting in the provision of
   a) a fibrous solid fraction comprising organic and inorganic nitrogen (N) parts, and
   b) at least one liquid fraction comprising solid and liquid organic and inorganic nitrogen (N) and phosphor (P) containing parts,
iii) subjecting said liquid fraction to one or more separation steps, resulting in the provision of
   a) a fibrous liquid fraction or concentrate, and
   b) an essentially non-fibrous liquid fraction or permeate,
iv) subjecting the essentially non-fibrous liquid fraction or permeate to a gaseous fraction comprising nitrogen containing volatile compounds, wherein the pH and temperature are individually adjusted to shift the balance from ammonia to ammonium, thereby generating
   a) an N-comprising fraction or N-fertilizer in liquid form,
   b) a P-comprising fraction or sediment,
   c) a heating source suitable for drying and other heating purposes,
v) optionally subjecting the P-comprising fraction or sediment to a heating and drying treatment sufficient to evaporate water and volatile nitrogen (N) containing compounds, and thereby generating
   a) a P-comprising fraction or P-fertilizer.

The permeate is high in NH⁴⁺-N content but low in Org N and P content. For step iv) above, the permeate is usually led into a Nitrogen stripper tank, such as NS2, wherein absorption of ammonia into the liquid phase can take place due to a combination of low temperature and low pH that shift the balance from ammonia to ammonium. For example, the permeate may in a first step be heated by injection of the second gaseous fraction from NS1 and thereafter cooled by use of a heat pump and pH may be individually adjusted by adding acid, such as to pH 6.5 or lower, such as 6.0, for example 5.5.

In NS2, sedimentation of inorganic solids will take place and thereby is generated:
a) a cooled permeate being an N-enriched liquid fertilizer high in NH⁴⁺-N content and low in inorganic solid content that can be stored until final use as fertilizer in agriculture,
b) a sediment containing mainly inorganic solids and phosphor containing compounds, and
c) a third gaseous fraction or N-steam 3 comprising nitrogen containing compounds, including ammonia, CO₂ and water having a temperature of 30°C or less that can be finally treated in a bio-filter.

Sediments from NS1 and NS2 with high content of P and low in volatile dry matters can be pumped to a sedimentation and dewatering device (Sed & dew device) thereby generating:
a) a P-sediment or dewatered P-sed with dry matter content of 25% or more,
b) a liquid fraction or permeate which may be pumped to and incorporated in the N-enriched liquid fertilizer.

P-sediment may be further dried in a drum dryer and/or band dryer and thus converted to high valuable dry P-fertilizer.

In one embodiment according to the present disclosure, there is provided a method for generating one or more fertilizers, such as one or more N- and P-fertilizers comprising the steps of:
i) providing a an essentially non-fibrous liquid fraction or permeate having an increased amount of volatile nitrogen containing compounds and a reduced amount of organic nitrogen compounds obtained by fractionating a spent biomass material, such as a permeate fraction disclosed herein above and/or in PCT/DK2014/050220 (WO 2015/007290),
ii) subjecting the essentially non-fibrous liquid fraction or permeate to a second N-stripping and sedimentation tank, inject a gaseous fraction comprising nitrogen containing volatile compounds, including ammonia, CO₂ and water, adjust the pH and temperature to shift the balance from ammonia to ammonium, such as by adding an acid to reduce the pH to about 6.5 or less and preferable about 6.0 and by reducing the temperature to about 15°C or less, such as about 10°C, thereby generating
   a) a liquid N-fertilizer fraction,
   b) a phosphor (P) comprising fraction or sediment,
   c) a heating source suitable for drying and other heating purposes,
iii) optionally subjecting the said phosphor (P) comprising fractions or sediments to a heating and drying treatment such as by heating said sediments to a temperature of at least 70°C sufficient to evaporate water and volatile nitrogen containing compounds, and thereby generating a) a dry phosphor (P) comprising fraction or P-fertilizer.

In one embodiment according to the present disclosure the fibrous liquid fraction or concentrate has a reduced amount of volatile nitrogen containing compounds, including ammonium and inorganic nitrogen precursor volatile compounds as compared to the liquid fraction.

In one embodiment according to the present disclosure the essentially non-fibrous liquid fraction or permeate has an increased amount of volatile nitrogen containing compounds and a reduced amount of organic nitrogen compounds.

In one embodiment according to the present disclosure the essentially non-fibrous liquid fraction or permeate has a solids or dry matter content of less than about 10%, for example less than about 5%, such as less than 2%.

In one embodiment according to the present disclosure the permeate is treated in an N-stripping and sedimentation tank, such as wherein the temperature and pH conditions are such that they allow for N-absorption to take place.

In one embodiment according to the present disclosure the gaseous fraction comprising nitrogen containing volatile compounds comprises ammonia and further comprises CO₂ and water. The gaseous fraction may e.g. be obtained from earlier fractionation steps of the spent biomass, e.g. from fractionating the fibrous solid fraction (resulting in a first gaseous fraction) or the concentrate (resulting in a second gaseous fraction). In one embodiment according to the present disclosure the gaseous fraction obtained from fractionating the concentrate is re-used in the step that converts the permeate into liquid N fertilizer and further fractions.

In one embodiment according to the present disclosure the pH that allows for N-absorption to take place (e.g. in NS2) is achieved by addition of an acid, such as an acid sufficient to reduce the pH to about 6.5 or less, such as about 6.0.

In one embodiment according to the present disclosure the temperature that allows for N-absorption to take place (e.g. in NS2) is a temperature of about 15°C or less, such as about 10°C.

In one embodiment according to the present disclosure the liquid N-fertilizer fraction has an increased amount of volatile nitrogen containing compounds including ammonium as compared to the permeate, and a reduced amount of solid and phosphor (P) containing compounds.

In one embodiment according to the present disclosure the phosphor (P) comprising fraction or sediment contains mainly inorganic solid and phosphor (P) containing compounds.

In one embodiment according to the present disclosure the heating and drying treatment sufficient to evaporate water and volatile nitrogen containing compounds is by heating to a temperature of at least 70°C, such as at least 75°C, for example at least 80°C.

In one embodiment according to the present disclosure the P-fertilizer is essentially dry, such as dry.

In one embodiment according to the present disclosure the P-fertilizer contains mainly inorganic solid and phosphor (P) containing compounds.

In one embodiment according to the present disclosure the fibrous solid fraction comprising organic and inorganic nitrogen (N) parts is subjected to a heating and drying treatment sufficient to strip volatile nitrogen containing compounds, such as by heating said solid fraction to a temperature of at least 80°C and preferable 90°C and adjusting the pH to alkaline conditions, such as a pH of at least 9.0 or more such as 10.5, such as by adding CaO, and thereby generating:
a) a dry fibrous solid fraction or substrate having a reduced amount of volatile nitrogen containing compounds, including ammonium and inorganic nitrogen precursor volatile compounds,
b) a first gaseous fraction comprising nitrogen containing volatile compounds, including ammonia, CO₂ and water and preferably having a temperature of at least 80°C and preferably about 90°C.

In one embodiment according to the present disclosure the fibrous liquid fraction or concentrate is subjected to a N-stripping and sedimentation tank, wherein the pH and temperature is adjusted in order to strip volatile nitrogen containing compounds alkaline conditions, such as by adjusting the pH to at least 9.0 or more, such as about pH 10.5, for example by adding limestone, and by heating said fibrous liquid fraction to a temperature of at least 70°C, such as about 80°C by injection of a heated gaseous fraction comprising nitrogen containing volatile compounds, including ammonia, CO₂ and water, such as by injection of the first gaseous fraction obtained from fractionating the fibrous solid fraction, thereby generating:
a) a fibrous concentrate or substrate suitable as substrate for biogas production, wherein said fibrous concentrate or substrate is preferably heated to a temperature of at least 70°C and wherein said concentrate or substrate has a reduced amount of volatile nitrogen containing compounds, including ammonium and inorganic nitrogen precursor volatile compounds, and a reduced amount of inorganic solid and phosphor (P) containing compounds,
b) a second gaseous fraction comprising nitrogen containing volatile compounds, including ammonia, CO₂ and water and preferably having a temperature of at least 70°C, and
c) a phosphor (P) comprising fraction or sediment containing mainly inorganic solid and phosphor (P) containing compounds.

The following items further define these aspects according to the present disclosure:
Item 1. A method for generating one or more fertilizers, such as one or more N or P-fertilizers comprising the steps of:
   i) providing a partly degasified biomass material comprising solid and liquid nitrogen (N) and phosphor (P) containing parts from a biogas fermenter following an anaerobic fermentation and biogas production,
   ii) subjecting the partly degasified biomass material to one or more separation steps resulting in the provision of
      a) a fibrous solid fraction comprising organic and inorganic nitrogen (N) parts, and
      b) at least one liquid fraction comprising solid and liquid organic and inorganic nitrogen (N) and phosphor (P) containing parts,
   iii) subjecting said liquid fraction to one or more separation steps, resulting in the provision of
      a) a fibrous liquid fraction or concentrate, and
      b) an essentially non-fibrous liquid fraction or permeate,
   iv) subjecting the essentially non-fibrous liquid fraction or permeate to a gaseous fraction comprising nitrogen containing volatile compounds, wherein the pH and temperature are individually adjusted to shift the balance from ammonia to ammonium, thereby generating
      a) an N-comprising fraction or N-fertilizer in liquid form,
      b) a P-comprising fraction or sediment,
      c) a heating source suitable for drying and other heating purposes,
   v) optionally subjecting the P-comprising fraction or sediment to a heating and drying treatment sufficient to evaporate water and volatile nitrogen (N) containing compounds, and thereby generating
      a) a P-comprising fraction or P-fertilizer.
Item 2. The method according to any of the preceding claims, wherein the fibrous liquid fraction or concentrate has a reduced amount of volatile nitrogen containing compounds, including ammonium and inorganic nitrogen precursor volatile compounds as compared to the liquid fraction.
Item 3. The method according to any of the preceding items, wherein the essentially non-fibrous liquid fraction or permeate has an increased amount of volatile nitrogen containing compounds and a reduced amount of organic nitrogen compounds.
Item 4: The method according to any of the preceding items, wherein the essentially non-fibrous liquid fraction or permeate has a solids or dry matter content of less than about 10%, for example less than about 5%, such as less than 2%.
Item 5: The method according to any of the preceding items, wherein step iv) is performed in a N-stripping and sedimentation tank.
Item 6: The method according to any of the preceding items, wherein the gaseous fraction comprising nitrogen containing volatile compounds comprises ammonia and further comprises CO₂ and water.
Item 7: The method according to any of the preceding items, wherein the pH in step iv) is adjusted by addition of an acid, such as an acid sufficient to reduce the pH to about 6.5 or less, such as about 6.0.
Item 8: The method according to any of the preceding items, wherein the temperature in step iv) is reduced to about 15°C or less, such as about 10°C.
Item 9: The method according to any of the preceding items, wherein the liquid N-fertilizer fraction has an increased amount of volatile nitrogen containing compounds including ammonium as compared to the permeate, and a reduced amount of solid and phosphor (P) containing compounds.
Item 10: The method according to any of the preceding items, wherein the phosphor (P) comprising fraction or sediment contains mainly inorganic solid and phosphor (P) containing compounds.
Item 11: The method according to any of the preceding items, wherein the heating and drying treatment of step v) sufficient to evaporate water and volatile nitrogen containing compounds is by heating to a temperature of at least 70°C.
Item 12: The method according to any of the preceding items, wherein the P-fertilizer is dry.
Item 13: The method according to any of the preceding claims, wherein the P-fertilizer contains mainly inorganic solid and phosphor (P) containing compounds.
Item 14: The method according to any of the preceding items, wherein the fibrous solid fraction comprising organic and inorganic nitrogen (N) parts is subjected to a heating and drying treatment sufficient to strip volatile nitrogen containing compounds, such as by heating said solid fraction to a temperature of at least 80°C and preferable 90°C and adjusting the pH to alkaline conditions, such as a pH of at least 9.0 or more such as 10.5, such as by adding CaO, and thereby generating:
   a) a dry fibrous solid fraction or substrate having a reduced amount of volatile nitrogen containing compounds, including ammonium and inorganic nitrogen precursor volatile compounds,
   b) a first gaseous fraction comprising nitrogen containing volatile compounds, including ammonia, CO₂ and water and preferably having a temperature of at least 80°C and preferably about 90°C.
Item 15: The method according to any of the preceding items, wherein the fibrous liquid fraction or concentrate is subjected to a N-stripping and sedimentation tank, wherein the pH and temperature is adjusted in order to strip volatile nitrogen containing compounds alkaline conditions, such as by adjusting the pH to at least 9.0 or more, such as about pH 10.5, for example by adding limestone, and by heating said fibrous liquid fraction to a temperature of at least 70°C, such as about 80°C by injection of a heated gaseous fraction comprising nitrogen containing volatile compounds, including ammonia, CO₂ and water, such as by injection of the first gaseous fraction of claim 13, thereby generating:
   a) a fibrous concentrate or substrate suitable as substrate for biogas production, wherein said fibrous concentrate or substrate is preferably heated to a temperature of at least 70°C and wherein said concentrate or substrate has a reduced amount of volatile nitrogen containing compounds, including ammonium and inorganic nitrogen precursor volatile compounds, and a reduced amount of inorganic solid and phosphor (P) containing compounds,
   b) a second gaseous fraction comprising nitrogen containing volatile compounds, including ammonia, CO₂ and water and preferably having a temperature of at least 70°C, and
   c) a phosphor (P) comprising fraction or sediment containing mainly inorganic solid and phosphor (P) containing compounds.
Item 16: A method fractionating a partly degasified biomass material, said partly degasified biomass material being a biomass material comprising solid and liquid parts, comprising the steps of:
   i) providing a partly degasified biomass material comprising solid and liquid nitrogen (N) and phosphor (P) containing parts from a biogas fermenter following an anaerobic fermentation and biogas production,
   ii) subjecting the partly degasified biomass material to one or more separation steps resulting in the provision of
      a) a fibrous solid fraction comprising organic and inorganic nitrogen (N) parts, and
      b) at least one liquid fraction comprising solid and liquid organic and inorganic nitrogen and phosphor (P) containing parts,
   iii) subjecting said fibrous solid fraction to a heating and drying treatment, such as by heating said solid fraction to a temperature of at least 80°C and preferable 90°C and adjusting the pH to alkaline conditions, such as a pH of at least 9.0 or more such as 10.5, such as by adding CaO sufficient to strip volatile nitrogen containing compounds, and thereby generating:
      g) a dry fibrous solid fraction or substrate having a reduced amount of volatile nitrogen containing compounds, including ammonium and inorganic nitrogen precursor volatile compounds,
      h) a first gaseous fraction comprising nitrogen containing volatile compounds, including ammonia, CO₂ and water and preferably having a temperature of at least 80°C and preferable about 90°C,
   iv) subjecting said liquid fraction to one or more separation steps, resulting in the provision of
      a) a fibrous liquid fraction or concentrate having a reduced amount of volatile nitrogen containing compounds, including ammonium and inorganic nitrogen precursor volatile compounds, and
      b) an essentially non-fibrous liquid fraction or permeate having an increased amount of volatile nitrogen containing compounds and a reduced amount of organic nitrogen compounds,
   v) subjecting the said concentrate to a N-stripping and sedimentation tank wherein the pH is adjusted to alkaline conditions, such as at least pH 9.0 or more, such as about pH 10.5 for example by adding limestone and heating said fibrous liquid fraction to a temperature sufficient to strip volatile nitrogen containing compounds, such as a temperature of at least 70°C and preferable 80°C by injection of a heated gaseous fraction comprising nitrogen containing volatile compounds, including ammonia, CO₂ and water, such as by injection of the first gaseous fraction, thereby generating:
      a) a fibrous concentrate or substrate suitable as substrate for biogas production, wherein said fibrous concentrate or substrate is preferably heated to a temperature of at least 70°C and wherein said concentrate or substrate has a reduced amount of volatile nitrogen containing compounds, including ammonium and inorganic nitrogen precursor volatile compounds, and a reduced amount of inorganic solid and phosphor (P) containing compounds,
      b) a second gaseous fraction comprising nitrogen containing volatile compounds, including ammonia, CO₂ and water and preferably having a temperature of at least 70°C,
      c) a phosphor (P) comprising fraction or sediment containing mainly inorganic solid and phosphor (P) containing compounds,
   vi) subjecting the essentially non-fibrous liquid fraction or permeate to a second N-stripping and sedimentation tank, inject a gaseous fraction comprising nitrogen containing volatile compounds, including ammonia, CO₂ and water, such as the second gaseous fraction, adjust the pH and temperature to shift the balance from ammonia to ammonium, such as by adding an acid to reduce the pH to about 6.5 or less and preferable about 6.0 and by reducing the temperature to about 15°C or less, such as about 10°C, thereby generating
      a) a liquid N-fertilizer fraction having an increased amount of volatile nitrogen containing compounds, including ammonium and a reduced amount of solid and phosphor (P) containing compounds,
      b) a phosphor (P) comprising fraction or sediment containing mainly inorganic solid and phosphor (P) containing compounds
      c) a heating source suitable for drying and other heating purposes
   vii) subjecting the said phosphor (P) comprising fractions or sediments to a heating and drying treatment such as by heating said sediments to a temperature of at least 70°C sufficient to evaporate water and volatile nitrogen containing compounds, and thereby generating
      a dry phosphor (P) comprising fraction or P-fertilizer containing mainly inorganic solid and phosphor (P) containing compounds.

### Examples

Table 1 shows the characteristics for types of waste products and biomasses suitable for production of mushroom substrate and the possible conversion of organic N to ammonia N if used as single biomass as feed stock for biogas.

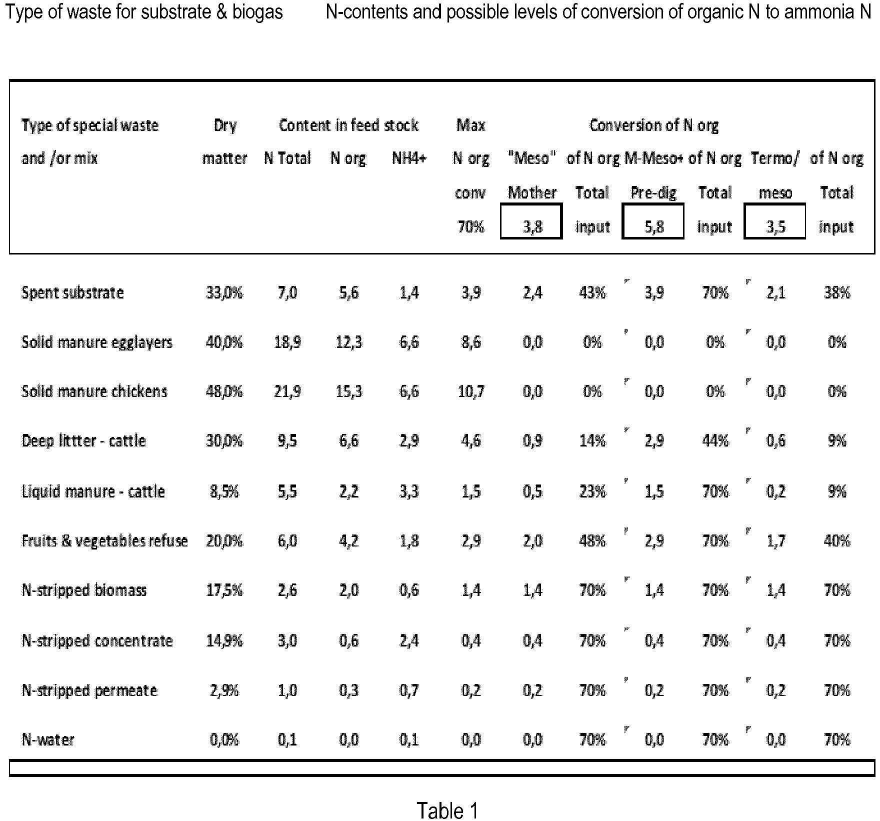

Use of spent substrate in feed stocks for biogas is illustrated in tables 2, 3 and 4.

### Example 1: Spent substrate + manure egglayers & chicken&cattle + N-stripped biomass + N-water

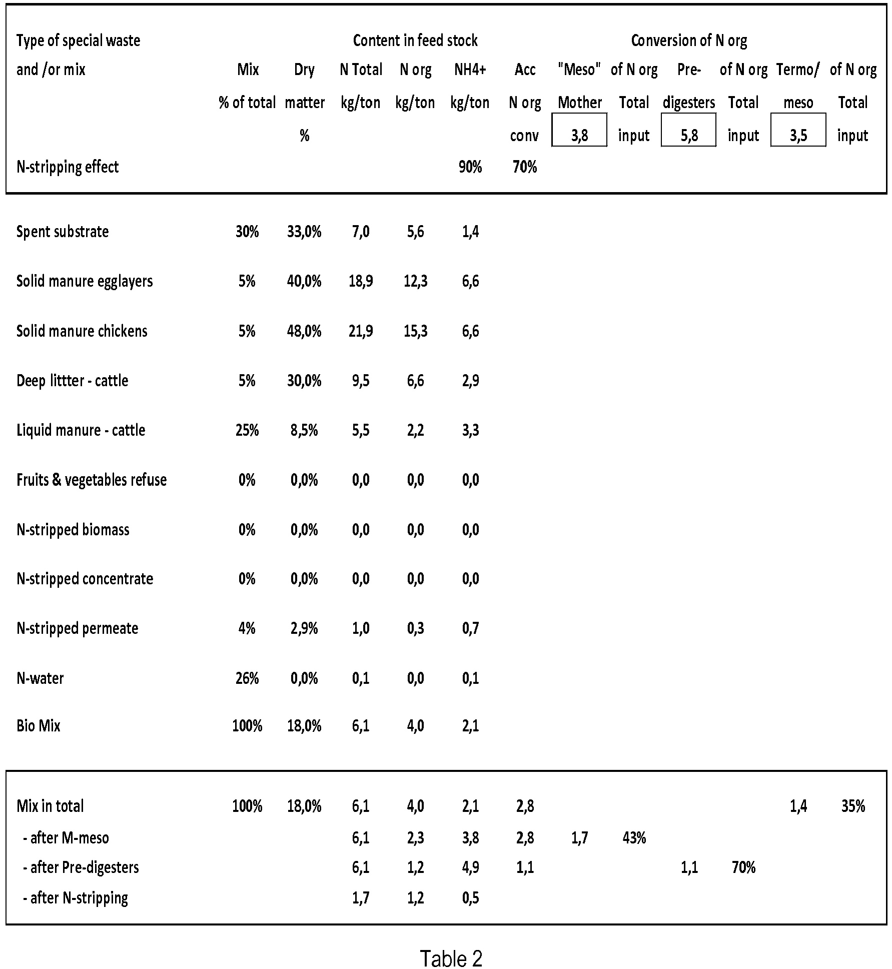

### Example 2: Spent substrate + manure egglayers & cattle + N-stripped biomass + N-water

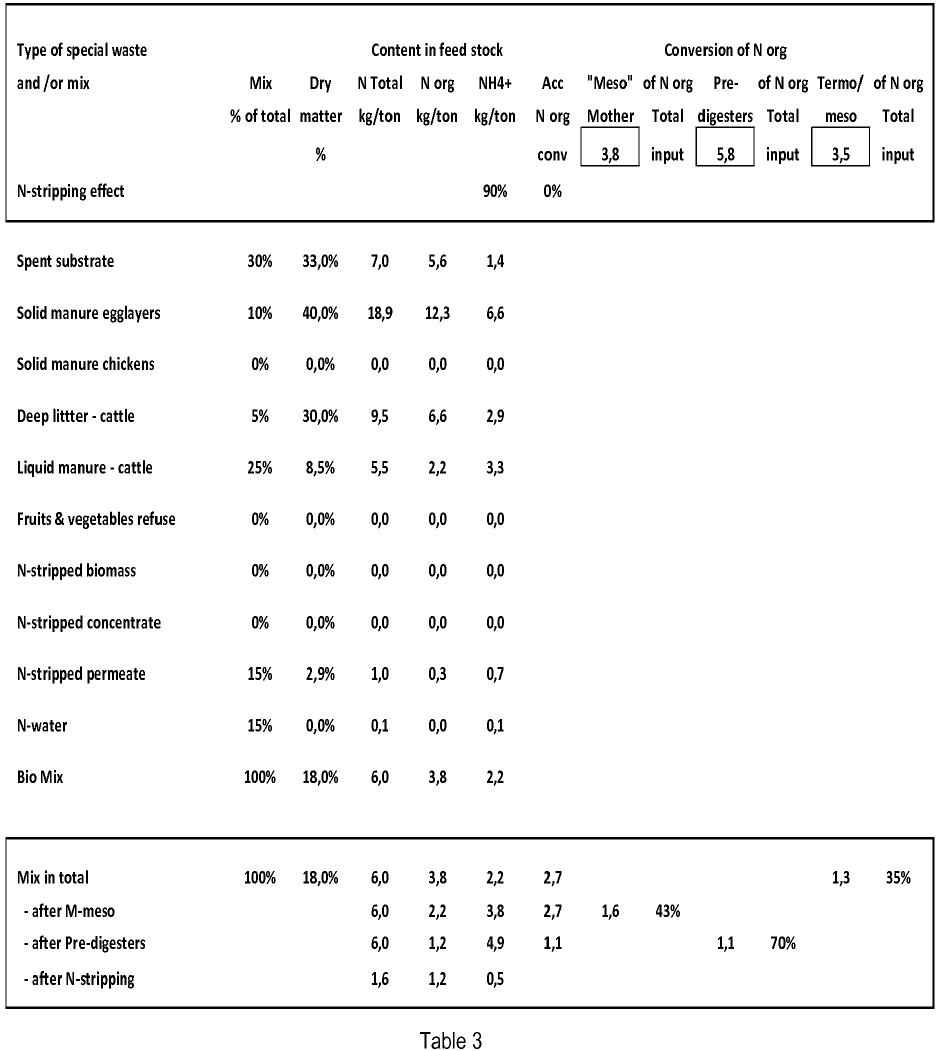

### Example 3: Spent substrate + manure egglayers+fruits&vegetable refuse + N-stripped biomass + N-water

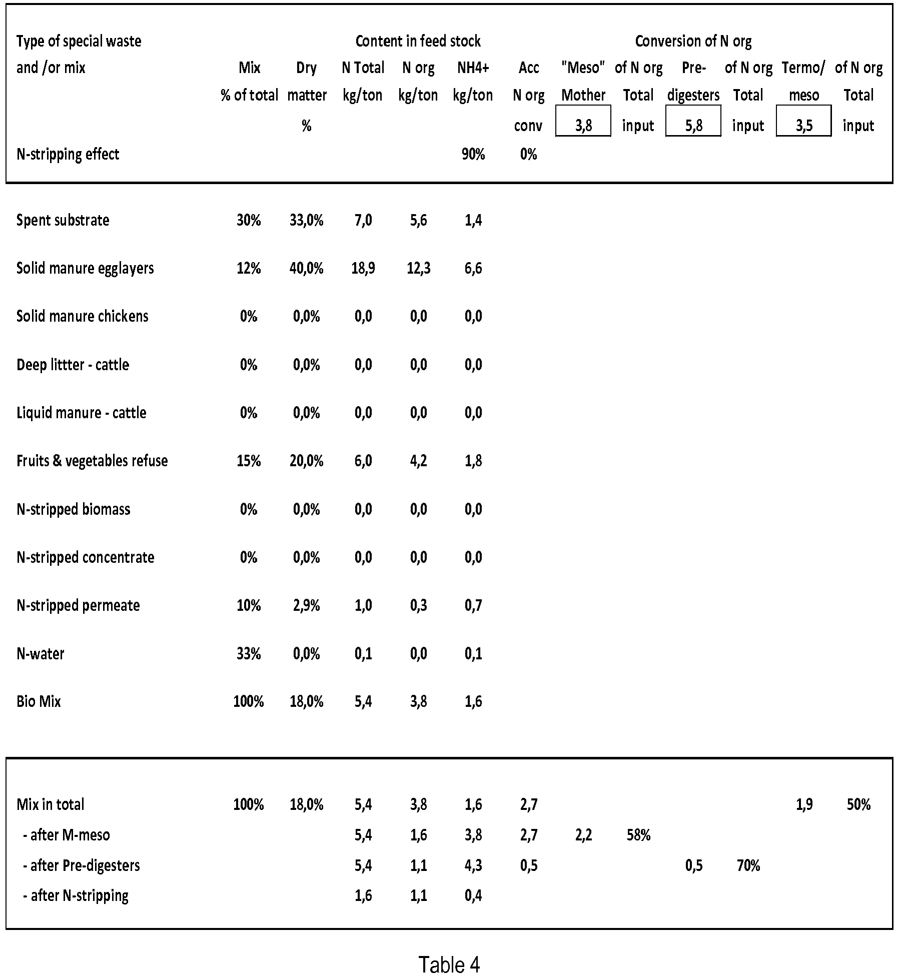

In Examples 1 and 2 the advantages linked to the present disclosure are clearly visible. Without recycling of N-stripped permeate and dilution with N- water from N-absorption, the feed stock mix will have an inhibitory level of NH4⁺-N before entering the anaerobic digesters. In the absence of N stripping, one would have to mix organic materials high in N with organic materials low in N - i.e. add high N containing organic materials in small portions to a mixture of organic materials to be fermented.

The anaerobic fermentation resulting in the production of biogas is followed by one or more processing steps aimed to strip ammonia N from the organic material after to the biogas production from pre-digestion in fermentation facilities, heating and drying.

The data in Table 5 are based on a specific mix of biomass and interaction between a "mushroom substrate" production unit with Meso Mother reactor, pre-reactors, dryer, N- strippers and N-absorber facilities and a conventional termo/meso biogas plant with Sep & Sed facilities as add on.

The termo/meso biogas plant is supplying fiber from separation to the "mushroom substrate" production unit and gets in return N-stripped pre-digested biomass and N-stripped concentrate - both pre-heated - as new feed stock. The main scope is to produce basic substrate for mushroom production according to Figure 8 and adjust moisture content by adding N-water and specific types of supplementary fibers.

Based on the findings in Table 5 the share of basic substrate will depend on desired content moisture and composition of nutrients in the final substrate.

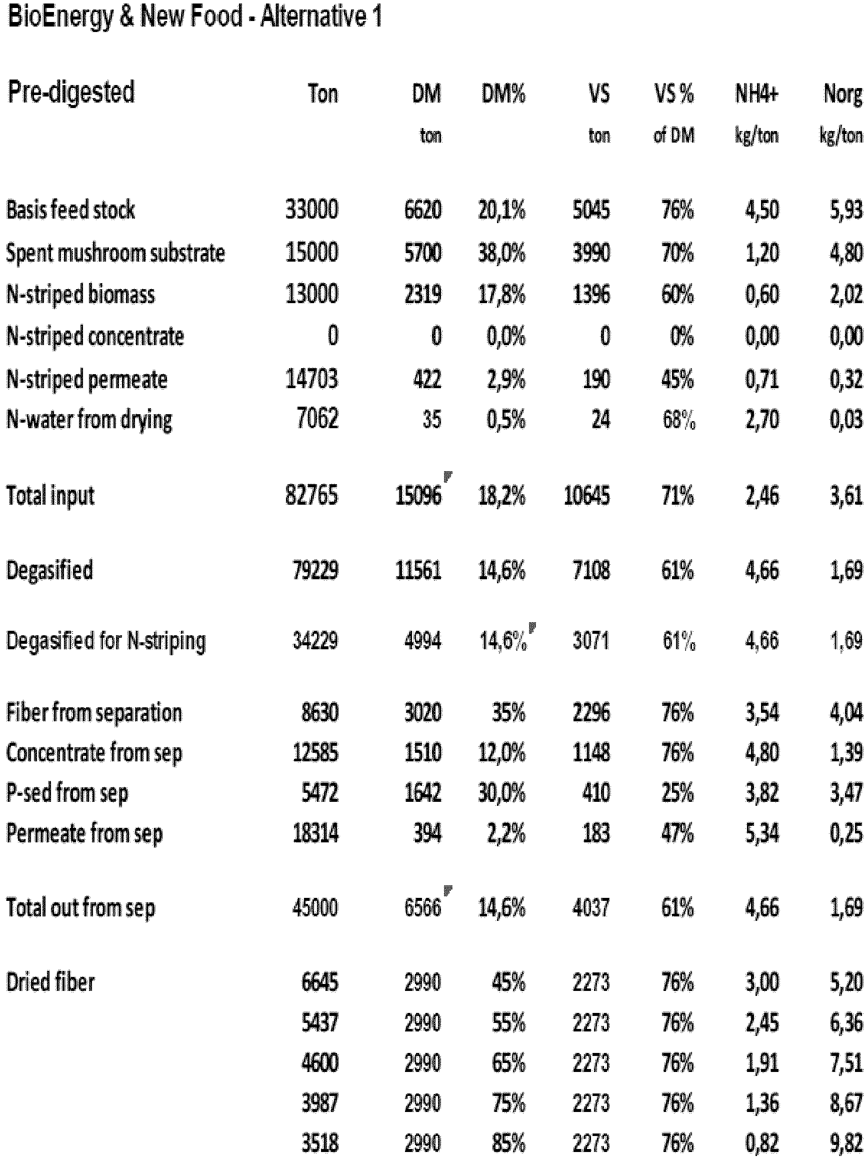

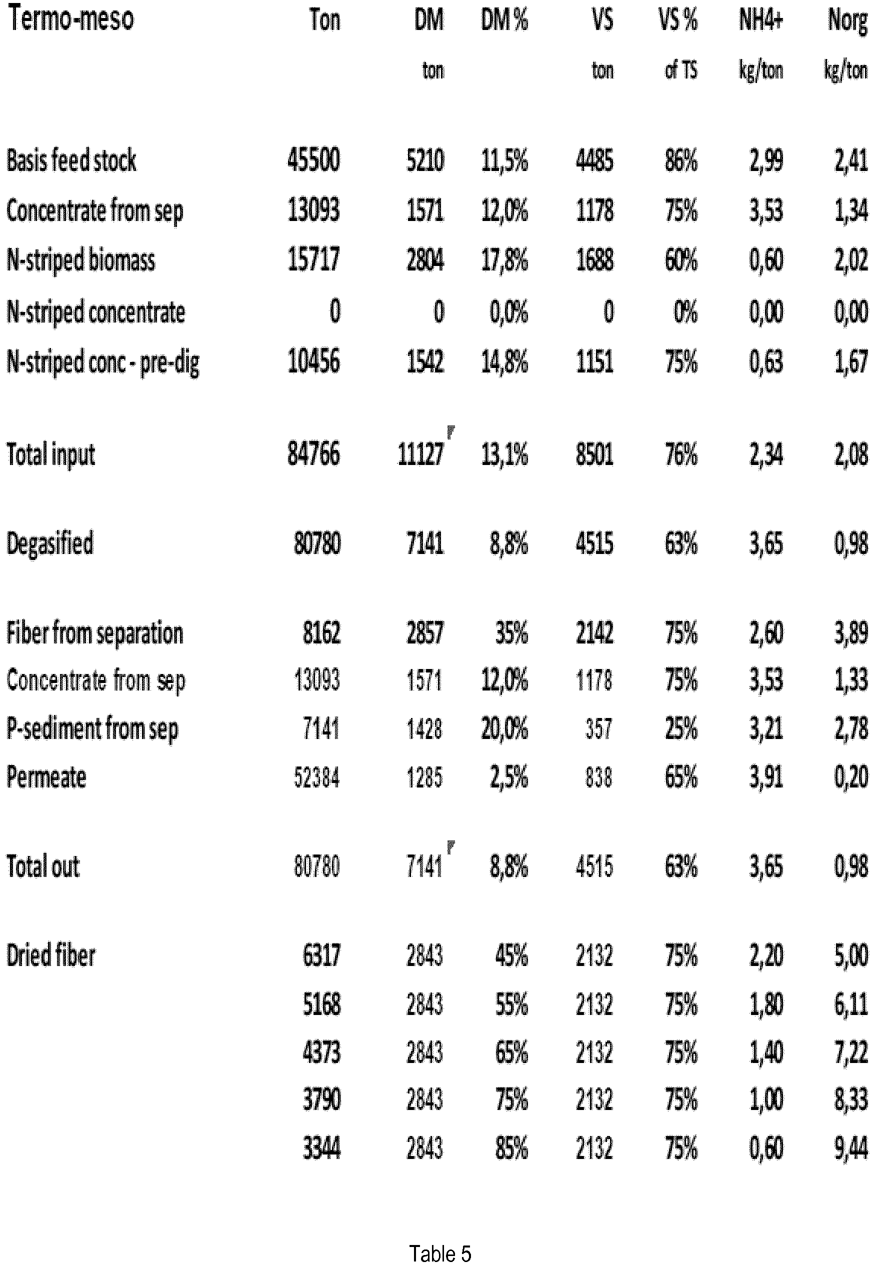

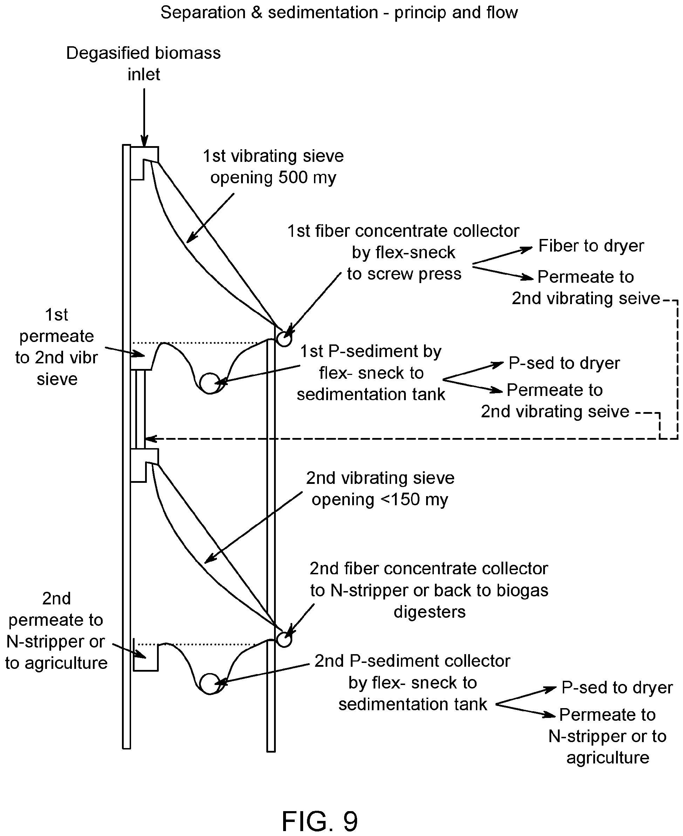

In Table 6 is a scenario to be considered as minimum share equal to a situation where 70% of the dry matter comes from dried pre-digested or dried termo/meso fibers. Size of production is 18000 ton of mushroom substrate, 3000 ton of mushrooms and 15000 ton of spent substrate, as illustrated in figure 9.

**Table 6**

| **New food linked to Bioenergy Plant** | | | | **Number of bottles 15 mio** | | | | | |
|---|---|---|---|---|---|---|---|---|---|
| INPUT | | | | | | | | | |
| | **DM in %** | **% of total DM** | **g pr bottle** | **value Euro/kg** | **uro cent or bottle** | **Total ton** | **Total mio Euro** | **NH4 kg/ton** | **Norg kg/ton** |
| **Dried fiber 1** | **45%** | **35%** | **309** | **0,10** | **30,0** | **4631** | **0,45** | **3,00** | **5,20** |
| **Dried fiber 2** | **45%** | **35%** | **309** | **0,08** | **26,0** | **4631** | **0,39** | **2,20** | **5,00** |
| **Rise bran** | **90%** | **0%** | **0** | **0,22** | **0,0** | **0** | **0,00** | **0,00** | **0,00** |
| **Residue from soya** | **90%** | **3%** | **13** | **0,35** | **4,6** | **198** | **0,07** | **0,00** | **0,00** |
| **Wheat bran** | **90%** | **5%** | **22** | **0,19** | **4,2** | **331** | **0,06** | **0,30** | **23,00** |
| **Oister shells** | **90%** | **0%** | **0** | **0,06** | **0,0** | **0** | **0,00** | **0,00** | **0,00** |
| **Sugar beet pulp** | **90%** | **2%** | **9** | **0,32** | **2,8** | **132** | **0,04** | **0,10** | **12,00** |
| **Sawdust dried** | **90%** | **20%** | **88** | **0,08** | **7,1** | **1323** | **0** | **0,00** | **0,00** |
| **Total** | **53%** | **100%** | **750** | **0,10** | **74,7** | **11246** | **1,12** | **2,15** | **5,02** |
| **Drymatter** | | | **397** | | | | | | |
| **Water** | | | **353** | | | | | | |
| **Added N-water** | **0%** | | **453** | **0,00** | **0,0** | **6795** | **0,00** | **0,10** | **0,00** |
| | | | | | | | | | |

| Substrate | | | | | | | | | |
|---|---|---|---|---|---|---|---|---|---|
| **Total** | **33%** | | **1203** | | **74,7** | **18041** | **1,12** | **1,38** | **3,13** |

In Table 7 is a scenario to be considered as maximum share equal to a situation where 90% of the dry matter comes from dried pre-digested or dried termo/meso fibers.

**Table 7**

| **Substrate production - Mushroom** | | | | **Number of bottles 15 mio** | | | | | |
|---|---|---|---|---|---|---|---|---|---|
| **Input** | **DM in %** | **% of total DM** | **g pr bottle** | **value Euro/kg** | **Euro cent pr bottle** | **Total ton** | **Total mio Euro** | **NH4 kg/ton** | **Norg kg/ton** |
| **Dried fiber 1** | **85%** | **45%** | **210** | **0,10** | **20,4** | **3152** | **0,31** | **0,82** | **9,82** |
| **Dried fiber 2** | **85%** | **45%** | **210** | **0,08** | **17,7** | **3152** | **0,27** | **0,60** | **9,44** |
| **Rise bran** | **90%** | **0%** | **0** | **0,22** | **0,0** | **0** | **0,00** | **0,00** | **0,00** |
| **Residue from soya** | **90%** | **3%** | **13** | **0,35** | **4,6** | **198** | **0,07** | **0,00** | **0,00** |
| **Wheat bran** | **90%** | **5%** | **22** | **0,19** | **4,2** | **331** | **0,06** | **0,30** | **23,00** |
| **Oister shells** | **90%** | **0%** | **0** | **0,06** | **0,0** | **0** | **0,00** | **0,00** | **0,00** |
| **Sugar beet pulp** | **90%** | **2%** | **9** | **0,32** | **2,8** | **132** | **0,04** | **0,10** | **12,00** |
| **Sawdust dried** | **90%** | **0%** | **0** | **0,08** | **0,0** | **0** | **0,00** | **0,00** | **0,00** |
| **Total** | **85%** | **100%** | **464** | **0,11** | **49,7** | **6965** | **0,75** | **0,66** | **10,04** |
| **Drymatter** | | | **397** | | | | | | |
| **Water** | | | **67** | | | | | | |
| **Added N-water** | **0%** | | **738** | **0,00** | **0,0** | **11076** | **0,00** | **0,10** | **0,00** |
| **Total** | **33%** | | **1203** | | **49,7** | **18041** | **0,75** | **0,32** | **3,87** |

Table 8 below contains an illustration of one scenario to be considered as expected share equal to a situation where 80% of the dry matter comes from dried pre-digested fibers with 75% dry matter and dried termo/meso fibers with 65% dry matter.

**Table 8**

| **Substrate production - Mushroom** | | | | **Number of bottles 15 mio** | | | | | |
|---|---|---|---|---|---|---|---|---|---|
| **Input** | **DM in %** | **% of total DM** | **g pr bottle** | **value Euro/kg** | **uro cent pr bottle** | **Total ton** | **Total mio Euro** | **NH4 kg/ton** | **Norg kg/ton** |
| **Dried fiber 1** | **75%** | **40%** | **212** | **0,10** | **20,6** | **3175** | **0,31** | **1,36** | **8,67** |
| **Dried fiber 2** | **65%** | **40%** | **244** | **0,08** | **20,6** | **3664** | **0,31** | **1,40** | **7,22** |
| **Rise bran** | **90%** | **0%** | **0** | **0,22** | **0,0** | **0** | **0,00** | **0,00** | **0,00** |
| **Residue from soya** | **90%** | **3%** | **13** | **0,35** | **4,6** | **198** | **0,07** | **0,00** | **0,00** |
| **Wheat bran** | **90%** | **5%** | **22** | **0,19** | **4,2** | **331** | **0,06** | **0,30** | **23,00** |
| **Oister shells** | **90%** | **0%** | **0** | **0,06** | **0,0** | **0** | **0,00** | **0,00** | **0,00** |
| **Sugar beet pulp** | **90%** | **2%** | **9** | **0,32** | **2,8** | **132** | **0,04** | **0,10** | **12,00** |
| **Sawdust dried** | **90%** | **10%** | **44** | **0,08** | **3,5** | **662** | **0,05** | **0,00** | **0,00** |
| **Total** | **73%** | **100%** | **544** | **0,10** | **56,3** | **8162** | **0,84** | **1,17** | **7,74** |
| **Drymatter** | | | **397** | | | | | | |
| **Water** | | | **147** | | | | | | |
| **Added N-water** | **0%** | | **659** | **0,00** | **0,0** | **9879** | **0,00** | **0,10** | **0,00** |
| **Total** | **33%** | | **1203** | | **56,3** | **18041** | **0,84** | **0,59** | **3,50** |

### Example 4 - Use of compressed fibrous solid substrate for cultivating mushrooms

The compressed fibrous solid substrate may be used as a "ready to use" substrate for production of several types of mushroom including - but not limited to - White Button, Eryngii, Enoki and Shitake. The "ready to use" term has to be understood as a substrate that can be stored until time of use and when used it is easy to handle - just add water and mycelium.

Important for a "ready to use" substrate is that it contains the needed nutrients - protein, C, N, P, K and more - and that it absorbs water easy and fast and can keep water and still remain fluffy enough to avoid creation of anaerobic zones during mushroom production.

The desired nutrients composition is obtained by adding one or more additional protein and/or C sources to the dry fibrous solid fraction and the structure is maintained by use of dies in the pellet press with openings or holes with 12 to 16 mm and a press way that secures a compression of the material and a density of the pellets of 450 to 550 kg per m3 - a density that is 6 to 8 times higher than the density of the dry fibrous solid substrate before compression.

After pressing the pellets have dry content of 90% or more and a temperature of 90°C or more and therefore cooling is needed before packaging in bags and the "ready to use" substrate pellets can be stored until time of use.

Use of the substrate pellets takes place at the mushroom production units. At time of use the normal desired moisture content of 67% in the substrate is reached by adding water: 2.8 ton of water per ton of substrate pellets is usual.

Finally mycelium is added and the production cycle is up and running.

After use the spent mushroom substrate with its content of organic dry matter and nutrients can be recycled back and used as feed stock for biogas production.

### Example 5 - Use of compressed fibrous solid substrate for production of litter pellets for animals

The compressed fibrous solid substrate as disclosed herein may also conveniently be used for litter pellets for a large variety of animals such as horses, poultry or cattle. Here the fibrous solid fraction is not supplemented with nutrients before compression. In the press are used dies with 10 or 12 mm holes and a press way that secures a density of up to 600 kg per m3.

The litter pellets are not pre-wetted and can be used directly as litter pellets instead of the conventionally used straw. Contrary to straw, the litter pellets as disclosed herein are not consumed by the animals. For instance, horses have a tendency to eat the straw when it is used as litter which can make it difficult to control what and how much a horse eats. Furthermore, straw consumption can also be bad for the digestion and lead to colic.

After use the litter pellets with additional manure and urine from animals can be recycled back and used as feed stock for biogas production in parallel with spent mushroom substrate.

## Claims

1. Use of a fibrous solid substrate in pellet form in a method of cultivating fungal cells and/or spores; as a fertilizer; as a soil improver; and/or as litter for animals; wherein said fibrous solid substrate in pellet form is obtained by a method comprising the steps of
a. providing a biomass material comprising solid and liquid parts from a biogas fermenter following an anaerobic fermentation and biogas production,
b. subjecting the fermented biomass material of step a. to one or more separation steps resulting in the provision of
i) a fibrous solid fraction comprising organic and inorganic nitrogen parts and having a reduced content (w/w) of water, and comprising one or more macromolecular nutrient constituents selected from the group consisting of cellulose, hemicellulose, lignin and lignocellulose, and
ii) at least one liquid fraction comprising solid and liquid organic and inorganic phosphor-containing parts,
c. subjecting the fibrous solid fraction of step b. to a sanitation treatment, said treatment
comprising the steps of i) heating the fibrous solid fraction to a temperature of more than 70°C, optionally under alkaline pH conditions, and optionally ii) subjecting the fibrous solid fraction comprising organic and inorganic nitrogen parts to a pressure of more than 1 bar,
wherein said treatment i) reduces or eliminates viable microorganisms present in the fibrous solid fraction, and/or ii) reduces the contents of volatile nitrogen-containing compounds and/or precursor volatile compounds present in the fibrous solid fraction, and
d. obtaining a fibrous solid fraction comprising organic and inorganic nitrogen parts having a reduced content of volatile nitrogen-containing compounds,
e. optionally subjecting said fibrous solid fraction to one or more of heating, drying, evaporation, pressure, and/or alkaline pH conditions,
f. optionally adding to said fibrous solid fraction one or more solid and/or liquid supplemental nutrient substrate compositions, and
g. compressing said fibrous solid fraction of step d., e. and/or f., thereby generating a fibrous solid substrate in pellet form.

2. The use according to claim 1, wherein the density of the pellet is from about 200 kg/m³ to about 800 kg/m³, such as from about 300 kg/m³ to about 700 kg/m³, for example from about 400 kg/m³ to about 600 kg/m³, such as from about 450 kg/m³ to about 550 kg/m³, and/or
wherein the density of the pellet is less than 800 kg/m³, such as less than 700 kg/m³, for example less than 650 kg/m³, for example less than 600 kg/m³, for example less than 550 kg/m³, such as less than 500 kg/m³, for example less than 450 kg/m³, such as less than 400 kg/m³.

3. The use according to any of the preceding claims, wherein the diameter of the pellet is about 6 to 20 mm, such as 6 to 8 mm, for example 8 to 10 mm, such as 10 to 12 mm, for example 12 to 14 mm, such as 14 to 16 mm, for example 16 to 18 mm, such as 18 to 20 mm, and/or
wherein compression is performed using a pellet press, and/or
wherein the density of the pellet is about 4 to 20 times higher than before the compression, such as 4 to 6 times, such as 6 to 8 times, for example 8 to 10 times, such as 10 to 12 times, for example 12 to 14 times, such as 14 to 16 times, for example 16 to 18 times, such as 18 to 20 times higher than before the compression.

4. The use according to any of the preceding claims, wherein the pellets have a dry content of at least 75%, such as at least 80%, for example at least 85%, such as at least 90%, for example 95%, and/or
wherein the pellet have a moisture content of less than or equal to 25%, such as less than or equal to 20%, for example less than or equal to 15%, such as less than or equal to 10%, for example less than or equal to 8%, and/or
wherein the pellet comprises one or more solid and/or liquid supplemental nutrient substrate compositions, and/or
wherein the one or more solid and/or liquid supplemental nutrient substrate compositions are individually selected from the group consisting of protein, C, N, P, and K, and/or
wherein the use comprises one or more solid and/or liquid supplemental nutrient substrate compositions, wherein the fibrous solid substrate is obtained by controlling the nutrient composition and/or the moisture content by converting said one or more supplemental nutrient substrate compositions into one or more volatile compounds and evaporating said one or more volatile compounds from said fibrous solid fraction.

5. The use according to any of the preceding claims, wherein the pellet is supplemented with a pre-treated complex agricultural waste, such as straw and the like, which has been treated with warm and moist steam comprising N, such as via an N-steamer, and wherein said pre-treated complex agricultural waste is optionally grinded and/or dried prior to compression into a pellet, and/or
wherein the pellet is cooled, such as by active cooling in a cooling facility or by passive cooling by allowing the pellet to reach ambient temperature, and/or
wherein the pellet is packaged in bags, and/or
wherein the pellet is wetted by addition of water to obtain a wetted fibrous substrate.

6. The use according to any of the preceding claims which contains from about 0.2 kg to about 4.0 kg inorganic nitrogen (NH₄ - N) per ton, for example 0.2 to 0.5 kg, such as 0.5 to 1.0 kg, for example 1.0 to 1.2 kg, such as 1.2 to 1.4 kg, for example 1.4 to 1.5 kg, such as 1.5 to 1.6 kg, for example 1.6 to 1.8 kg, such as 1.8 to 2 kg, for example 2 to 2.5 kg, such as 2.5 to 3 kg, for example 3 to 3.5 kg, such as 3.5 to 4 kg inorganic nitrogen (NH₄ - N) per ton fibrous solid substrate in pellet form, and/or
which contains from about 3 kg to about 30 kg organic nitrogen per ton, for example 3 to 5, such as 5 to 10, for example 10 to 11, such as 11 to 12, for example 12 to 13, such as 13 to 14, for example 14 to 15, such as 15 to 16, for example 16 to 17, such as 17 to 18, for example 18 to 19, such as 19 to 20, for example 20 to 25, such as 25 to 30 kg organic nitrogen per ton fibrous solid substrate in pellet form, and/or
which contains less than 1.0 kg NH₃ per ton fibrous solid substrate in pellet form, such as less than 0.8 kg, for example less than 0.7 kg, such as less than 0.6 kg, for example less than 0.5 kg, such as less than 0.4 kg, for example less than 0.3 kg, such as less than 0.2 kg, and/or
which contains 0.01 - 0.05 kg NH₃ per ton fibrous solid substrate in pellet form, such as 0.05 - 0.1 kg NH₃, for example 0.1 - 0.2 kg NH₃, such as 0.2 - 0.3 kg NH₃, for example 0.3 - 0.4 kg NH₃, such as 0.4 - 0.5 kg NH₃.

7. The use according to any of the preceding claims, wherein said fibrous solid fraction of step d) and/or e) is heated to a temperature of from 70°C to 300°C, such as 70 to 80 °C, for example 80 to 90 °C, such as 90 to 100 °C, for example 100 to 110 °C, such as 110 to 120 °C, for example 120 to 130 °C, such as 130 to 140 °C, for example 140 to 150 °C, such as 150 to 160 °C, for example 160 to 170 °C, such as 170 to 180 °C, for example 180 to 190 °C, such as 190 to 200 °C, for example 200 to 250 °C, such as 250 to 300 °C, and/or
wherein said fibrous solid fraction of step d) and/or e) is subjected to a pressure of from 1 to 10 bar; such as 1 to 2 bar, for example 2 to 3 bar, such as 3 to 4 bar, for example 4 to 5 bar, such as 5 to 6 bar, for example 6 to 7 bar, such as 7 to 8 bar, for example 8 to 9 bar, such as 9 to 10 bar, and/or
wherein said fibrous solid fraction of step d) and/or e) is subjected to alkaline pH conditions, i.e. a pH above 7, such as above 7.5, for example a pH of above 8.0, and/or
wherein said fibrous solid fraction of step d) and/or e) is heated and dried, such as heated and dried in a dryer, such as a drum dryer.

8. The use according to any of the preceding claims further comprising draining liquid parts from the fibrous solid fraction comprising solid and liquid parts and obtaining a fibrous solid fraction comprising organic and inorganic nitrogen parts and having a total dry matter content of more than 25% (w/w), such as more than 30% (w/w), for example more than 35% (w/w), and a residual liquid fraction, and/or
further comprising degassing, wherein the biomass material comprising solid and liquid parts from a biogas fermenter following an anaerobic fermentation and biogas production is a degassed or partly degassed biomass, such as a biomass material which has been subject to anaerobic fermentation thereby producing a biogas and a degassed biomass material comprising organic and inorganic nitrogen parts.

9. The use according to any of the preceding claims, wherein said sanitation treatment that reduces the contents of volatile nitrogen-containing compounds and/or precursor volatile compounds present in the fibrous solid fraction, produces and/or evaporates volatile nitrogen-containing compounds and/or precursor volatile compounds, and/or
wherein said volatile nitrogen-containing compounds and/or precursor volatile compounds are diverted to and/or collected in a stripper and sanitation tank, and preferably
wherein said volatile nitrogen-containing compounds and/or precursor volatile compounds are diverted to an N-steamer, and optionally subsequently to a stripper and sanitation tank, and/or
wherein said sanitation treatment exploits primary and secondary combustion air sources, including exhaust air sources, present in or generated in the biogas fermentation facility as a result of performing said sanitation, wherein said primary and secondary combustion air sources are diverted to a stripper and sanitation tank for conversion and/or collection as solids.

10. The use according to any of the preceding claims, wherein said anaerobic fermentation and biogas production is performed using a biomass material, such as a fermentable biomass material, such as selected from the group consisting of: Biomasses comprising manures and slurries thereof, biomasses comprising crop residues, biomasses comprising silage crops, biomasses comprising animal carcasses and fractions thereof, slaughterhouse waste products, dairy waste products, meat and bone meal, animal category 2 waste products; spent fungal substrate; one or more complex biomasses; complex agricultural waste, such as crop residues, specifically straw, grass and the like; a complex biomass comprising protein, oily substances and fats; a complex biomass selected from the group consisting of organic municipal waste, foodstuff waste, fermentable organic industrial waste products, fish waste products, slaughterhouse waste; deep litter or manure from animals, especially from cattle, pigs and poultry holdings; animal carcasses and/or fractions thereof, meat and bone meal, blood plasma and any produce originating from animals, straw, grass, fibres and sawdust, and/or
wherein the biomass material is pre-treated prior to anaerobic fermentation and biogas production to increase the fermentation potential of and biogas production from said biomasses, and preferably
wherein the biomass material is pre-treated with warm and moist steam comprising N, such as from the dryer via the N-steamer, prior to anaerobic fermentation and biogas production; wherein said pre-treated biomass material is optionally grinded prior to anaerobic fermentation and biogas production,
and/or
wherein said biomass material is in a fluid condition, such as a liquid slurry, such as comprising a maximum of 10% solid parts.

11. The use according to any of the preceding claims, wherein said anaerobic fermentation is
a. conducted under thermophilic conditions and/or under mesophilic conditions, and/or
b. performed for about 5 to 15 days, such as 5 to 7 days, for example 7 to 10 days, such as 10 to 12 days, for example 12 to 15 days
and/or
wherein the fermentable biomass material is subjected to one or more pre-treatments prior to and/or during anaerobic fermentation of said biomass material, such as one or more of a chemical, mechanical and biological pre-treatment, wherein said pre-treatment reduce the size of the solids and macromolecular constituents making up the biomass material, and/or
wherein said pre-treatment is selected from the group consisting of a pre-fermentation, pressure cooking such as a lime pressure cooker, wet-oxidation, pH-controlled hydro-thermolysis, solvent pre-treatment such as organic solvent and cellulose solvent, steam explosion, auto-hydrolysis, pre-treatment with dilute acid, mild acid, lime, lime hydrate, alkaline, NaOH, Na₂CO₃, NaHCO₃, Ca(OH)₂, ammonia, KOH, sulfur dioxide, carbon dioxide, comminution, homogenization, milling, irradiation, ammonia fiber explosion, lignin-solubilizing microorganisms, enzymatic pre-treatment or hydrolysis; enzymatic hydrolysis comprising treatment with an amylase, a lipase, a protease, a hemicellulase, a pectinase, a cellulase, an oxidoreductase, a plant cell-wall degrading enzyme and enzymes derived from microbial organisms.

12. The use of a fibrous solid substrate in pellet form in a method for cultivating fungal cells and/or spores comprising the steps of
a. providing fungal cells and/or spores,
b. providing the fibrous solid substrate in pellet form according to any of the preceding claims, wherein the fibrous solid substrate in pellet form optionally further comprises one or more supplemental nutrient substrate compositions suitable for fungal growth,
c. adding water to the fibrous solid substrate in pellet form to obtain a wetted fibrous substrate,
d. contacting the fungal cells and/or spores with the wetted fibrous substrate,
e. cultivating the fungal cells and/or spores in said substrate, and
f. optionally obtaining a spent fungal substrate.

13. The use according to claim 12, wherein the moisture content of the wetted fibrous substrate is about 55% w/w to about 80% w/w, such as about 60% w/w to about 75% w/w, for example about 65% w/w to about 70% w/w, and/or
wherein the fungal cells and/or spores is selected from the group consisting of Basidiomycetes, Agaricomycetidae, Exobasidiomycetidae, Tremellomycetidae, Ustilaginomycetidae, Agaricus, Lentinula (Lentinus), Flammulina, Pleurotus, Lentinula edodes (shiitake); edible Agaricus species such as Agaricus bisporus, Agaricus campestris, Agaricus subrufescens; Flammulina velutipes (Enokitake), Pleurotus eryngii (Eryngii), Pleurotus ostreatus; Shimeji such as Lyophyllum shimejl, Buna-shimeji, Bunapi-shimeji, Hatake-shimeji, shirotamogidake, velvet pioppino; and a fungus capable of digesting cellulose, hemicellulose, lignin and lignocellulose, and/or
wherein the cultivation of the Basidiomycete cell takes place at a temperature of from 15°C to 35°C, for example 15°C to 17°C, such as 17°C to 20°C, for example 20°C to 22°C, such as 22°C to 25°C, for example 25°C to 30°C, such as 30°C to 35°C; and/or takes place in a fibrous solid substrate having a moisture content of from 50% by mass to 70% by mass.

14. a. The use according to any of claims 12 to 13 further comprising a step of collecting the spent fungal substrate, wherein said spent fungal substrate is at least partially digested by the cultivation of the fungal cells and is suitable as a feed stock for an anaerobic fermentation and biogas production, and/or
further comprising the steps of
a. fermenting the spent fungal substrate under anaerobic fermentation conditions,
b. producing a biogas and a degassed spent fungal substrate biomass material comprising organic and inorganic nitrogen parts.

15. The use according to any of claims 1 to 14 further comprising the steps of
i. subjecting said liquid fraction comprising solid and liquid organic and inorganic phosphor-containing parts to one or more separation steps, resulting in the provision of
a) a fibrous liquid fraction or concentrate, and
b) an essentially non-fibrous liquid fraction or permeate,
ii. subjecting the essentially non-fibrous liquid fraction or permeate to a gaseous fraction comprising nitrogen containing volatile compounds, wherein the pH and temperature are individually adjusted to shift the balance from ammonia to ammonium, thereby generating
a) an N-comprising fraction or N-fertilizer in liquid form,
b) a P-comprising fraction or sediment,
c) a heating source suitable for drying and other heating purposes,
iii) optionally subjecting the P-comprising fraction or sediment to a heating and drying treatment sufficient to evaporate water and volatile nitrogen (N) containing compounds, and thereby generating a P-comprising fraction or P-fertilizer.

## Patentansprüche

1. Verwendung eines faserförmigen Feststoffsubstrats in Granulatform in einem Verfahren zur Kultivierung von Pilzzellen und/oder -sporen; als Dünger; als Bodenverbesserer; und/oder als Einstreu für Tiere; wobei das faserförmige Feststoffsubstrat in Granulatform durch ein Verfahren erhalten wird, das die folgenden Schritte umfasst
a. Bereitstellen eines Biomassematerials, das feste und flüssige Teile umfasst, aus einem Biogasfermenter im Anschluss an anaerobe Fermentation und Biogaserzeugung,
b. Unterziehen der fermentierten Biomasse aus Schritt a. einem oder mehreren Trennschritten, wodurch Folgendes bereitgestellt wird
i) ein faserförmiger Feststoffanteil, der organische und anorganische Stickstoffteile umfasst und einen reduzierten Wassergehalt (Gew.-%) aufweist, und einen oder mehrere makromolekulare Nährstoffbestandteile, die aus der Gruppe bestehend aus Zellulose, Hemizellulose, Lignin und Lignozellulose ausgewählt sind, und
ii) mindestens einen flüssigen Anteil, der feste und flüssige organische und anorganische Phosphor enthaltende Teile umfasst,
c. Unterziehen des faserförmigen Feststoffanteils aus Schritt b. einer Aufbereitungsbehandlung,
wobei die Behandlung die Schritte i) Erwärmen des faserförmigen Feststoffanteils auf eine Temperatur von mehr als 70 °C, optional unter alkalischen pH-Wert-Bedingungen und optional ii) Aussetzen des faserförmigen Feststoffanteils, der organische und anorganische Stickstoffteile umfasst, einem Druck von mehr als 1 bar umfasst,
wobei die Behandlung i) im faserförmigen Feststoffanteil vorhandene lebensfähige Mikroorganismen reduziert oder beseitigt und/oder ii) den Gehalt an im faserförmigen Feststoffanteil vorhandenen flüchtigen stickstoffhaltigen Verbindungen und/oder flüchtigen Vorstufenverbindungen reduziert, und
d. Erhalten eines faserförmigen Feststoffanteils, der organische und anorganische Stickstoffteile umfasst, die einen reduzierten Gehalt an flüchtigen stickstoffhaltigen Verbindungen aufweisen,
e. optional Aussetzen des faserförmigen Feststoffanteils einem oder mehrerer von Erwärmen, Trocknen, Verdampfen, Druck und/oder alkalischen pH-Wert-Bedingungen,
f. optional Hinzufügen einer oder mehrerer fester und/oder flüssiger ergänzender Nährsubstratzusammensetzungen zu dem faserförmigen Feststoffanteil und
g. Verdichten des faserförmigen Feststoffanteils aus Schritt d., e. und/oder f. und dadurch Erzeugen eines faserförmigen Feststoffsubstrats in Granulatform.

2. Verwendung nach Anspruch 1, wobei die Diente des Granulats von etwa 200 kg/m³ bis etwa 800 kg/m³ reicht, wie etwa von etwa 300 kg/m³ bis etwa 700 kg/m³, beispielsweise von etwa 400 kg/m³ bis etwa 600 kg/m³, wie etwa von etwa 450 kg/m³ bis etwa 550 kg/m³, und/oder
wobei die Dichte des Granulats bei weniger als 800 kg/m³, wie etwa bei weniger als 700 kg/m³, beispielsweise bei weniger als 650 kg/m³, beispielsweise bei weniger als 600 kg/m³, beispielsweise bei weniger als 550 kg/m³, wie etwa bei weniger als 500 kg/m³, beispielsweise bei weniger als 450 kg/m³, wie etwa bei weniger als 400 kg/m³ liegt.

3. Verwendung nach einem der vorhergehenden Ansprüche, wobei der Durchmesser des Granulats etwa 6 bis 20 mm beträgt, wie etwa 6 bis 8 mm, beispielsweise 8 bis 10 mm, wie etwa 10 bis 12 mm, beispielsweise 12 bis 14 mm, wie etwa 14 bis 16 mm, beispielsweise 16 bis 18 mm, wie etwa 18 bis 20 mm, und/oder
wobei die Verdichtung unter Verwendung einer Pelletierpresse durchgeführt wird und/oder wobei die Dichte des Granulats um etwa 4- bis 20-mal höher ist als vor der Verdichtung, wie etwa 4- bis 6-mal, wie etwa 6- bis 8-mal, beispielsweise 8- bis 10-mal, wie etwa 10- bis 12-mal, beispielsweise 12- bis 14-mal, wie etwa 14-bis 16-mal, beispielsweise 16- bis 18-mal, wie etwa 18- bis 20-mal höher als vor der Verdichtung.

4. Verwendung nach einem der vorhergehenden Ansprüche, wobei das Granulat einen Trockengehalt von mindestens 75 % aufweist, wie etwa mindestens 80 %, beispielsweise mindestens 85 %, wie etwa mindestens 90 %, beispielsweise 95 %, und/oder
wobei das Granulat einen Feuchtigkeitsgehalt von weniger als oder gleich 25 % aufweist, wie etwa weniger als oder gleich 20 %, beispielsweise weniger als oder gleich 15 %, wie etwa weniger als oder gleich 10 %, beispielsweise weniger als oder gleich 8 %, und/oder
wobei das Granulat eine oder mehrere feste und/oder flüssige ergänzende Nährsubstratzusammensetzung umfasst und/oder
wobei die eine oder die mehreren festen und/oder flüssigen ergänzenden Nährsubstratzusammensetzungen einzeln aus der Gruppe bestehend aus Protein, C, N, P und K ausgewählt sind und/oder
wobei die Verwendung eine oder mehrere feste und/oder flüssige ergänzende Nährsubstratzusammensetzungen umfasst, wobei das faserförmige Feststoffsubstrat dadurch erhalten wird, dass die Nährstoffzusammensetzung und/oder der Feuchtigkeitsgehalt gesteuert wird, indem die eine oder die mehreren ergänzenden Nährsubstratzusammensetzungen in eine oder mehrere flüchtige Verbindungen umgewandelt werden und man die eine oder die mehreren flüchtigen Verbindungen aus dem faserförmigen Feststoffanteil verdampfen lässt.

5. Verwendung nach einem der vorhergehenden Ansprüche, wobei dem Granulat vorbehandelte komplexe landwirtschaftliche Abfälle, wie etwa Stroh oder dergleichen, zugesetzt werden, die mit warmem und feuchtem N-haltigen Dampf behandelt wurden, wie etwa über eine N-Dampfvorrichtung, und wobei die vorbehandelten komplexen landwirtschaftlichen Abfälle optional vor dem Verdichtung zu Granulat zermahlen und/oder getrocknet werden und/oder wobei das Granulat gekühlt wird, wie etwa durch aktive Kühlung in einer Kühlanlage oder durch passive Kühlung, indem ermöglicht wird, dass das Granulat eine Umgebungstemperatur erreicht, und/oder wobei das Granulat in Säcke verpackt wird und/oder wobei das Granulat durch Hinzugeben von Wasser befeuchtet wird, um ein befeuchtetes faserförmiges Substrat zu erhalten.

6. Verwendung nach einem der vorhergehenden Ansprüche, die etwa 0,2 kg bis etwa 4,0 kg anorganischen Stickstoff (NH₄-N) pro Tonne enthält, beispielsweise 0,2 bis 0,5 kg, wie etwa 0,5 bis 1,0 kg, beispielsweise 1,0 bis 1,2 kg, wie etwa 1,2 bis 1,4 kg, beispielsweise 1,4 bis 1,5 kg, wie etwa 1,5 bis 1,6 kg, beispielsweise 1,6 bis 1,8 kg, wie etwa 1,8 bis 2 kg, beispielsweise 2 bis 2,5 kg, wie etwa 2,5 bis 3 kg, beispielsweise 3 bis 3,5 kg, wie etwa 3,5 bis 4 kg anorganischen Stickstoff (NH₄-N) pro Tonne faserförmiges Feststoffsubstrat in Granulatform, und/oder die etwa 3 kg bis etwa 30 kg organischen Stickstoff pro Tonne enthält, beispielsweise 3 bis 5, wie etwa 5 bis 10, beispielsweise 10 bis 11, wie etwa 11 bis 12, beispielsweise 12 bis 13, wie etwa 13 bis 14, beispielsweise 14 bis 15, wie etwa 15 bis 16, beispielsweise 16 bis 17, wie etwa 17 bis 18, beispielsweise 18 bis 19, wie etwa 19 bis 20, beispielsweise 20 bis 25, wie etwa 25 bis 30 kg organischen Stickstoff pro Tonne faserförmiges Feststoffsubstrat in Granulatform, und/oder
die weniger als 1,0 kg NH₃ pro Tonne faserförmiges Feststoffsubstrat in Granulatform enthält, wie etwa weniger als 0,8 kg, beispielsweise weniger als 0,7 kg, wie etwa weniger als 0,6 kg, beispielsweise weniger als 0,5 kg, wie etwa weniger als 0,4 kg, beispielsweise weniger als 0,3 kg, wie etwa weniger als 0,2 kg, und/oder
die 0,01-0,05 kg NH₃ pro Tonne faserförmiges Feststoffsubstrat in Granulatform enthält, wie etwa 0,05-0,1 kg NH₃, beispielsweise 0,1-0,2 kg NH₃, wie etwa 0,2-0.3 kg NH₃, beispielsweise 0,3-0,4 kg NH₃, wie etwa 0,4-0,5 kg NH₃.

7. Verwendung nach einem der vorhergehenden Ansprüche, wobei der faserförmige Feststoffanteil aus Schritt d) und/oder e) auf eine Temperatur von 70 °C bis 300 °C erwärmt wird, wie etwa 70 bis 80 °C, beispielsweise 80 bis 90 °C, wie etwa 90 bis 100 °C, beispielsweise 100 bis 110 °C, wie etwa 110 bis 120 °C, beispielsweise 120 bis 130 °C, wie etwa 130 bis 140 °C, beispielsweise 140 bis 150 °C, wie etwa 150 bis 160 °C, beispielsweise 160 bis 170 °C, wie etwa 170 bis 180 °C, beispielsweise 180 bis 190 °C, wie etwa 190 bis 200 °C, beispielsweise 200 bis 250 °C, wie etwa 250 bis 300 °C, und/oder
wobei der faserförmige Feststoffanteil aus Schritt d) und/oder Schritt e) einem Druck von 1 bis 10 bar ausgesetzt wird; wie etwa 1 bis 2 bar, beispielsweise 2 bis 3 bar, wie etwa 3 bis 4 bar, beispielsweise 4 bis 5 bar, wie etwa 5 bis 6 bar, beispielsweise 6 bis 7 bar, wie etwa 7 bis 8 bar, beispielsweise 8 bis 9 bar, wie etwa 9 bis 10 bar, und/oder wobei der faserförmige Feststoffanteil aus Schritt d) und/oder e) alkalischen pH-Wert-Bedingungen ausgesetzt wird, d. h. einem pH-Wert über 7, wie etwa über 7,5, beispielsweise einem pH-Wert über 8,0, und/oder wobei der faserförmige Feststoffanteil aus Schritt d) und/oder e) erwärmt und getrocknet wird, wie etwa in einem Trockner, wie etwa einem Trommeltrocknet, erwärmt und getrocknet.

8. Verwendung nach einem der vorhergehenden Ansprüche, ferner umfassend ein Ablassen der flüssigen Teile aus dem faserförmigen Feststoffanteil, der feste und flüssige Teile umfasst, und ein Erhalten eines faserförmigen Feststoffanteils, der organische und anorganische Stickstoffteile umfasst und einen Gesamttrockensubstanzgehalt von mehr als 25 Gew.-% aufweist, wie etwa mehr als 30 Gew.-%, beispielsweise mehr als 35 Gew.-%, und einen Restanteil an Flüssigkeit, und/oder
ferner umfassend ein Entgasen, wobei das Biomassematerial, das feste und flüssige Teile umfasst, aus einem Biogasfermenter im Anschluss an eine anaerobe Fermentierung und Biogasproduktion eine entgaste oder teilweise entgaste Biomasse ist, wie etwa ein Biomassematerial, das einer anaeroben Fermentierung unterzogen wurde, durch die ein Biogas und ein entgastes Biomassematerial, das organische und anorganische Stickstoffteile umfasst, erzeugt wurden.

9. Verwendung nach einem der vorhergehenden Ansprüche, wobei die Aufbereitungsbehandlung, die den Gehalt an in dem faserförmigen Feststoffanteil vorhandenen flüchtigen stickstoffhaltigen Verbindungen und/oder flüchtigen Vorstufenverbindungen reduziert, flüchtige stickstoffhaltige Verbindungen und/oder flüchtige Vorstufenverbindungen erzeugt und/oder verdampfen lässt und/oder
wobei die flüchtigen stickstoffhaltigen Verbindungen und/oder flüchtigen Vorstufenverbindungen zu einem Abscheide- und Aufbereitungstank umgeleitet und/oder darin gesammelt werden und vorzugsweise
wobei die flüchtigen stickstoffhaltigen Verbindungen und/oder flüchtigen Vorstufenverbindungen zu einer N-Dampfvorrichtung und optional anschließend in einen Abscheide- und Aufbereitungstank umgeleitet werden und/oder
wobei die Aufbereitungsbehandlung primäre und sekundäre Verbrennungsluftquellen, einschließlich Abgasluftquellen, nutzt, die in der Biogasfermentierungsanlage infolge der Durchführung der Aufbereitung vorhanden sind oder erzeugt werden, wobei die primären und sekundären Verbrennungsluftquellen zur Umwandlung in und/oder Sammlung als Feststoffe zu einem Abscheide- und Aufbereitungstank umgeleitet werden.

10. Verwendung nach einem der vorhergehenden Ansprüche, wobei die anaerobe Fermentierung und Biogaserzeugung unter Verwendung eines Biomassematerials, wie etwa eines fermentierbaren Biomassematerials durchgeführt wird, das beispielsweise aus der Gruppe ausgewählt ist, die aus Folgendem besteht: Biomassen, die Mist und Gülle umfassen, Biomassen, die Ernterückstände umfassen, Biomassen, die Silagebestände umfassen, Biomassen, die Tierkadaver und Teile davon, Schlachtabfallprodukte, Molkereiabfallprodukte, Fleisch- und Knochenmehl, tierische Abfallprodukte der Kategorie 2 umfassen; einem verbrauchten Pilzsubstrat; einer oder mehreren komplexe Biomassen; komplexen landwirtschaftlichen Abfällen, wie etwa Ernterückstände, insbesondere Stroh, Gras oder dergleichen; einer komplexen Biomasse, die Protein, ölige Substanzen und Fette umfasst; einer komplexen Biomasse, die aus der Gruppe Bestehend aus Bio-Hausabfällen, Essensabfällen, fermentierbaren organischen Industrieabfallprodukten, Fischabfallprodukten, Schlachtabfällen ausgewählt ist; Tiefstreu und Mist von Tieren, besonders aus Kuh-, Schweine- und Geflügelhaltung; Tierkadavern und/oder Teilen davon, Fleisch- und Knochenmehl, Blutplasma und allen Tier-, Stroh-, Gras-, Holzfasern- und Sägemehlerzeugnissen, und/oder wobei das Biomassematerial vor der anaeroben Fermentierung und Biogaserzeugung vorbehandelt wird, um das Fermentierungspotential der Biomassen und die Biogaserzeugung daraus zu erhöhen, und vorzugsweise wobei das Biomassematerial vor der anaeroben Fermentierung und Biogaserzeugung mit warmem und feuchtem N-haltigem Dampf vorbehandelt wird, wie etwa aus dem Trockner über die N-Dampfvorrichtung; wobei das vorbehandelte Biomassematerial optional vor der anaeroben Fermentierung und Biogaserzeugung zermahlen wird, und/oder wobei das Biomassematerial in flüssigem Zustand vorliegt, wie etwa als flüssige Aufschlämmung, die beispielsweise maximal 10 % Festanteile umfasst.

11. Verwendung nach einem der vorhergehenden Ansprüche, wobei die anaerobe Fermentierung
a. unter thermophilen Bedingungen und/oder unter mesophilen Bedingungen erfolgt wird und/oder
b. etwa 5 bis 15 Tage lang durchgeführt wird, wie etwa 5 bis 7 Tage, beispielsweise 7 bis 10 Tage, wie etwa 10 bis 12 Tage, beispielsweise 12 bis 15 Tage, und/oder
wobei das fermentierbare Biomassematerial vor und/oder während der anaeroben Fermentierung des Biomassematerials einer oder mehreren Vorbehandlungen unterzogen wird, wie etwa einer oder mehreren chemischen, mechanischen und biologischen Vorbehandlungen, wobei die Vorbehandlungen die Größe der Feststoffe und makromolekularen Bestandteile reduziert, aus denen das Biomassematerial besteht, und/oder
wobei die Vorbehandlung aus der Gruppe bestehend aus Vorfermentierung, Druckkochen, wie etwa in einem Kalkdruckkocher, Nassoxidation, pH-Wert-gesteuerter Hydro-Thermolyse, Lösungsmittelvorbehandlung, wie etwa mit einem organischen Lösungsmittel und einem Zelluloselösungsmittel, Dampfexplosion, Autohydrolyse, Vorbehandlung mit Dünnsäure, Milchsäure, Kalk, Kalkhydrat, Alkalisalz, NaOH, Na₂CO₃, NaHCO₃, Ca(OH)₂, Ammoniak, KOH, Schwefeldioxid, Kohlendioxid, Zerkleinerung, Homogenisierung, Zerfräsung, Bestrahlung, Ammoniakfaserexplosion, Lignin solubilisierenden Mikroorganismen, Enzymvorbehandlung oder -hydrolyse ausgewählt ist; Enzymhydrolyse umfassend eine Behandlung mit einer Amylase, einer Lipase, einer Protease, einer Hemicellulase, einer Pectinase, einer Cellulase, einer Oxidoreduktase, einem Enzym zum Abbau von Pflanzenzellwänden und von mikrobiellen Organismen gewonnen Enzymen.

12. Verwendung eines faserförmigen Feststoffsubstrats in Granulatform in einem Verfahren zur Kultivierung von Pilzzellen und/oder -sporen, das die folgenden Schritte umfasst
a. Bereitstellen von Pilzzellen und/oder -sporen,
b. Bereitstellen des faserförmigen Feststoffsubstrats in Granulatform nach einem der vorhergehenden Ansprüche, wobei das faserförmige Feststoffsubstrat in Granulatform optional ferner eine oder mehrere ergänzende Nährsubstratzusammensetzungen umfasst, die für Pilzwachstum geeignet sind,
c. Hinzufügen von Wasser zu dem faserförmigen Feststoffsubstrat in Granulatform, um ein befeuchtetes faserförmiges Substrat zu erhalten,
d. Kontaktieren der Pilzzellen und/oder -sporen mit dem befeuchteten faserförmigen Substrat,
e. Kultivieren der Pilzzellen und/oder -sporen in dem Substrat und
f. optional Erhalten eines verbrauchten Pilzsubstrats.

13. Verwendung nach Anspruch 12, wobei der Feuchtigkeitsgehalt des befeuchteten faserförmigen Substrats bei etwa 55 Gew.-% bis etwa 80 Gew.-& liegt, wie etwa bei etwa 60 Gew.-% bis etwa 75 Gew.-%, beispielsweise bei etwa 65 Gew.-% bis etwa 70 Gew.-%, und/oder wobei die Pilzzellen und/oder -sporen aus der Gruppe ausgewählt sind, die aus Folgendem besteht: Basidiomycetes, Agaricomycetidae, Exobasidiomycetidae, Tremellomycetidae, Ustilagino-mycetidae, Agaricus, Lentinula (Lentinus), Flammulina, Pleurotus, Lentinula edodes (Shiitake); essbaren Agaricus-Spezies, wie etwa Agaricus bisporus, Agaricus campestris, Agaricus subrufescens; Flammulina velutipes (Enokitake), Pleurotus eryngii (Eryngii), Pleurotus ostreatus; Shimeji, wie etwa Lyophyllum shimeji, Buna-shimeji, Bunapi-shimeji, Hatake-shimeji, Shirotamogidake, Samt-Pioppino; und einem Pilz, der zur Verdauung von Zellulose, Hemizellulose, Lignin und Lignozellulose in der Lage ist, und/oder
wobei die Kultivierung der Basidiomycete-Zelle bei einer Temperatur von 15 °C bis 35 °C stattfindet, beispielsweise bei 15 °C bis 17 °C, wie etwa bei 17 °C bis 20 °C, beispielsweise bei 20 °C bis 22 °C, wie etwa bei 22 °C bis 25 °C, beispielsweise bei 25 °C bis 30 °C, wie etwa bei 30 °C bis 35 °C und/oder in einem faserförmigen Feststoffsubstrat stattfindet, das einen Feuchtigkeitsgehalt von 50 Gewichtsprozent bis 70 Gewichtsprozent aufweist.

14. Verwendung nach einem der Ansprüche 12 bis 13, ferner umfassend einen Schritt des Sammelns des verbrauchten Pilzsubstrats, wobei das verbrauchte Pilzsubstrat durch die Kultivierung der Pilzzellen zumindest teilweise verdaut ist und als Ausgangsmaterial für eine anaerobe Fermentierung und Biogaserzeugung geeignet ist, und/oder ferner umfassend die folgenden Schritte:
a. Fermentieren des verbrauchten Pilzsubstrats unter anaeroben Fermentierungsbedingungen,
b. Erzeugen eines Biogases und eines entgasten verbrauchten Pilzsubstrat-Biomassematerials, das organische und anorganische Stickstoffteile umfasst.

15. Verwendung nach einem der Ansprüche 1 bis 14, ferner umfassend die folgenden Schritte
i. Unterziehen des flüssigen Anteils, der feste und flüssige organische und anorganische phosphorhaltige Teile umfasst, einem oder mehreren Trennschritten, die dazu führen, dass Folgendes bereitgestellt wird:
a) ein faserförmiger flüssiger Anteil oder Konzentrat und
b) ein im Wesentlichen nicht-faserförmiger flüssiger Anteil oder Permeat,
ii. Aussetzen des im Wesentlichen nicht-faserförmigen flüssigen Anteils oder Permeats einem Gasanteil, der stickstoffhaltige flüchtige Verbindungen umfasst, wobei der pH-Wert und die Temperatur individuell so eingestellt werden, dass das Gleichgewicht von Ammoniak zu Ammonium verschoben wird, wodurch Folgendes erzeugt wird
a) ein N-umfassender Anteil oder N-Dünger in flüssiger Form,
b) ein P-umfassender Anteil oder Sediment,
c) eine Wärmequelle, die zum Trocknen oder zu anderen Wärmezwecken geeignet ist,
iii. optional Unterziehen des P-umfassenden Anteils oder Sediments einer Erwärmungs- oder Trocknungsbehandlung, die ausreicht, um Wasser und flüchtigen Stickstoff (N) enthaltende Verbindungen verdampfen zu lassen, wodurch ein P-umfassender Anteil oder P-Dünger erzeugt wird.

## Revendications

1. Utilisation d'un substrat solide fibreux sous forme de granules dans un procédé de culture de cellules fongiques et/ou de spores ; en tant qu'engrais ; en tant que produit d'amendement du sol ; et/ou en tant que litière pour animaux ; dans laquelle ledit substrat solide fibreux sous forme de granules est obtenu par un procédé comprenant les étapes de
a. fourniture d'un matériau de biomasse comprenant des parties solides et liquides â partir d'un fermenteur de biogaz après une fermentation anaérobie et la production de biogaz,
b. soumission du matériau de biogaz fermenté de l'étape a. à une ou plusieurs étapes de séparation résultant en la fourniture de i) une fraction solide fibreuse comprenant des parties d'azote organique et inorganique et ayant une teneur réduite (p/p) en eau, et comprenant un ou plusieurs constituants nutritifs macromoléculaires sélectionnés dans le groupe constitué par la cellulose, l'hémicellulose, la lignine et la lignocellulose, et ii) au moins une fraction liquide comprenant des parties solides et liquides contenant du phosphore organique et inorganique,
c. soumission de la fraction solide fibreuse de l'étape b. à un traitement d'assainissement
ledit traitement comprenant les étapes de i) chauffage de la fraction solide fibreuse à une température supérieure à 70 °C, facultativement dans des conditions de pH alcalin, et facultativement ii) soumission de la fraction solide fibreuse comprenant des parties d'azote organique et inorganique à une pression supérieure à 1 bar,
dans laquelle ledit traitement i) réduit ou élimine les microorganismes viables présents dans la fraction solide fibreuse, et/ou ii) réduit la teneur en composés volatils contenant de l'azote et/ou en composés volatils précurseurs présents dans la fraction solide fibreuse, et
d. obtention d'une fraction solide fibreuse comprenant des parties d'azote organique et inorganique ayant une teneur réduite en composés volatils contenant de l'azote,
e. soumission facultative de ladite fraction solide fibreuse à un ou plusieurs d'un chauffage, d'un séchage, d'une évaporation, d'une pression et/ou de conditions de pH alcalin,
f. ajout facultatif à ladite fraction solide fibreuse d'une ou plusieurs compositions de substrat nutritif supplémentaires solides et/ou liquides, et
g. compression de ladite fraction solide fibreuse de l'étape d.,
e. et/ou f., générant ainsi un substrat solide fibreux sous la forme de granules.

2. Utilisation selon la revendication 1, dans laquelle la densité du granule est d'environ 200 kg/m³ à environ 800 kg/m³, comme d'environ 300 kg/m³ à environ 700 kg/m³, par exemple d'environ 400 kg/m³ à environ 600 kg/m³, comme d'environ 450 kg/m³ à environ 550 kg/m³, et/ou
dans laquelle la densité du granule est inférieure à 800 kg/m³, comme inférieure à 700 kg/m³, par exemple inférieure à 650 kg/m³, par exemple inférieure à 600 kg/m³, par exemple inférieure à 550 kg/m³, comme inférieure à 500 kg/m³, par exemple inférieure à 450 kg/m³, comme inférieure à 400 kg/m³.

3. Utilisation selon l'une quelconque des revendications précédentes, dans laquelle le diamètre du granule est d'environ 6 à 20 mm, comme de 6 à 8 mm, par exemple de 8 à 10 mm, comme de 10 à 12 mm, par exemple de 12 à 14 mm, comme de 14 à 16 mm, par exemple de 16 à 18 mm, comme de 18 à 20 mm et/ou dans laquelle la compression est réalisée en utilisant une presse de pastillage, et/ou
dans laquelle la densité du granule est environ 4 à 20 fois plus élevée qu'avant la compression, comme 4 à 6 fois, comme 6 à 8 fois, par exemple 8 à 10 fois, comme 10 à 12 fois, par exemple 12 à 14 fois, comme 14 à 16 fois, par exemple 16 à 18 fois, comme 18 à 20 fois plus élevée qu'avant la compression.

4. Utilisation selon l'une quelconque des revendications précédentes, dans laquelle les granules ont une teneur en matière sèche d'au moins 75 %, comme d'au moins 80 %, par exemple d'au moins 85 %, comme d'au moins 90 %, par exemple 95 %, et/ou
dans laquelle le granule a une teneur en eau inférieure ou égale à 25 %, par exemple inférieure ou égale à 20 %, par exemple inférieure ou égale à 15 %, comme inférieure ou égale à 10 %, par exemple inférieure ou égale à 8 %, et/ou
dans laquelle le granule comprend une ou plusieurs compositions de substrat nutritif supplémentaires solides et/ou liquides, et/ou
dans laquelle les unes ou plusieurs compositions de substrat nutritif supplémentaires solides et/ou liquides sont individuellement choisies dans le groupe constitué par les protéines, le C, le N, le P, et le K, et/ou
dans laquelle l'utilisation comprend une ou plusieurs compositions de substrat nutritif supplémentaires solides et/ou liquides, dans laquelle le substrat solide fibreux est obtenu par régulation de la composition nutritive et/ou de la teneur en eau par conversion desdites une ou plusieurs compositions de substrat nutritif supplémentaires en un ou plusieurs composés volatils et évaporation desdits un ou plusieurs composés volatils de ladite fraction solide fibreuse.

5. Utilisation selon l'une quelconque des revendications précédentes, dans laquelle le granule est supplémenté d'un déchet agricole complexe prétraité, par exemple la paille et équivalents, qui a été traité avec de la vapeur chaude et humide comprenant du N, par exemple via un vaporisateur de N, et dans laquelle ledit déchet agricole complexe prétraité est facultativement broyé et/ou séché avant la compression en un granule, et/ou dans laquelle le granule est refroidi, par exemple par refroidissement actif dans une installation de refroidissement ou par refroidissement passif en permettant que le granule atteigne température ambiante, et/ou dans laquelle le granule est emballé dans des sacs, et/ou dans laquelle le granule est mouillé par ajout d'eau pour obtenir un substrat fibreux mouillé.

6. Utilisation selon l'une quelconque des revendications précédentes qui contient d'environ 0,2 kg à environ 4,0 kg d'azote inorganique (NH₄ - N) par tonne, par exemple de 0,2 à 0,5 kg, comme de 0,5 à 1,0 kg, par exemple de 1,0 à 1,2 kg, comme de 1,2 à 1,4 kg, par exemple de 1,4 à 1,5 kg, comme de 1,5 à 1,6 kg, par exemple de 1,6 à 1,8 kg, comme de 1,8 à 2 kg, par exemple de 2 à 2,5 kg, comme de 2,5 à 3 kg, par exemple de 3 à 3,5 kg, comme de 3,5 à 4 kg d'azote inorganique (NH₄ - N) par tonne de substrat solide fibreux sous la forme de granule, et/ou
qui contient d'environ 3 kg à environ 30 kg d'azote organique par tonne, par exemple de 3 à 5, comme de 5 à 10, par exemple de 10 à 11, comme de 11 à 12, par exemple de 12 à 13, comme de 13 à 14, par exemple de 14 à 15, comme de 15 à 16, par exemple de 16 à 17, comme de 17 à 18, par exemple de 18 à 19, comme de 19 à 20, par exemple de 20 à 25, comme de 25 à 30 kg d'azote inorganique par tonne de substrat solide fibreux sous la forme de granule, et/ou
qui contient moins de 1,0 kg de NH₃ par tonne de substrat solide fibreux sous la forme de granule, comme moins de 0,8 kg, par exemple moins de 0,7 kg, comme moins de 0,6 kg, par exemple moins de 0,5 kg, comme moins de 0,4 kg, par exemple moins de 0,3 kg, comme moins de 0,2 kg, et/ou
qui contient de 0,01 à 0,05 kg de NH₃ par tonne de substrat solide fibreux sous la forme de granule, comme de 0,05 à 0,1 kg de NH₃, par exemple de 0,1 à 0,2 kg de NH₃, comme de 0,2 à 0,3 kg de NH₃, par exemple de 0,3 à 0,4 kg de NH₃, comme de 0,4 à 0,5 kg de NH₃.

7. Utilisation selon l'une quelconque des revendications précédentes, dans laquelle ladite fraction solide fibreuse des étapes d) et/ou e) est chauffée à une température de 70 °C à 300 °C, comme de 70 à 80 °C, par exemple de 80 à 90 °C, comme de 90 à 100 °C, par exemple de 100 à 110 °C, comme de 110 à 120 °C, par exemple de 120 à 130 °C, comme de 130 à 140 °C, par exemple de 140 à 150 °C, comme de 150 à 160 °C, par exemple de 160 à 170 °C, comme de 170 à 180 °C, par exemple de 180 à 190 °C, comme de 190 à 200 °C, par exemple de 200 à 250 °C, comme de 250 à 300 °C, et/ou
dans laquelle ladite fraction solide fibreuse des étapes d) et/ou e) est soumise à une pression de 1 à 10 bars ; comme de 1 à 2 bars, par exemple de 2 à 3 bars, comme de 3 à 4 bars, par exemple de 4 à 5 bars, comme de 5 à 6 bars, par exemple de 6 à 7 bars, comme de 7 à 8 bars, par exemple de 8 à 9 bars, comme de 9 à 10 bars, et/ou
dans laquelle ladite fraction solide fibreuse de l'étape d) et/ou e) est soumise à des conditions de pH alcalin, en d'autres termes un pH supérieur à 7, comme supérieur à 7,5, par exemple un pH supérieur à 8,0, et/ou
dans laquelle ladite fraction solide fibreuse de l'étape d) et/ou e) est chauffée et séchée, par exemple chauffée et séchée dans un séchoir, par exemple un séchoir à tambour.

8. Utilisation selon l'une quelconque des revendications précédentes comprenant en outre le drainage de parties liquides de la fraction solide fibreuse comprenant des parties liquides et solides et l'obtention d'une fraction solide fibreuse comprenant des parties d'azote organique et inorganique ayant une teneur en matière sèche totale supérieure à 25 %(p/p), comme supérieure à 30 % (p/p), par exemple supérieure à 35 % (p/p), et une fraction liquide résiduelle, et/ou comprenant en outre le dégazage, dans laquelle le matériau de biomasse comprenant des parties solides et liquides d'un fermenteur à biogaz après une fermentation anaérobie et la production de biogaz est une biomasse dégazée ou partiellement dégazée, par exemple un matériau de biomasse qui a été soumis à une fermentation anaérobie produisant ainsi un biogaz et un matériau de biomasse dégazée comprenant des parties d'azote organique et inorganique.

9. Utilisation selon l'une quelconque des revendications précédentes, dans laquelle ledit traitement d'assainissement qui réduit la teneur en composés volatils contenant de l'azote et/ou en composés volatils précurseurs présents dans la fraction solide fibreuse, produit et/ou dégage des composés volatils contenant de l'azote et/ou des composés volatils précurseurs, et/ou
dans laquelle lesdits composés volatils contenant de l'azote et/ou composés volatils précurseurs sont déviés vers et/ou collectés dans une cuve de dépouillage et d'assainissement, et de préférence
dans laquelle lesdits composés volatils contenant de l'azote et/ou composés volatils précurseurs sont déviés vers un vaporisateur de N, et facultativement ensuite vers une cuve de dépouillage et d'assainissement, et/ou
dans laquelle ledit traitement d'assainissement exploite des sources d'air de combustion primaire et secondaire, y compris des sources d'air d'évacuation, présentes ou générées dans l'installation de fermentation de biogaz en résultat de la réalisation dudit assainissement, dans laquelle lesdites sources d'air de combustion primaire et secondaire sont déviées vers une cuve de dépouillage et d'assainissement pour la conversion et/ou la collecte sous la forme de solides.

10. Utilisation selon l'une quelconque des revendications précédentes, dans laquelle ladite fermentation anaérobie et production de biogaz est réalisée en utilisant un matériau de biomasse un matériau de biomasse, par exemple un matériau de biomasse fermentable, par exemple choisi dans le groupe constitué par : les biomasses comprenant des lisiers et des suspensions de ceux-ci, les biomasses comprenant des résidus de culture, les biomasses comprenant des cultures d'ensilage, les biomasses comprenant des carcasses animales et des fractions de celles-ci, les produits de résidus d'abattoirs, les produits de résidus laitiers, la viande et la farine d'os, les produits de résidus d'animaux de catégorie 2 ; un substrat fongique épuisé ; une ou plusieurs biomasses complexes ; des déchets agricoles complexes, tels que des résidus de culture, spécifiquement la paille, l'herbe et équivalents ; une biomasse complexe comprenant des protéines, des substances huileuses et des graisses ; une biomasse complexe sélectionnée dans le groupe constitué par les déchets municipaux organiques, les déchets alimentaires, les produits de résidu industriels organiques fermentables, les produits de déchet de poisson, les déchets d'abattoir ; la litière profonde ou le lisier d'animaux, notamment d'exploitations bétaillères, avicoles et porcines ; les carcasses d'animaux et/ou les fractions de celles-ci, la viande et la farine d'os, le plasma sanguin et tout produit provenant d'animaux, la paille, l'herbe, les fibres et la sciure, et/ou
dans laquelle le matériau de biomasse est prétraité avant la fermentation anaérobie et production de biogaz pour faire augmenter le potentiel de fermentation et la production de biogaz à partir desdites biomasses, et de préférence dans laquelle le matériau de biomasse est prétraité avec de la vapeur chaude et humide comprenant du N, par exemple issue du séchoir via le vaporisateur de N, avant la fermentation anaérobie et la production de biogaz ; dans laquelle ledit matériau de biogaz prétraité est facultativement broyé avant la fermentation anaérobie et la production de biogaz,
et/ou
dans laquelle ledit matériau de biomasse est dans une condition fluide, par exemple une suspension liquide, par exemple comprenant un maximum de 10 % de parties solides.

11. Utilisation selon l'une quelconque des revendications précédentes, dans laquelle ladite fermentation anaérobie est
a. réalisée dans des conditions thermophiles et/ou dans des conditions mésophiles, et/ou
b. réalisée pendant environ 5 à 15 jours, comme 5 à 7 jours, par exemple 7 à 10 jours, comme 10 à 12 jours, par exemple 12 à 15 jours et/ou
dans laquelle le matériau de biomasse fermentable est soumis à un ou plusieurs prétraitements avant et/ou pendant la fermentation anaérobie dudit matériau de biomasse, comme un ou plusieurs d'un prétraitement chimique, mécanique et biologique, dans laquelle ledit prétraitement réduit la taille des solides et des constituants macromoléculaires constituant le matériau de biomasse, et/ou
dans laquelle ledit prétraitement est sélectionné dans le groupe constitué par une pré-fermentation, la cuisson sous pression par exemple un cuiseur sous pression à la chaux, l'oxydation par voie humide, l'hydro-thermolyse à pH régulé, le prétraitement au solvant tel qu'un solvant organique et un solvant de cellulose, l'explosion à la vapeur, l'auto-hydrolyse, le prétraitement avec un acide dilué, un acide modéré, un hydrate de chaux, un alcalin, du NaOH, du Na₂CO₃, du NaHCO₃, du Ca(OH)₂, de l'ammoniac, du KOH, du dioxyde de soufre, du dioxyde de carbone, la comminution, l'homogénéisation, le broyage, l'irradiation, l'explosion des fibres à l'ammoniac, des microorganismes de solubilisation de lignine, le prétraitement enzymatique ou l'hydrolyse ; l'hydrolyse enzymatique comprenant un traitement avec une amylase, une lipase, une protéase, une hémicellulase, une pectinase, une cellulase, une oxydoréductase, une enzyme de dégradation de la paroi des cellules végétales et des enzymes dérivées d'organismes microbiens.

12. Utilisation d'un substrat solide fibreux sous la forme de granules dans un procédé de culture de cellules fongiques et/ou de spores comprenant les étapes suivantes
a. fourniture de cellules fongiques et/ou de spores,
b. fourniture du substrat solide fibreux sous la forme de granules selon l'une quelconque des revendications précédentes, dans laquelle le substrat solide fibreux sous la forme de granules comprend facultativement en outre une ou plusieurs compositions de substrat nutritif supplémentaires adaptées pour la croissance fongique,
c. ajout d'eau au substrat solide fibreux sous la forme de granules pour obtenir un substrat fibreux mouillé,
d. mise en contact des cellules fongiques et/ou des spores avec le substrat fibreux mouillé,
e. culture des cellules fongiques et/ou des spores dans ledit substrat, et
f. obtention facultative d'un substrat fongique épuisé.

13. Utilisation selon la revendication 12, dans laquelle la teneur en eau du substrat fibreux mouillé est d'environ 55 % p/p à environ 80 % p/p, comme d'environ 60 % p/p à environ 75 % p/p, par exemple d'environ 65 % p/p à environ 70 % p/p, et/ou dans laquelle les cellules fongiques et/ou les spores sont choisies dans le groupe constitué par les basidiomycètes, Agaricomycetidae, Exobasidiomycetidae, Tremellomycetidae, Ustilaginomycetidae, Agaricus, Lentinula (Lentinus), Flammulina, Pleurotus, Lentinula edodes (shiitake) ; les espèces comestibles d'Agaricus comme Agaricus bisporus, Agaricus campestris, Agaricus subrufescens ; Flammulina velutipes (enoki), Pleurotus eryngii (Eryngii), Pleurotus ostreatus ; les shimeji comme Lyophyllum shimeji, Buna-shimeji, Bunapi-shimeji, Hatake-shimeji, shirotamogidake, la pholiote du peuplier ; et un champignon capable de digérer la cellulose, l'hémicellulose, la lignine et la lignocellulose, et/ou
dans laquelle la culture de la cellule de basidiomycètes a lieu à une température de 15 °C à 35 °C, par exemple de 15 °C à 17 °C, comme de 17 °C à 20 °C, par exemple de 20 °C à 22 °C, comme de 22 °C à 25 °C, par exemple de 25 °C à 30 °C, comme de 30 °C à 35 °C ; et/ou a lieu dans un substrat solide fibreux ayant une teneur en eau de 50 % en masse à 70 % en masse.

14. Utilisation selon l'une quelconque des revendications 12 et 13 comprenant en outre une étape de collecte du substrat fongique épuisé, dans laquelle ledit substrat fongique épuisé est au moins partiellement digéré par culture des cellules fongiques et est adapté en tant que charge d'alimentation pour une fermentation anaérobie et production de biogaz, et/ou comprenant en outre les étapes de
a. fermentation du substrat fongique épuisé dans des conditions de fermentation anaérobie,
b. production d'un biogaz et d'un matériau de biomasse de substrat fongique dégazé comprenant des parties d'azote organique et inorganique.

15. Utilisation selon l'une quelconque des revendications 1 à 14 comprenant en outre les étapes suivantes
i. soumission de ladite fraction liquide comprenant des parties contenant du phosphore organique et inorganique liquide et solide à une ou plusieurs étapes de séparation, résultant en la fourniture de
a) une fraction liquide fibreuse ou concentrée, et
b) une fraction ou perméat liquide sensiblement non fibreux(se),
ii. soumission de la fraction ou perméat liquide sensiblement non fibreux(se à une fraction gazeuse comprenant des composés volatils contenant de l'azote, dans laquelle le pH et la température sont individuellement ajustés pour décaler l'équilibre de l'ammoniac à l'ammonium, générant ainsi
a) une fraction comprenant du N ou engrais à base de N sous forme liquide,
b) une fraction ou un sédiment comprenant du P,
c) une source de chauffage adaptée à sécher et à d'autres objectifs de chauffage,
iii. facultativement la soumission de la fraction ou sédiment comprenant du P à un traitement de chauffage et de séchage suffisant pour faire évaporer l'eau et les composés volatils contenant de l'azote (N), et générant ainsi une fraction comprenant du P ou un engrais à base de P.
